(19) Europäisches Patentamt
European Patent Office
Office européen des brevets

(11) **EP 3 459 453 B1**

(12) **EUROPEAN PATENT SPECIFICATION**

(45) Date of publication and mention
of the grant of the patent:
**12.05.2021 Bulletin 2021/19**

(21) Application number: **16902325.6**

(22) Date of filing: **16.05.2016**

(51) Int Cl.:
**A61B 5/11** *(2006.01)*   **A61B 5/00** *(2006.01)*

(86) International application number:
**PCT/JP2016/064516**

(87) International publication number:
**WO 2017/199305 (23.11.2017 Gazette 2017/47)**

(54) **INFORMATION PROCESSING DEVICE, INFORMATION PROCESSING METHOD, AND INFORMATION PROCESSING PROGRAM**

INFORMATIONSVERARBEITUNGSVORRICHTUNG, INFORMATIONSVERARBEITUNGSVERFAHREN UND INFORMATIONSVERARBEITUNGSPROGRAMM

DISPOSITIF, PROCÉDÉ ET PROGRAMME DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.03.2019 Bulletin 2019/13**

(73) Proprietor: **Fujitsu Limited**
**Kawasaki-shi, Kanagawa 211-8588 (JP)**

(72) Inventors:
• **HOTTA, Shinji**
**Kawasaki-shi**
**Kanagawa 211-8588 (JP)**
• **INOMATA, Akihiro**
**Kawasaki-shi**
**Kanagawa 211-8588 (JP)**

(74) Representative: **Haseltine Lake Kempner LLP**
**138 Cheapside**
**London EC2V 6BJ (GB)**

(56) References cited:
**JP-A- 2011 045 524     JP-A- 2015 109 945**
**JP-A- 2015 165 895     US-A1- 2012 083 705**
**US-A1- 2016 007 934**

• **MARJAN GHAZVININEJAD ET AL: "HMM based semi-supervised learning for activity recognition", PROCEEDINGS OF THE 2011 INTERNATIONAL WORKSHOP ON SITUATION ACTIVITY & GOAL AWARENESS, SAGAWARE '11, 18 September 2011 (2011-09-18), pages 95-100, XP055577827, New York, New York, USA DOI: 10.1145/2030045.2030065 ISBN: 978-1-4503-0926-4**

## Description

Technical Field

The present invention relates to an information processing apparatus, an information processing method, and an information processing program.

Background Art

Many elderly people have problems in daily movement such as walking. Thus, recognizing ability to move of elderly people in daily life is attracting the interest of medical workers.

In order to correctly recognize this, it is important to quantify the ways of activities that are frequently daily performed, such as walking, ascending/descending stairs, bicycling, and turning, with sensors. Owing to this, first, it is desirable to correctly detect the duration in which an activity has been performed (hereinafter, referred to as "activity duration").

In related art, there is suggested a method of detecting an activity duration by applying machine learning using data of a wearable sensor.

For example, sensors are attached to five locations of a body of a subject, and sensor data during a variety of activities, such as walking, running, ascending stairs, and the like, are previously collected. The sensor data is used, and a learning model for discriminating an activity type based on a feature value of the data is learned according to a decision tree. The leaning model is applied to test data, and an activity is determined (see NPL 1).

Since the way of activity varies in accordance with a person, a model is learned per person, and hence the accuracy of determining an activity is improved (see NPL 2).

However, it is difficult to collect a large amount of learning data of patients.

To decrease the cost of collecting leaning data, there is suggested a method of creating an expected model for an activity duration by using a small amount of leaning data and non-labeled data.

For example, a plurality of expected models are created from previously obtained leaning data. There is suggested a method (En-Co-Training) of adding non-labeled data to leaning data if the plurality of expected models are applied to the non-labeled data and all assumption results meet the non-labeled data (NPL 3).

There is a method of constructing an expected model of a subject by transferring previously obtained leaning data of another person. For example, there is suggested a method (TransEMDT) of first using leaning data of a person A and learning a decision tree, and then, using non-labeled observation data of a person B and changing the decision tree (NPL 4). For example, a threshold of the decision tree is updated when discrimination is made with a high confidence factor.

Citation List Non Patent Literature

NPL 1: Bao, "Activity Recognition from User-Annotated Acceleration Data", 2004; NPL 2: Weiss, "The Impact of Personalization on Smartphone-Based Activity Recognition", 2012; NPL 3: Guan, "Activity recognition based on semi-supervised learning", 2007; NPL 4: Zhao, "Cross-People Mobile-Phone Based Activity Recognition", 2007

**<INSERT PAGE 2a HERE>**

Summary of Invention

Technical Problem

However, with the method in NPL 3, since the way of movement varies in accordance with a person, it is difficult to learn an optimal estimated model.

With the method in NPL 4, whether the assumption is successfully performed depends on the previously obtained leaning model, and hence the Reference may be made to any of:

US 2012/083705 A1, which relates to an activity monitoring system comprising a fixture having size/shape adapted to couple to a location on a user's body and a particular signature, and a portable monitoring device adapted to detect the fixture's particular signature, wherein the monitoring device includes a housing that is adapted to engage the fixture, activity sensors disposed in the housing to detect activity of the user and to generate data which is representative of the activity of the user, and processing circuitry disposed in the housing to calculate an activity-related quantity of the user using the data which is representative of the activity of the user, wherein the processing circuitry determines the monitoring device is engaging the fixture by detecting the fixture's particular signature, and, in response thereto, calculates the activity-related quantity using data from a set of the activity sensors;

US 2016/007934 A1, which relates to aspects of generating movement measures that quantify motion data samples generated by a wearable electronic device, wherein motion data samples generated by the set of motion sensors in a wearable electronic device may be obtained, the wearable electronic device may then generate a first movement measure based on a quantification of the first set of motion data samples, the wearable electronic device may also generate a second movement measure based on a quantification of the second set of the motion data samples, the wearable electronic device may then cause the non-transitory machine readable storage medium to store the first movement measure and the second movement measure as time series data; and

a paper titled "HMM based semisupervised learning for activity recognition", submitted by Marjan Ghazvininejad et al, in Proceedings Of The 2011 International Workshop On Situation Activity & Goal Awareness, Sagaware '11, 18 September 2011, pages 95-100, XP055577827, New York, USA DOI: 10.1145/2030045.2030065 ISBN: 978-1-4503-0926-4. possibility of failing to make assumption for a new user who moves in an unknown way may be high.

According to an aspect, it is an object to be able to correctly detect an activity duration when the way of movement is changed in accordance with a subject, a surrounding environment of the subject, or a state of the subject. Solution to Problem

The present invention is defined by the independent claims, to which reference should now be made. Specific embodiments are defined in the dependent claims. According to an idea, an information processing apparatus includes a specification unit that specifies a duration expected such that a predetermined activity has been performed based on sensor data indicating a motion of at least one region of a body in time series; a detection unit that detects a motion group in the duration based on the sensor data; and a calculation unit that calculates, based on a feature of a detected motion group, a likelihood that the motion group is the predetermined activity.

Effects of Invention

With an exemplary embodiment, the activity duration may be correctly detected when the way of movement is changed in accordance with the subject, the surrounding environment of the subject, or the state of the subject. Brief Description of Drawings

[FIG. 1] FIG. 1 is a diagram illustrating a hardware configuration example of an activity duration determination apparatus according to a first embodiment.
[FIG. 2] FIG. 2 is a diagram illustrating a functional configuration example of the activity duration determination apparatus according to the first embodiment.
[FIG. 3] FIG. 3 is a diagram illustrating a functional configuration example of an activity duration determination unit according to the first embodiment.
[FIG. 4] FIG. 4 includes diagrams illustrating respective directions.
[FIG. 5] FIG. 5 is a diagram illustrating a natural motion that is detected per activity type and per body region.
[FIG. 6] FIG. 6 is a diagram illustrating left-right alternation degree.
[FIG. 7] FIG. 7 is a diagram illustrating interval consistency degree.
[FIG. 8] FIG. 8 is a diagram illustrating speed consistency degree.
[FIG. 9] FIG. 9 is a diagram illustrating inverse-leg support degree.
[FIG. 10] FIG. 10 is a diagram illustrating both-leg landing degree.
[FIG. 11] FIG. 11 is a diagram illustrating simultaneous movement degree.
[FIG. 12] FIG. 12 is a diagram illustrating landing flexion degree.
[FIG. 13] FIG. 13 is a diagram illustrating takeoff flexion degree.
[FIG. 14] FIG. 14 is a diagram illustrating up-down alternation degree.
[FIG. 15] FIG. 15 is a diagram illustrating simultaneous impact degree.
[FIG. 16] FIG. 16 is a diagram illustrating feature likelihoods of a calculation target per activity type.
[FIG. 17] FIG. 17 includes diagrams each illustrating a function example of a likelihood calculation rule.
[FIG. 18] FIG. 18 is a diagram illustrating correlation between respective characteristics of natural patterns and respective feature likelihoods.
[FIG. 19] FIG. 19 is a flowchart illustrating an example of a procedure that is executed by the activity duration determination apparatus according to the first embodiment.
[FIG. 20] FIG. 20 is a diagram illustrating a setting example of activity types of determination targets.
[FIG. 21] FIG. 21 is a diagram illustrating an extraction example of a candidate duration.
[FIG. 22] FIG. 22 is a diagram illustrating an example of confirmation results of activity types of each candidate duration.

[FIG. 23] FIG. 23 is a flowchart illustrating an example of a procedure for activity duration determination processing according to the first embodiment.

[FIG. 24] FIG. 24 is a diagram illustrating an example of a parameter set.

[FIG. 25] FIG. 25 includes diagrams each illustrating an example of a filter pass band.

[FIG. 26] FIG. 26 is a diagram illustrating an example of sensor data and peaks before and after filtering.

[FIG. 27] FIG. 27 is a diagram illustrating integration of a plurality of peaks.

[FIG. 28] FIG. 28 is a diagram illustrating an example of a method of specifying a motion range.

[FIG. 29] FIG. 29 is a diagram illustrating information obtained by specifying a motion range.

[FIG. 30] FIG. 30 is a diagram illustrating an example of a likelihood calculation rule group.

[FIG. 31] FIG. 31 is a diagram illustrating an example of calculation results of natural pattern likelihoods per natural motion detector.

[FIG. 32] FIG. 32 is a flowchart illustrating an example of a procedure for processing of extracting an inter-motion feature value.

[FIG. 33] FIG. 33 is a diagram illustrating an example of extraction results of inter-motion feature values per candidate motion.

[FIG. 34] FIG. 34 is a flowchart illustrating an example of a procedure for processing of calculating feature likelihoods.

[FIG. 35] FIG. 35 is a diagram illustrating a calculation example of a left-right alternation likelihood.

[FIG. 36] FIG. 36 is a flowchart illustrating an example of a procedure for processing of calculating a natural pattern likelihood.

[FIG. 37] FIG. 37 is a diagram illustrating an example of a predetermined range per activity type for a natural pattern likelihood.

[FIG. 38] FIG. 38 is a diagram illustrating an example of determination results of activity durations relating to "walking".

[FIG. 39] FIG. 39 is a diagram illustrating an example in which activity feature values are added to the determination results of the activity durations relating to "walking".

[FIG. 40] FIG. 40 is a diagram illustrating an example of visualization of activity feature values.

[FIG. 41] FIG. 41 includes diagrams illustrating an example of determination results of activity durations of "walking" when this embodiment is applied to various ways of walking.

[FIG. 42] FIG. 42 is a diagram illustrating a second functional configuration example of the activity duration determination apparatus according to the first embodiment.

[FIG. 43] FIG. 43 is a diagram illustrating a third functional configuration example of the activity duration determination apparatus according to the first embodiment.

[FIG. 44] FIG. 44 is a diagram illustrating an example in which candidate motions of a candidate motion group are classified into a walking motion and a non-walking motion.

[FIG. 45] FIG. 45 is a diagram illustrating an extraction example of inter-motion feature values according to a second embodiment.

[FIG. 46] FIG. 46 is a diagram illustrating a strategy for classifying candidate motions of a candidate motion group into a walking motion and a non-walking motion.

[FIG. 47] FIGs. 47A and 47B are a diagram illustrating an example in which each state is assigned to a corresponding candidate motion.

[FIG. 48] FIG. 48 is a diagram illustrating a functional configuration example of an activity duration determination unit according to the second embodiment.

[FIG. 49] FIG. 49 is a flowchart illustrating an example of a procedure for activity duration determination processing according to the second embodiment.

[FIG. 50] FIG. 50 is a flowchart illustrating an example of a procedure for processing of classifying candidate durations.

[FIG. 51] FIGs. 51A and 51B are a diagram illustrating likelihood calculation rules according to the second embodiment.

[FIG. 52] FIG. 52 is a diagram illustrating an example of calculation results of state observation probability according to hidden Markov model.

[FIG. 53] FIGs. 53A and 53B are a diagram illustrating a first example of state transition probability information.

[FIG. 54] FIG. 54 is a diagram illustrating an example of initial state probability information.

[FIG. 55] FIG. 55 is a diagram illustrating an example of an optimal state sequence.

[FIG. 56] FIG. 56 is a diagram illustrating an example of specification results of a target duration.

[FIG. 57] FIG. 57 is a diagram illustrating an example of determination results of activity durations based on one natural motion detector for one candidate duration according the second embodiment.

[FIG. 58] FIG. 58 is a diagram illustrating an example of determination results of activity durations based on all natural motion detectors for one candidate duration according the second embodiment.

[FIG. 59] FIG. 59 is a diagram illustrating an example of evaluation values of determination results of activity durations based on all natural motion detectors for one candidate duration.

[FIG. 60] FIG. 60 is a diagram illustrating an example of state transition of "descending stairs".

[FIG. 61] FIG. 61 is a diagram illustrating a second example of state transition probability information.

[FIG. 62] FIG. 62 is a diagram illustrating a result example in which state transition probability information is used and a state is assigned to a candidate motion group.

[FIG. 63] FIG. 63 is a diagram illustrating an example of plantar pressure data.

[FIG. 64] FIG. 64 is a diagram illustrating a detection example of a natural motion based on plantar pressure data.

[FIG. 65] FIG. 65 is a diagram illustrating an example of sensor data indicating a motion of an upper body.

[FIG. 66] FIG. 66 is a diagram illustrating an example of data indicating amplitude in an up-down direction among upper-body motion data.

[FIG. 67] FIG. 67 is a diagram illustrating a functional configuration example of an activity duration determination unit according to a fifth embodiment.

[FIG. 68] FIG. 68 is a flowchart illustrating an example of a procedure for activity duration determination processing according to the fifth embodiment.

[FIG. 69] FIG. 69 includes diagrams each illustrating an example of a motion feature value of each natural motion.

[FIG. 70] FIGs. 70A and 70B include diagrams illustrating an example of a reference value parameter set for a leg swinging motion per type of natural motion detector.

[FIG. 71] FIG. 71 is a diagram illustrating an example of a method of calculating a likelihood element.

[FIG. 72] FIG. 72 is a diagram illustrating an example of calculation results of evaluation values based on a natural pattern likelihood and a natural motion likelihood.

[FIG. 73] FIGs. 73A and 73B are a diagram illustrating re-setting of a parameter list.

Description of Embodiments

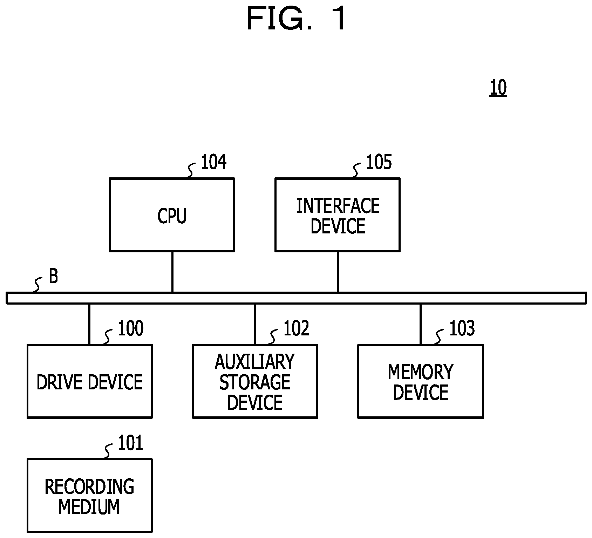

Embodiments of the present invention will be described below with reference to the accompanying drawings. FIG. 1 is a diagram illustrating a hardware configuration example of an activity duration determination apparatus according to a first embodiment. An activity duration determination apparatus 10 in FIG. 1 includes a drive device 100, an auxiliary storage device 102, a memory device 103, a CPU 104, an interface device 105, and the like, which are coupled to one another via a bus B.

A program for implementing processing with the activity duration determination apparatus 10 is provided from a recording medium 101. When the recording medium 101 storing the program is set in the drive device 100, the program is installed from the recording medium 101 to the auxiliary storage device 102 via the drive device 100. Note that the installation of the program does not have to be performed from the recording medium 101, and the program may be downloaded from another computer via a network. The auxiliary storage device 102 stores the installed program, and stores required files, data, and the like.

The memory device 103 reads the program from the auxiliary storage device 102 and stores the read program when receiving an instruction to activate the program. The CPU 104 executes a function relating to the activity duration determination apparatus 10 according to the program stored in the memory device 103. The interface device 105 is used as an interface for coupling to a network.

Note that an example of the recording medium 101 is a portable recording medium such as a CD-ROM, a DVD disk, or a USB memory. An example of the auxiliary storage device 102 is an HDD (Hard Disk Drive), a flash memory, or the like. Each of the recording medium 101 and the auxiliary storage device 102 corresponds to a computer-readable recording medium.

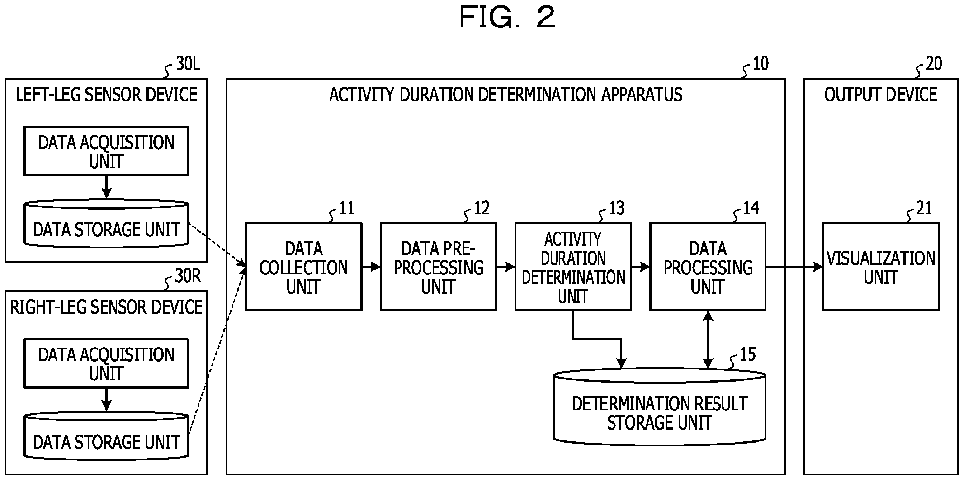

FIG. 2 is a diagram illustrating a functional configuration example of the activity duration determination apparatus according to the first embodiment. In FIG. 2, the activity duration determination apparatus 10 includes a data collection unit 11, a data pre-processing unit 12, an activity duration determination unit 13, a data processing unit 14, and the like. Each of these units is implemented by processing to be executed by the CPU 104, which is caused by one or more programs installed in the activity duration determination apparatus 10. The activity duration determination apparatus 10 also uses a determination result storage unit 15. The determination result storage unit 15 may be implemented by using, for example, the auxiliary storage device 102, or a storage device or the like connectable to the activity duration determination apparatus 10 via a network.

The data collection unit 11 collects sensor data (motion sensor data) from sensors attached to a human body, and stores the collected sensor data in, for example, the auxiliary storage device 102. The sensor data is, for example, data containing gyro (angular velocity) data and acceleration data in time series. FIG. 2 illustrates, as examples of sensors, a left-leg sensor device 30L, a right-leg sensor device 30R, and the like.

The left-leg sensor device 30L is, for example, a sensor device attached to a left ankle. The right-leg sensor device 30R is, for example, a sensor device attached to a right ankle. Described according to this embodiment is, as a system requirement desirable on a site, an example in which sensor devices are attached to both ankles considering a burden on a patient who wears the sensor devices is light.

However, without limiting to both ankles, a sensor may be attached to one or any combination of heels, toes, thighs, feet, of both legs, a pocket, a body or the like. For example, as illustrated in FIG. 2, one or a plurality of upper-body sensor devices 30U may be attached. The upper-body sensor device 30U is a sensor device attached to an upper body.

Note that each sensor device incorporates a gyro sensor or an acceleration sensor with at least one axis.

Each sensor device includes a data acquisition unit and a data storage unit. The data acquisition unit acquires sensor data. The data storage unit stores sensor data. The data collection unit 11 of the activity duration determination apparatus 10 collects sensor data stored in the data storage unit in batches or continuously.

The data pre-processing unit 12 performs pre-processing on the collected sensor data. For example, the data pre-processing unit 12 synchronizes the time points between the sensor data collected from the respective sensor devices. The time points may be synchronized by a known method. The processing results by the data pre-processing unit 12 overwrite the sensor data stored in the auxiliary storage device 102.

The activity duration determination unit 13 determines an activity duration, an activity type in the activity duration, and the like, of a person to which the sensor devices are attached (hereinafter, referred to as "subject"). The activity duration is a duration in which an activity has been performed. The activity type is a type of the activity. In this embodiment, seven types of activities are discriminated as activity types including "walking", "running", "descending stairs", "ascending stairs", "skipping", "turning", and "bicycling". In other words, the activity duration or activity durations of one or a plurality of the seven types of activities are determined. For example, when the person walks straight on a flat ground, only the activity duration of "walking" is output. When the person makes a turn during walking, the activity durations of "walking" and "turning" are output in an overlapping manner. The determination results by the activity duration determination unit 13 are stored in the determination result storage unit 15. Note that "turning" represents an activity of changing a direction. "Bicycling" represents an activity of pedaling a bicycle. Note that the activity types are not limited to the above-listed activity types, and may include "standing", "sitting", "sleeping", "sitting up", "picking up something", "washing hands", "washing face", "opening door", "closing door", "brushing teeth", and the like.

The data processing unit 14 processes the determination results by the activity duration determination unit 13 into, for example, a format suitable for visualization. The processed data is visualized (output) by a visualization unit 21 of an output device 20. The output device 20 is, for example, a display device, a printer, or the like.

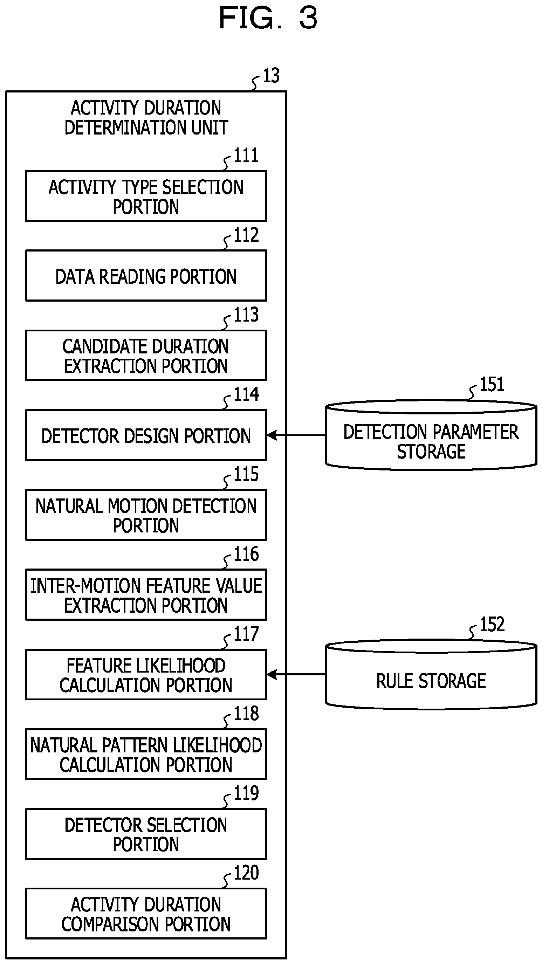

The activity duration determination unit 13 is described further in detail. FIG. 3 is a diagram illustrating a functional configuration example of the activity duration determination unit according to the first embodiment. In FIG. 3, the activity duration determination unit 13 includes an activity type selection portion 111, a data reading portion 112, a candidate duration extraction portion 113, a detector design portion 114, a natural motion detection portion 115, an inter-motion feature value extraction portion 116, a feature likelihood calculation portion 117, a natural pattern likelihood calculation portion 118, a detector selection portion 119, an activity duration comparison portion 120, and the like. The activity duration determination unit 13 also uses a detection parameter storage unit 151 and a rule storage unit 152. Each of these storage units may be implemented by using, for example, the auxiliary storage device 102, or a storage device or the like connectable to the activity duration determination apparatus 10 via a network.

The activity type selection portion 111 selects an activity type that is a determination target in an activity duration. Specifically, in this embodiment, the activity duration is sequentially determined for each activity type. Hereinafter, the selected activity type is referred to as "target activity type".

The data reading portion 112 reads sensor data of a plurality of regions (for example, both ankles) from the auxiliary storage device 102.

The candidate duration extraction portion 113 extracts a candidate duration that is a candidate for an activity duration based on the sensor data of the plurality of regions. The candidate for the activity duration represents a duration expected such that an activity has been performed. A plurality of candidate durations are extracted in ordinary situations. The plurality of regions are, for example, "left ankle and right ankle". However, another combination, such as "left ankle and hip" or "left-leg pocket and hip", may be employed.

The detector design portion 114 and the natural motion detection portion 115 execute processing for detecting a natural motion for each candidate duration based on the sensor data. No matter what kind person is, when a person performs a certain activity, a partial motion corresponding to the activity occurs by a method unique to the activity. For example, during "walking", a partial motion of "stepping one leg forward" appears, and the partial motion is performed alternately with left and right legs. During "bicycling", a partial motion of "rotating one leg" appears, and the partial motion is performed in inverse phases with left and right legs. In this embodiment, such a partial motion is referred to as "natural motion". In this embodiment, natural motions are classified into four types including "leg swinging motion" of moving one leg forward, "landing motion" of landing one leg, "traveling motion" of moving an upper body forward, and "rotating motion" of rotating a certain region in a left-right direction.

The natural motion is a motion occurring by rotation of at least one joint, and hence appears as a (large or small) peak in sensor data in a certain axial direction of a certain region. The natural motion detection portion 115 detects the peak and specifies an occurrence time point of the natural motion. For example, a leg swinging motion is detected by peak detection of an angular velocity signal of a leg in a sagittal plane direction. A landing motion is detected by peak detection of an acceleration signal in an up-down direction of a leg or an upper body, or a plantar pressure signal. A traveling motion is detected by peak detection of an acceleration signal of an upper body in a front-rear direction. A rotating motion is detected by peak detection of an angular velocity signal of a target region in a transverse plane direction.

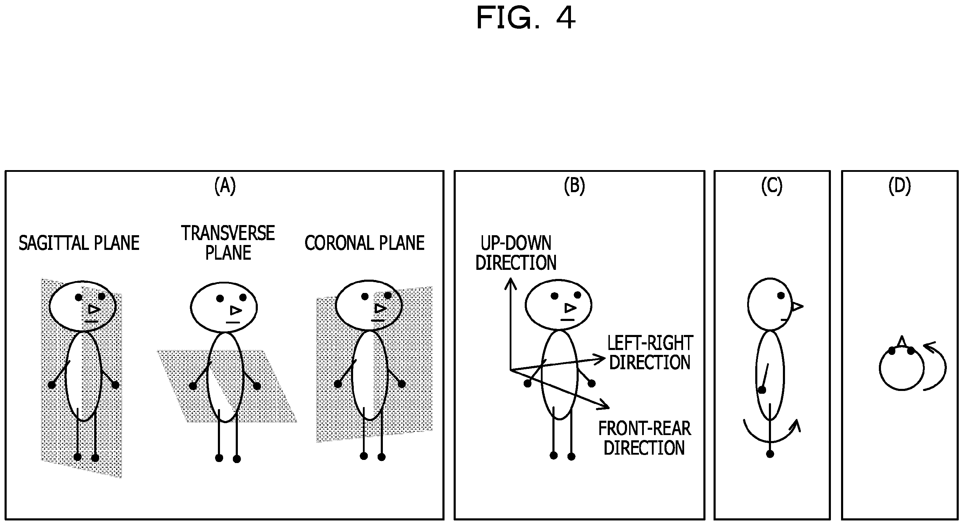

FIG. 4 illustrates respective directions such as a sagittal plane direction. FIG. 4(A) illustrates a sagittal plane direction, a transverse plane direction, and a coronal plane direction. FIG. 4(B) illustrates a front-rear direction, a left-right direction, and an up-down direction. FIG. 4(C) is a diagram when a subject is viewed from the right side, and indicates forward rotation in the sagittal plane direction. FIG. 4(D) is a diagram when a subject is viewed from the upper side, and indicates forward rotation in the transverse plane direction.

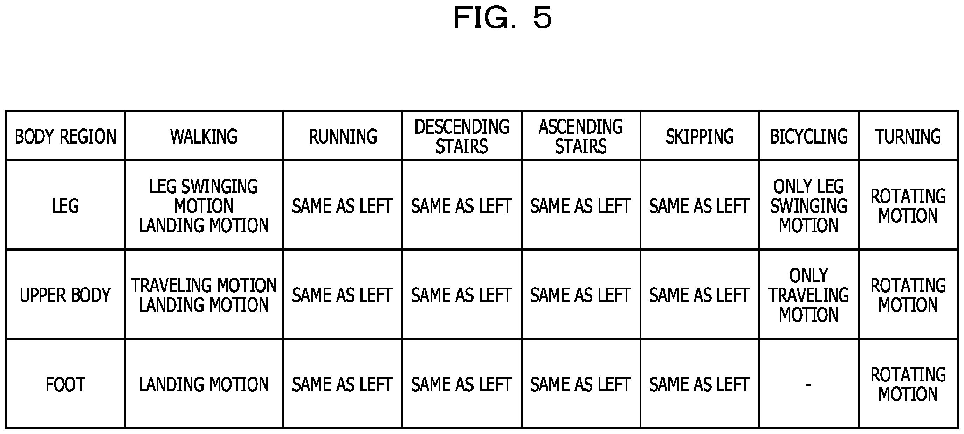

The natural motion varies in accordance with the activity type. Thus, the natural motion detection portion 115 detects the natural motion corresponding to the target activity type. FIG. 5 illustrates a natural motion that is detected per activity type and per body region. Regarding "body region" in FIG. 5, a leg may be a body region, such as a shank or a thigh, included in an area from buttocks to an ankle, or part of wear located at the same position as the position of that body region. An upper body may be a body region, such as a hip, a chest, a back, a head, an arm, or a wrist, included in an area above buttocks, or part of wear located at the same position as the position of that body region. Further, a foot may be a body region, such as a toe, a heel, an instep, or a sole, included in an area from an ankle to a toe, or part of wear located at the same position as the position of that body region. The wear represents clothing, a shoe, a wristwatch, eyeglasses, or the like.

The natural motion detection portion 115 attempts to detect a natural motion by a plurality of types of detection methods. For example, even for just "walking", the appearance of natural motion varies in accordance with various states, such as fast walking and slow walking. Parameters indicating conditions for detection (hereinafter, referred to as "detection parameters") are stored in the detection parameter storage unit 151 per detection method. In this embodiment, each detection method is referred to as "natural motion detector". The detector design portion 114 generates a plurality of natural motion detectors, such as a natural motion detector for detecting "fast walking" and a natural motion detector for detecting "slow walking", based on detection parameters. The natural motion detection portion 115 detects a natural motion based on each natural motion detector.

A motion that is detected as a natural motion by the natural motion detection portion 115 is in a strict sense a candidate for a natural motion. This is because the motion is not confirmed yet as a natural motion. A motion that is detected as a natural motion by the natural motion detection portion 115 is hereinafter referred to as "candidate motion". A set of candidate motions extracted from one candidate duration is referred to as "candidate motion group".

The inter-motion feature value extraction portion 116 extracts a feature value between candidate motions (hereinafter, referred to as "inter-motion feature value") for each candidate motion group detected by the natural motion detection portion 115. In this embodiment, as inter-motion feature values, 10 types of inter-motion feature values including left-right alternation degree, interval consistency degree, speed consistency degree, inverse-leg support degree, both-leg support degree, simultaneous movement degree, landing flexion degree, takeoff flexion degree, up-down alternation degree, and simultaneous impact degree are extracted.

The left-right alternation degree is an inter-motion feature value indicating performing leg swinging motions alternately with left and right legs. The left-right alternation degree is expressed with 1 or 0. Value 1 indicates that, when a candidate motion group is arranged in the order of the occurrence time point, a leg on the side belonging to a certain candidate motion differs from that of a preceding motion. Value 0 indicates that, when a candidate motion group is arranged in the order of the occurrence time point, a leg on the side belonging to a certain candidate motion is the same as that of a preceding motion.

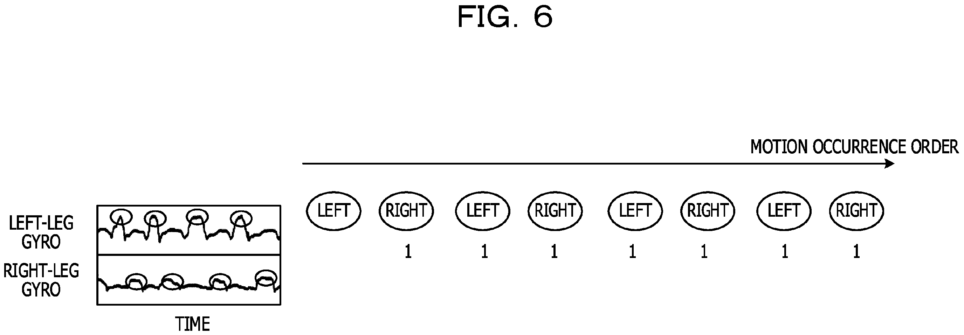

FIG. 6 is a diagram illustrating the left-right alternation degree. A left graph in FIG. 6 indicates gyro data in the sagittal plane direction of each of left and right legs. Areas surrounded by circles are peaks. FIG. 6 illustrates an example in which legs relating to respective candidate motions (peaks) are alternately moved. Thus, the left-right alternation degree of the respective candidate motions are 1. If left left and so on continues, the left-right alternation degree of at least the second left is 0.

The interval consistency degree is an inter-motion feature value indicating that the time intervals of leg swinging motions are approximately the same. The interval consistency degree is expressed as a numerical value indicating the degree of closeness of the time interval between successive candidate motions to a reference value.

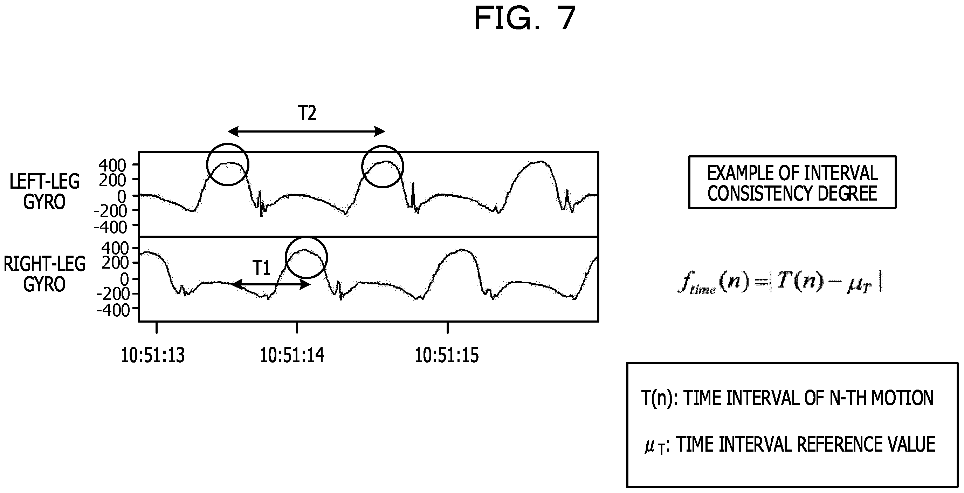

FIG. 7 is a diagram illustrating the interval consistency degree. The time interval between successive candidate motions represents a time interval (T1) between a certain motion and the next motion, or a time interval (T2) between a certain motion and the next motion on the same side. The reference value may be the mean of T1 or T2 in a candidate duration, or a previously determined constant. FIG. 7 illustrates on the right side an example of an arithmetic expression for the interval consistency degree.

The speed consistency degree is an inter-motion feature value indicating that the velocities of leg swinging motions are approximately the same. The speed consistency degree is expressed as a numerical value indicating the degree of closeness of the height of the peak of each candidate motion (hereinafter, referred to as "speed feature") to a reference value.

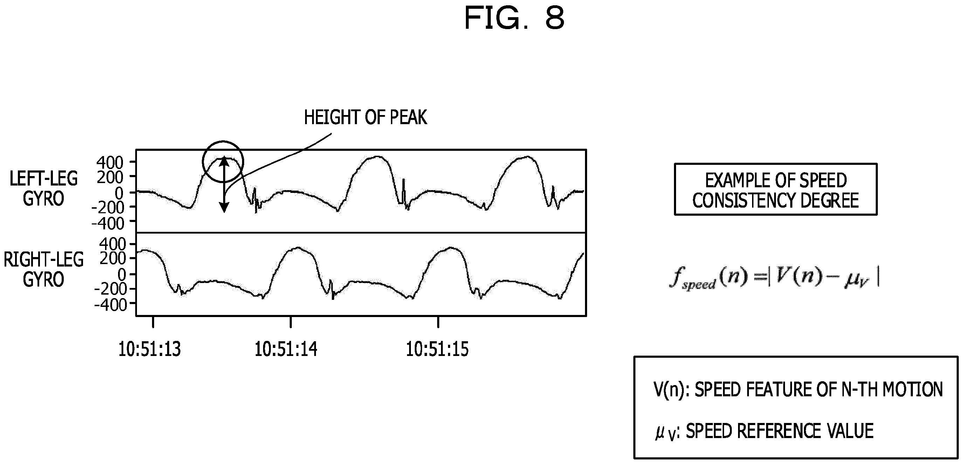

FIG. 8 is a diagram illustrating the speed consistency degree. FIG. 8 illustrates on the left side an example of the height of a peak. FIG. 8 illustrates on the right side an example of an arithmetic expression for the speed consistency degree. The reference value may be the mean of speed features in a candidate duration, or a previously determined constant.

The inverse-leg support degree is an inter-motion feature value indicating that, while one leg is in a leg swinging motion, the other leg is stopped. The inverse-leg support degree is expressed by the motion amount of the inverse leg in a neighborhood range close to the occurrence time point of each motion.

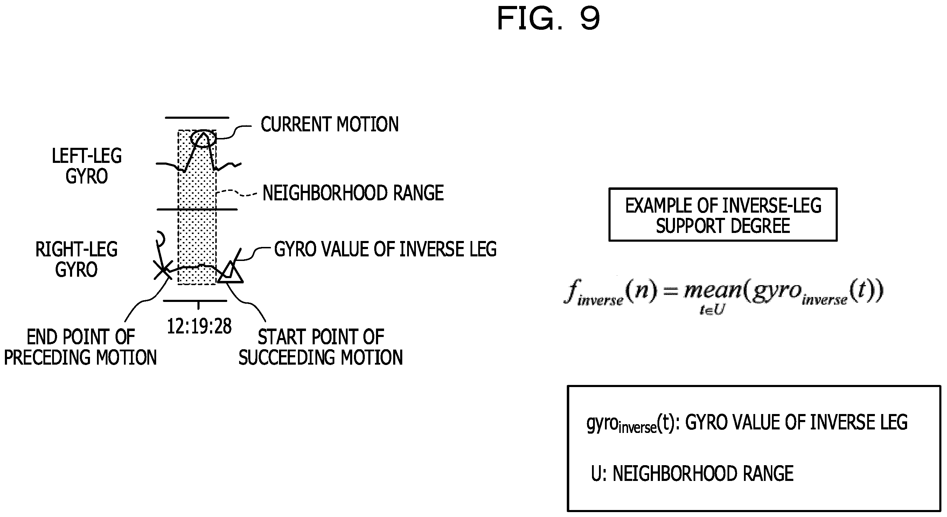

FIG. 9 is a diagram illustrating the inverse-leg support degree. The neighborhood range may be a duration before and after the candidate motion occurrence time point, or may be an intermediate range from an end point of a preceding motion to a start point of a succeeding motion. The motion amount is the mean, the maximum value, or the minimum value of gyro data in the sagittal plane direction or acceleration data in the front-rear direction. FIG. 9 illustrates on the right side an example of an arithmetic expression for the inverse-leg support degree.

The both-leg landing degree represents an inter-motion feature value indicating that a duration of standing still with both legs exists after landing. The both-leg landing degree is expressed by a duration length from when a certain candidate motion has been ended to when the next candidate motion is started.

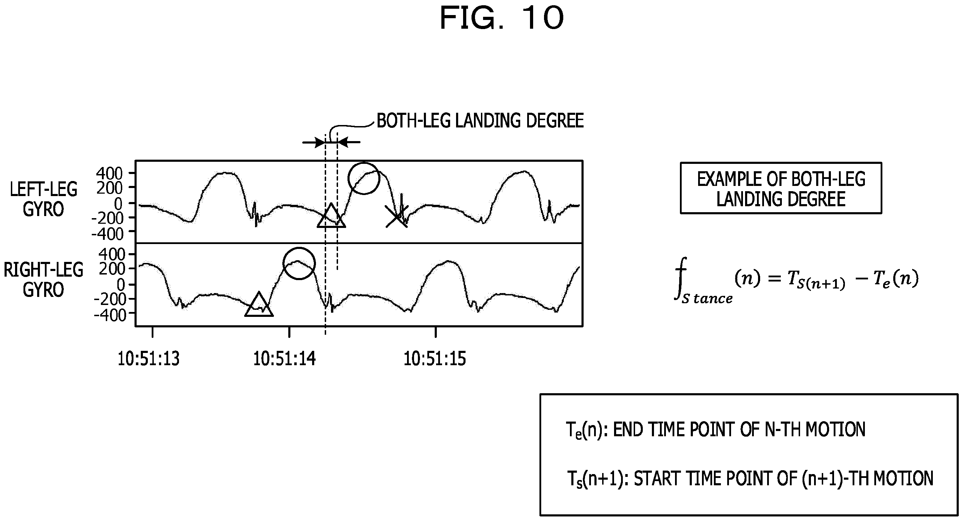

FIG. 10 is a diagram illustrating the both-leg landing degree. FIG. 10 illustrates on the left side that sign △ indicates a start time point of a candidate motion and sign × indicates an end time point of the candidate motion. Thus, the duration length from sign × of a preceding candidate motion to sign △ of a succeeding candidate motion is the both-leg landing degree. FIG. 10 illustrates on the right side an example of an arithmetic expression for the both-leg landing degree.

The simultaneous movement degree is an inter-motion feature value indicating appearance of a traveling motion of an upper body substantially simultaneously with a leg swinging motion of one leg. The simultaneous movement degree includes two features. A first feature is a minimum value $f_{upper1}$ of "upper-body advancement amount" in a neighborhood period at a landing time point. A second feature is a maximum value $f_{upper2}$ of "upper-body advancement amount" in a neighborhood period at a leg swinging time point of the inverse leg. The upper-body advancement amount is, for example, a value obtained by integrating acceleration data in the front-rear direction of the upper body, the mean of gyro data in the sagittal plane direction of the upper body or the like.

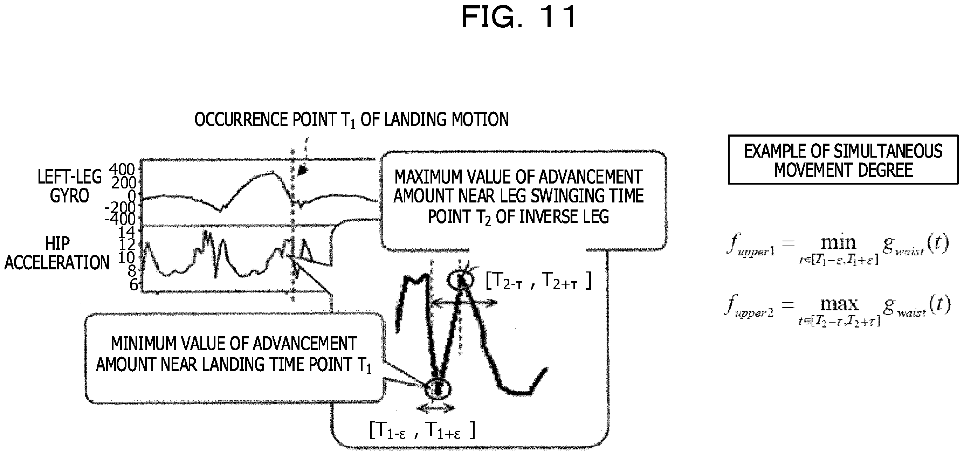

FIG. 11 is a diagram illustrating the simultaneous movement degree. FIG. 11 illustrates on the right side an example of an arithmetic expression for the simultaneous movement degree.

The landing flexion degree is an inter-motion feature value indicating that a knee is bent rearward immediately after a leg swinging motion. The landing flexion degree is expressed by a knee bending amount at the end time point of each candidate motion.

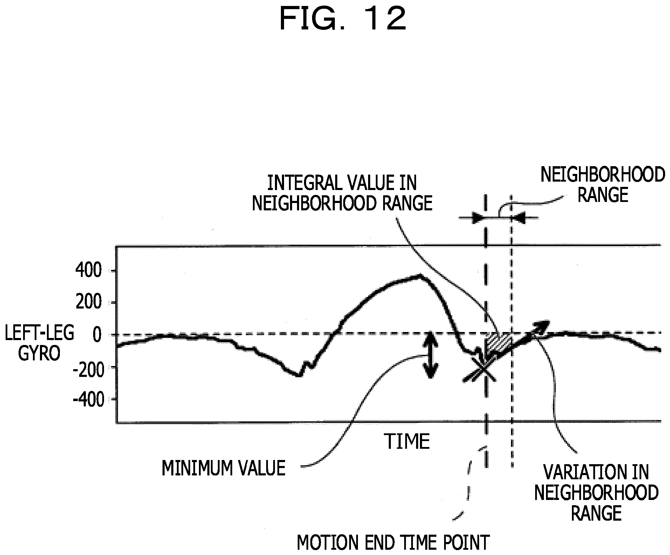

FIG. 12 is a diagram illustrating the landing flexion degree. The knee bending amount is a minimum value of sagittal-plane gyro of a leg, or a variation or an integral value in a neighborhood range of the minimum value.

The takeoff flexion degree is an inter-motion feature value indicating that a knee is bent rearward immediately before a leg swinging motion. The takeoff flexion degree is expressed by a knee bending amount at the start time point of each candidate motion.

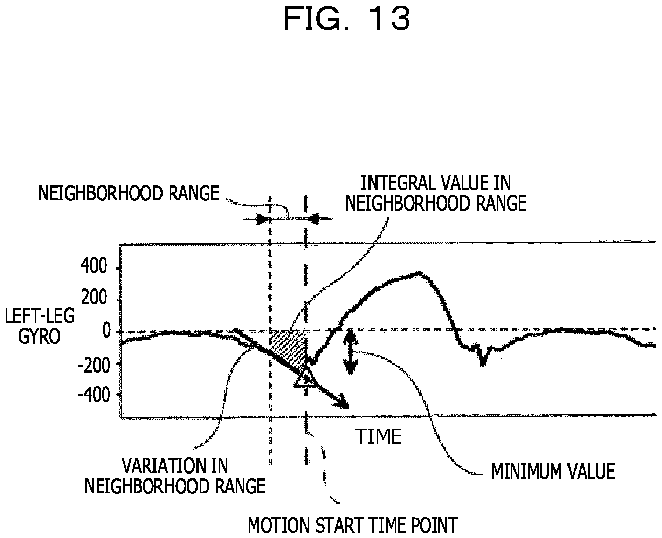

FIG. 13 is a diagram illustrating the takeoff flexion degree. The knee bending amount is a minimum value of sagittal-plane gyro of a leg, or a variation or an integral value in a neighborhood range of the minimum value.

The up-down alternation degree is a feature value indicating that advancement movement of an upper body and advancement movement of a lower body alternately appear. For example, during walking, motions of stepping one leg forward and then moving an upper body forward are alternately repeated between upper and lower body regions. The up-down alternation degree is calculated based on the timing of "landing motion" detected from acceleration data in the up-down direction of the upper body and the timing of "leg swinging motion" detected from gyro data in the sagittal plane direction of the one leg. For example, the number of times "landing motion of upper body" occurs in a period from when "leg swinging motion of one leg" is detected to when "next leg swinging motion" is detected represents the up-down alternation degree.

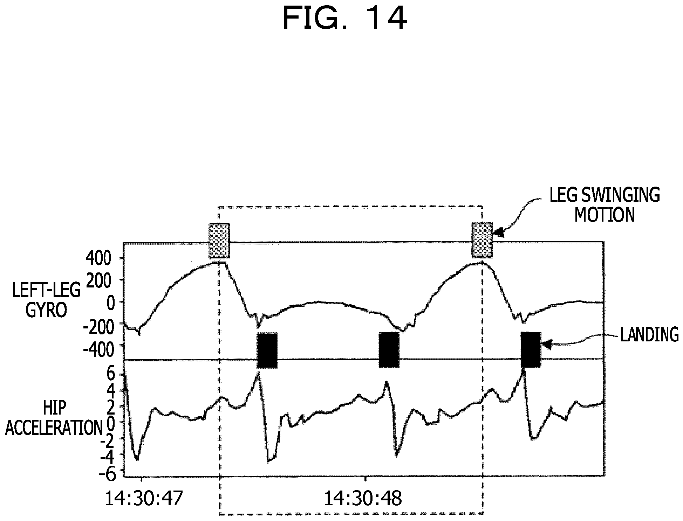

FIG. 14 is a diagram illustrating the up-down alternation degree. In the example in FIG. 14, landing occurs two times between the preceding leg swing motion and the succeeding leg swing motion, and hence the up-down alternation degree is 2.

The simultaneous impact degree is a feature value indicating that, when one leg is landed, an impact at landing occurs simultaneously at "a landing leg" and "an upper body". The simultaneous impact degree is calculated based on the timing of "landing motion 1" detected from acceleration in the up-down direction of the upper body and the timing of "landing motion 2" detected from acceleration in the up-down direction of the one leg. For example, the difference in occurrence time point between "the landing motion 2" and "the landing motion 1" whose occurrence time point is the closest to the occurrence time point of "the landing motion 2" is the simultaneous impact degree.

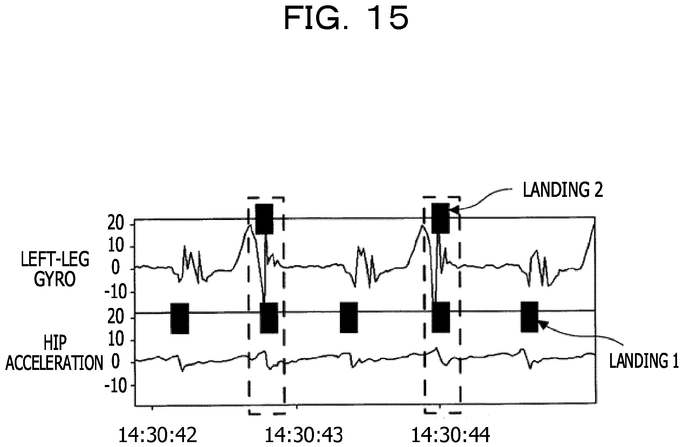

FIG. 15 is a diagram illustrating the simultaneous impact degree. In FIG. 15, the difference in occurrence time point between the landing 1 and the landing 2 surrounded by broken-line rectangles is the simultaneous impact degree.

The following description is given by referring back to FIG. 3. The feature likelihood calculation portion 117 converts an inter-motion feature value into a feature likelihood. The feature likelihood is an index indicating the degree of likelihood of an inter-motion feature value for a target activity type. Specifically, since the feature likelihood is a value dependent on the activity type, values which differ from one another individually for activity types may be calculated for the same inter-motion feature value. The rule storage unit 152 stores a rule for calculating the feature likelihood (hereinafter, referred to as "likelihood calculation rule") per activity type and per type of feature likelihood. The feature likelihood calculation portion 117 applies a likelihood calculation rule corresponding to a target activity type to an inter-motion feature value, and calculates a feature likelihood. In this embodiment, the value of the feature likelihood is in a range from 0 to 1.

The feature likelihood of the left-right alternation degree is referred to as left-right alternation likelihood. For example, if the target activity type is "walking", "it is likely that leg swinging motions are performed alternately with left and right legs during walking". Hence, a likelihood calculation rule with which the left-right alternation likelihood is high if the left-right alternation degree is 1 is applied.

The feature likelihood of the interval consistency degree is referred to as interval consistency likelihood. For example, if the target activity type is "walking", "it is likely that the time interval of leg swinging motions does not vary during walking". Hence, a likelihood calculation rule with which the interval consistency likelihood is higher as the interval consistency degree is lower is applied.

The feature likelihood of the speed consistency degree is referred to as speed consistency likelihood. For example, if the target activity type is "walking", "it is likely that the speed does not vary during walking". Hence, a likelihood calculation rule with which the speed consistency likelihood is higher as the speed consistency degree is lower is applied.

The feature likelihood of the inverse-leg support degree is referred to as inverse-leg support likelihood. For example, if the target activity type is "walking", "it is likely to "walking" that, when one leg swings, the inverse leg stays still (or slightly rotates backward) for support". Hence, a likelihood calculation rule with which the inverse-leg support likelihood is high as long as the inverse-leg support degree is in a predetermined range (for example, a negative constant range) is applied.

The feature likelihood of the both-leg landing degree is referred to as both-leg landing likelihood. If the target activity type is "walking", "it is likely that both legs are landed before performing a leg swinging motion". Hence, a likelihood calculation rule with which the both-leg landing likelihood is high if the both-leg landing degree is positive (and a predetermined value or smaller) is applied. In contrast, if the target activity type is "running" or "skipping", both legs are not simultaneously landed. Hence, a likelihood calculation rule with which the likelihood is high if the both-leg landing degree is negative is applied.

The feature likelihood of the simultaneous movement degree is referred to as simultaneous movement likelihood. For example, if the target activity type is "walking", "it is likely that an upper body immediately decelerates at landing so as not to fall, and then immediately accelerates simultaneously with a leg swinging motion of the inverse leg for advancement". Hence, a likelihood calculation rule with which the simultaneous movement likelihood is high if the first feature $f_{upper1}$ is equal to or smaller than a threshold and the second feature $f_{upper2}$ is equal to or larger than the threshold is applied.

The feature likelihood of the landing flexion degree is referred to as landing flexion likelihood. For example, if the target activity type is "walking", "it is likely that a knee is bent rearward at landing". Hence, a likelihood calculation rule with which the landing flexion likelihood is high if the landing flexion degree is high is applied. For example, if the minimum value of gyro data is negative, the landing flexion likelihood may be high. Alternatively, as the integral value of the gyro data is small, the landing flexion likelihood may be high. Still alternatively, as the variation of the gyro data is positive, the landing flexion likelihood may be high.

The feature likelihood of the takeoff flexion degree is referred to as takeoff flexion likelihood. For example, if the target activity type is "walking", "it is likely that a knee is bent rearward at takeoff". Hence, a likelihood calculation rule with which the feature likelihood is high if the takeoff flexion degree is high is applied. For example, if the minimum value of gyro data is negative, the takeoff flexion likelihood may be high. Alternatively, as the integral value of the gyro data is small, the takeoff flexion likelihood may be high. Still alternatively, as the variation of the gyro data is negative, the takeoff flexion likelihood may be high.

The feature likelihood of the up-down alternation degree is referred to as up-down alternation likelihood. For example, if the target activity type is "walking", "it is likely that both legs each are landed one time until the next leg swinging occurs". Hence, a likelihood calculation rule with which the up-down alternation likelihood is high if the up-down alternation degree is 2 is applied. Note that the state where the up-down alternation degree is 2 is a state where landing occurs two times before and after one leg swings. Hence, this state corresponds to a state where both legs each are landed one time until the next leg swinging occurs. In contrast, skipping has a feature that both legs each are landed two times. Hence, a likelihood calculation rule with which the up-down candidate likelihood is high if the up-down alternation degree is 4 is applied.

The feature likelihood of the simultaneous impact degree is referred to as simultaneous impact likelihood. For example, if the target activity type is neither "bicycling" nor "turning", "it is likely that the above-mentioned two of landing simultaneously occur. Hence, a likelihood calculation rule with which the simultaneous impact likelihood is high if the simultaneous impact degree is equal to or smaller than a threshold is applied.

Note that, if the target activity type is "running", the method for calculating the feature likelihood other than the both-leg landing likelihood is similar to that of "walking".

If the target activity type is "descending stairs", since a leg is largely bent rearward to descend stairs, the calculation threshold for the takeoff flexion likelihood is set to be larger than that during walking. The method of calculating other feature likelihood is similar to that of "walking".

If the target activity type is "ascending stairs", since a leg is largely bent rearward to ascend stairs, the calculation threshold for the takeoff flexion likelihood is set to be larger than that during walking. In this case, the region under the knee of the supporting leg is fixed and the thigh is bent to obtain a driving force forward, and hence the supporting leg tends not to be bent at landing. Hence, the likelihood is higher as the landing flexion degree is lower. The method of calculating other feature likelihood is similar to that of "walking".

If the target activity type is "bicycling", when one leg is brought forward, the inverse leg rotates rearward. Hence, the likelihood is obtained when the threshold range of the inverse-leg support degree is smaller than that during walking. When pedaling the bicycle, landing, taking off, and traveling motions do not exist, and hence the both-leg landing likelihood, simultaneous movement likelihood, landing flexion likelihood, takeoff flexion likelihood, up-down alternation likelihood, and simultaneous impact likelihood may not be calculated.

If the target activity type is "turning", the rotational speed and the rotational period may highly possibly vary. Hence, the interval consistency likelihood and speed consistency likelihood may not be calculated. When turning is performed, landing, taking off, and traveling motions are not related to turning, and hence the both-leg landing likelihood, simultaneous movement likelihood, landing flexion likelihood, takeoff flexion likelihood, up-down alternation likelihood, and simultaneous impact likelihood may not be calculated.

If the target activity type is "skipping", this is basically similar to "walking"; however, this differs from "walking" for the inverse-leg support likelihood and up-down alternation likelihood. Specifically, when "skipping" is performed, when one leg performs a leg swinging motion, the inverse leg jumps once, and the inverse leg rotates forward. Hence, the likelihood is obtained when the inverse-leg support degree is positive. Since landing occurs four times while a leg swinging motion of one leg occurs one time, the likelihood is obtained when the up-down alternation degree is 4.

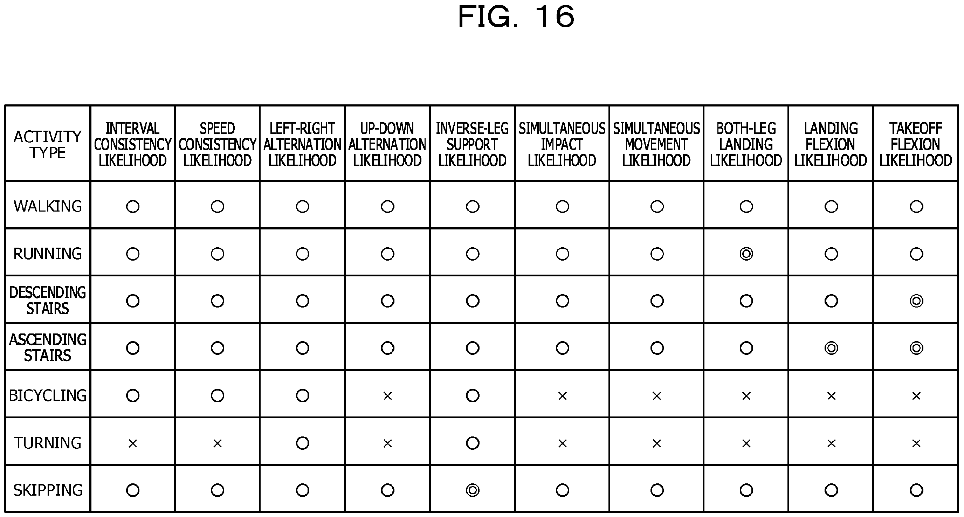

FIG. 16 illustrates a feature likelihood to be calculated per activity type. In FIG. 16, sign ○ indicates being a calculation target, and sign × indicates not being a calculation target. Sign ◎ indicates that the calculation method differs from that for "walking".

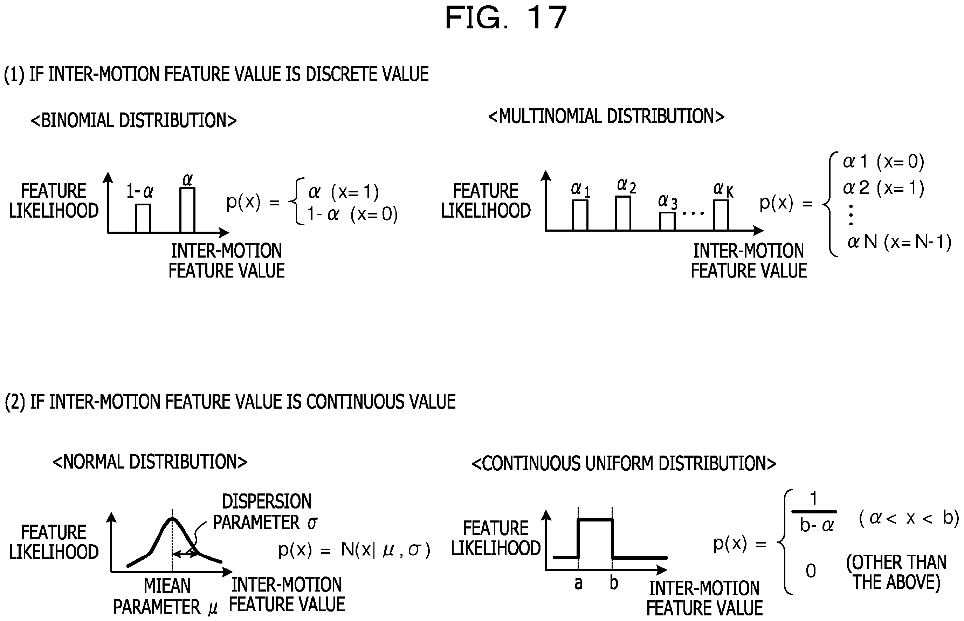

The likelihood calculation rule for converting the inter-motion feature value into the feature likelihood is defined, for example, based on the characteristics of the inter-motion feature value and the reference value, or for example, based on any of functions illustrated in FIG. 17. For example, to calculate the left-right alternation likelihood, a binomial distribution is used. To calculate the up-down alternation score, a multinomial distribution is used. To calculate the interval consistency likelihood, speed consistency likelihood, and inverse-leg support likelihood, a normal distribution is used. To calculate the inverse-leg support likelihood, both-leg landing likelihood, simultaneous movement likelihood, landing flexion likelihood, takeoff flexion likelihood, and simultaneous impact likelihood, a continuous uniform distribution is used. For the feature likelihood that is expressed by two or more parameters such as the simultaneous movement likelihood, the product of the results obtained by applying the likelihood calculation rules to each of the parameters may serve as a final feature likelihood.

The following description is given by referring back to FIG. 3. The natural pattern likelihood calculation portion 118 calculates a degree by which a natural pattern likely appears (hereinafter, referred to as "natural pattern likelihood") for each candidate motion group.

A natural pattern is an index that is defined by a combination of (1) similarity, (2) alternation, (3) simultaneity, and (4) ordering, for two or more natural motions.

The similarity of natural motions represents that similarity is established in the speed feature of "any one of natural motions" at one of left and right sides. The similarity of natural motions represents that similarity is established in the time interval feature of "any one of natural motions" at one of left and right sides. Thus, the similarity of natural motions may be evaluated based on the speed consistency likelihood and interval consistency likelihood.

The alternation of natural motions represents that alternation is established in "a leg swinging motion, or a rotating motion, or a landing motion" of a right leg, and "a leg swinging motion, or a rotating motion, or a landing motion" of a left leg. Also, the alternation of natural motions represents that "a traveling motion of an upper body" and "a leg swinging motion of a lower body" alternately appear. Thus, the alternation of natural motions may be evaluated based on the left-right alternation likelihood and up-down alternation likelihood.

The simultaneity of natural motions represents that when a leg swinging motion of one leg appears, stationarity of the inverse leg is in a predetermined range. Also, the simultaneity of natural motions represents that a leg swinging motion of one leg and a traveling motion of an upper body simultaneously occur in a predetermined period. Further, the simultaneity of natural motions represents that impacts of landing motions simultaneously occur at a plurality of regions in a predetermined period. Thus, the simultaneity of natural motions may be evaluated based on the inverse-leg support likelihood, simultaneous movement likelihood, and simultaneous impact likelihood.

The ordering of natural motions represents that the duration in which both legs are landed is in a predetermined range. Also, the ordering of natural motions represents that a change in posture after a landing motion occurs is in a predetermined range. Further, the ordering of natural motions represents that a change in posture before a leg swinging motion is started is in a predetermined range. Thus, the ordering of natural motions may be evaluated based on the both-leg landing likelihood, landing flexion likelihood, and takeoff flexion likelihood.

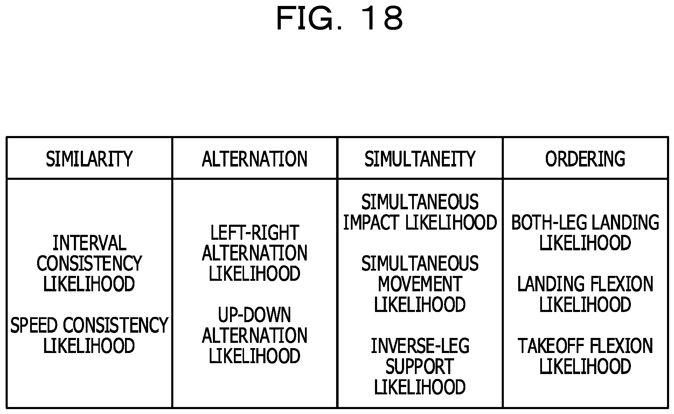

FIG. 18 illustrates correlation between respective characteristics (similarity, alternation, simultaneity, ordering) of natural patterns and respective feature likelihoods. As illustrated in FIG. 18, the natural pattern likelihood may be evaluated by each feature likelihood. In other words, to calculate the natural pattern likelihood, the feature likelihood is calculated.

In this way, the similarity, alternation, simultaneity, and ordering of natural patterns may be evaluated based on each feature likelihood. Thus, the natural pattern likelihood calculation portion 118 calculates the natural pattern likelihood of each candidate motion group based on each feature likelihood.

Note that which feature likelihood is used for evaluation of a natural pattern likelihood is changed in accordance with the body regions to which sensors are attached. For example, when sensors are attached to left and right legs, the left-right alternation likelihood, interval consistency likelihood, speed consistency likelihood, inverse-leg support likelihood, both-leg landing likelihood, landing flexion likelihood, and takeoff flexion likelihood are used.

The natural pattern likelihood depends on the feature likelihood. The feature likelihood depends on the inter-motion feature value. The inter-motion feature value is detected per natural motion detector. Thus, the feature likelihood and the natural pattern likelihood are calculated per natural motion detector (per detection method included in mutually different detection methods) for the target activity type.

The following description is given by referring back to FIG. 3. The detector selection portion 119 selects the maximum value from natural pattern likelihoods of each natural motion detector for each candidate duration. The detector selection portion 119 recognizes the candidate duration and the candidate detector with the maximum value exceeding a threshold, as the activity duration and the detector of the target activity type. However, when a plurality of activity types are recognized as determination targets, the recognition of the activity duration by the detector selection portion 119 does not represent confirmation of the activity duration.

The activity duration comparison portion 120 confirms the activity type for each candidate duration. When a plurality of activity types are recognized for one candidate duration, the activity duration comparison portion 120 selects the activity type the natural pattern likelihood of which is the highest among the plurality of activity types, as the activity type for the candidate duration. Alternatively, specific selection processing may be performed in accordance with the combination of the recognized activity types. For example, when walking and turning are recognized for one candidate section, both are output. For example, when walking and bicycling are recognized for one candidate section, one of both is output.

The following describes a procedure that is executed by the activity duration determination apparatus 10. Before the procedure to be explained as follows is executed, it is assumed that the left-leg sensor device 30L and the right-leg sensor device 30R are attached to left and right ankles of a subject and sensor data obtained by these sensor devices are collected by the data collection unit 11. For example, the sensor data may be collected by coupling the sensor devices with the activity duration determination apparatus 10 using dedicated cables. The timing at which the sensor data is collected is desirably determined. For example, the sensor data may be periodically collected once a day. The collected sensor data is stored in, for example, the auxiliary storage device 102.

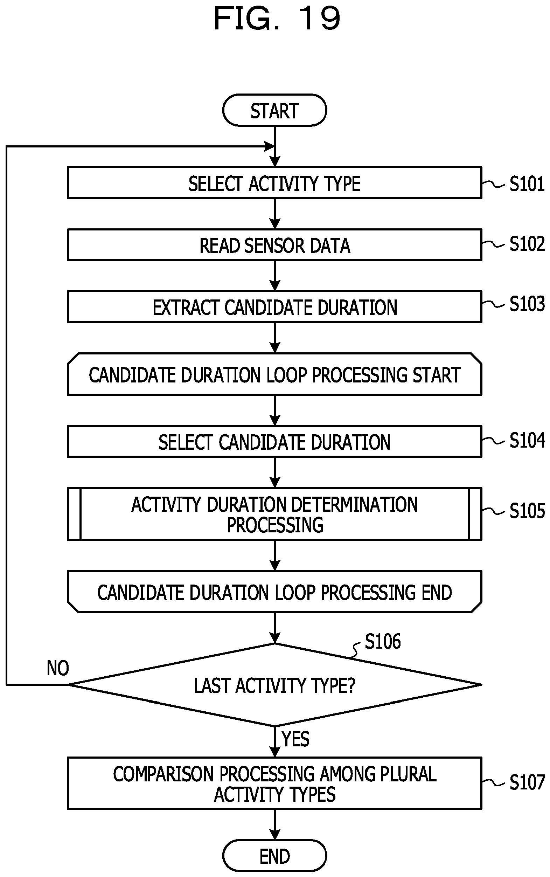

FIG. 19 is a flowchart illustrating an example of a procedure that is executed by the activity duration determination apparatus according to the first embodiment.

In step S101, the activity type selection portion 111 selects a target activity type. For example, one activity type is sequentially selected as a target activity type from activity types that are previously set as determination targets by a user.

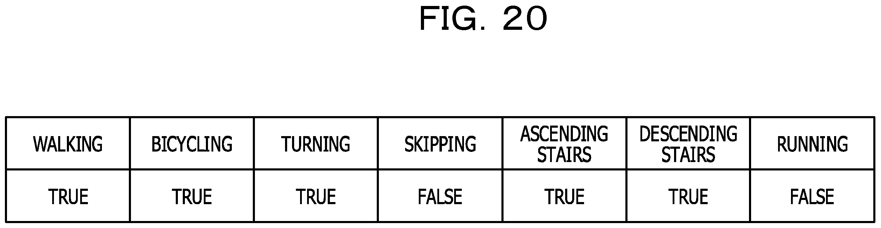

FIG. 20 is a diagram illustrating a setting example of activity types of determination targets. FIG. 20 illustrates an example in which "TRUE" or "FALSE" is set to each activity type. "TRUE" represents being a determination target. "FALSE" represents not being a determination target. Referring to the example in FIG. 20, "walking" is selected first as a target activity type.

Then, the data reading portion 112 reads sensor data stored in the auxiliary storage device 102 (S102). For example, for tri-axis sensor device, gyro data or acceleration data is stored in time series according to each direction of the sagittal plane direction, coronal plane direction, and transverse plane direction. The sensor data read in step S102 may be all sensor data, or the reading target may be changed in accordance with the target activity type. For example, for "walking", gyro data representing rotation in the sagittal plane direction for both left and right may be read. For "running", "bicycling", "ascending stairs", and "descending stairs", gyro data representing rotation in the sagittal plane direction may be read. For "turning", gyro data representing rotation in the transverse plane direction may be read.

Then, the candidate duration extraction portion 113 extracts a candidate duration based on the sensor data (S103). For example, the candidate duration extraction portion 113 extracts a duration in a non-stationary state as a candidate duration from the sensor data. For example, the candidate duration extraction portion 113 performs segmentation on left and right sensor data (gyro) while a window with a window width W is shifted in a time-axis direction by a shift width (W/2). The candidate duration extraction portion 113 recognizes a segment in which a mean Mg and a dispersion Vg of left and right gyro data respectively exceed predetermined thresholds TH_Mg and TH_Vg, as "a non-stationary segment". The candidate duration extraction portion 113 performs such processing on the sensor data from the beginning to end, then integrates continuous non-stationary segments, and hence obtains a single non-stationary duration. Consequently, each non-stationary duration is treated as a candidate duration.

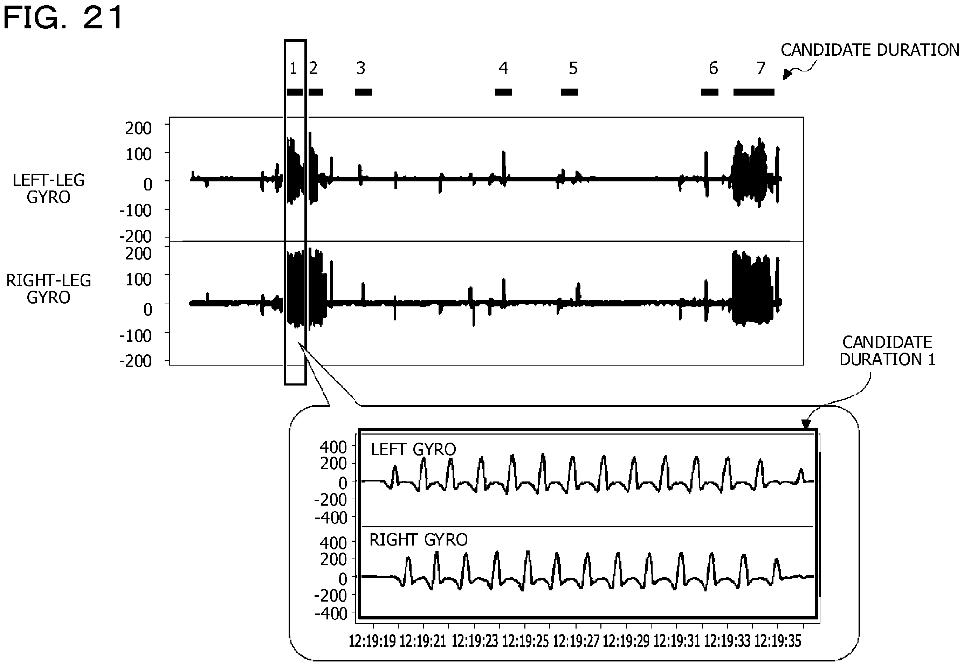

FIG. 21 is a diagram illustrating an extraction example of a candidate duration. In FIG. 21, gyro data in the sagittal plane direction of the left leg and gyro data in the sagittal plane direction of the right leg are arranged at upper and lower sides with the time points synchronized. In this case, strips displayed above the gyro data and provided with numerical values from 1 to 7 indicate candidate durations.

Then, steps S104 and S105 are executed per candidate duration. In step S104, the activity duration determination unit 13 selects, for example, one candidate duration from the candidate durations in order from the earliest candidate duration, and cuts out sensor data (gyro data) in the selected candidate duration. FIG. 21 illustrates an example in which gyro data in a candidate duration 1 is cut out. Hereinafter, the selected candidate duration is referred to as "target candidate duration".

Then, activity duration determination processing is executed for the target activity type and the target candidate duration (S105).

When steps S104 and S105 are executed for all candidate durations, the activity type selection portion 111 determines whether the target activity type is the last activity type among the activity types of determination targets (S106). If the target activity type is not the last activity type among the activity types of determination targets (No in S106), step S101 and later steps are repeated. If the target activity type is the last activity type among the activity types of determination targets (Yes in S106), the activity duration comparison portion 120 selects one activity type based on the natural pattern likelihood of each activity type for the candidate duration from which a plurality of activity types are recognized (S107). Consequently, the activity type of each candidate duration is confirmed.

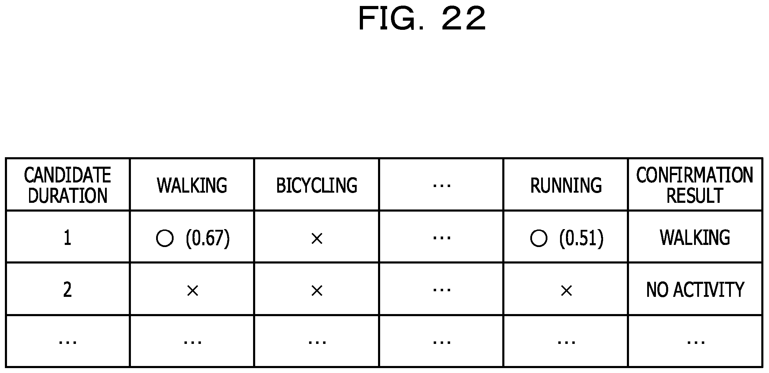

FIG. 22 is a diagram illustrating an example of confirmation results of activity types of each candidate duration. FIG. 22 illustrates the determination results of each activity type, and the confirmation results of the activity type per candidate duration. In FIG. 22, sign O indicates that the natural pattern likelihood exceeds a threshold, and sign × indicates that the natural pattern likelihood is equal to or smaller than the threshold. The numerical value in brackets next to sign O indicates the natural pattern likelihood. For example, in the candidate duration 1, the natural pattern likelihoods of "walking" and "running" exceed the thresholds. In this case, the activity duration comparison portion 120 selects "walking" with the higher natural pattern likelihood, as the activity type for the candidate duration 1. In contrast, in the candidate duration 2, there is no activity type whose natural pattern likelihood exceeds a threshold. In this case, the activity duration comparison portion 120 confirms that the candidate duration 2 is not an activity duration. For the candidate duration whose natural pattern likelihood of only one activity type exceeds a threshold, the activity duration comparison portion 120 confirms that activity type as the activity type for the candidate duration.

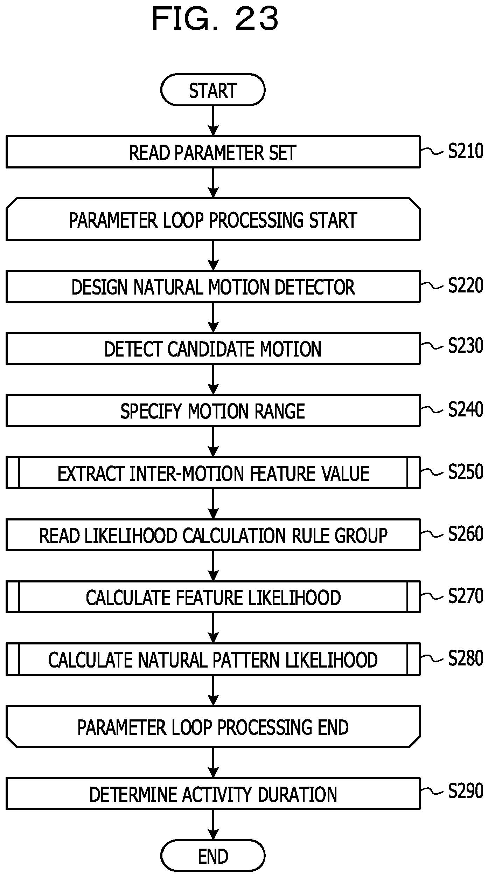

Subsequently, the details of step S105 will be described. FIG. 23 is a flowchart illustrating an example of a procedure for activity duration determination processing according to the first embodiment.

In step S210, the natural motion detection portion 115 reads a parameter list group (hereinafter, referred to as "parameter set") per natural motion detector corresponding to the target activity type, from a parameter storage unit.

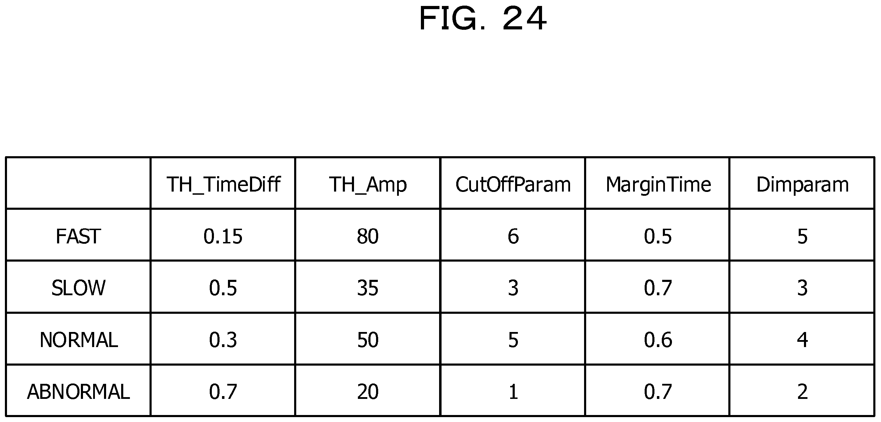

FIG. 24 is a diagram illustrating an example of a parameter set. In FIG. 24, the row direction corresponds to the types of natural motion detectors. FIG. 24 illustrates an example in which four types of natural motion detectors including "Fast", "Slow", "Normal", and "Abnormal" are set. "Fast" is a natural motion detector for detecting a fast walking state. "Slow" is a natural motion detector for detecting a slow walking state. "Normal" is a natural motion detector for detecting a walking state at normal speed. "Abnormal" is a natural motion detector for detecting an abnormal walking state.

The column direction corresponds to detection parameters that constitute a parameter list. The detection parameters include TH_TimeDiff, TH_Amp, CutOffParam, MarginTime, and Dimparam. The meanings and purposes of the detection parameters will be described later.

In this way, since the plurality of natural motion detectors are prepared for one activity type, the possibility of an omission of detecting a natural motion may be reduced as compared with detection of a natural motion by one type of detection method.

Then, steps S220 to S280 are loop processing per parameter list (that is, per natural motion detector). Hereinafter, a parameter list of a processing target is referred to as "target parameter list".

In step S220, the detector design portion 114 designs a natural motion detector based on a target parameter list. Designing the natural motion detector represents setting a filter pass band for sensor data (gyro data).

Specifically, when a motion of stepping a leg forward is performed in any type of two-leg motion, an acceleration and deceleration motion of a certain region of a leg occurs in a certain direction. Based on this, by detecting a peak from sensor data in a target candidate duration, the motion may be detected. A peak is detected by applying a band-pass filter to the sensor data, hence shaping the gyro waveform, and making a threshold determination at a peak.

The detector design portion 114 sets the filter pass band of the band-pass filter based on a cutoff frequency CutoffParam and a filter order DimParam that are detection parameters of the target parameter list. In ordinary situations, to determine slow walking, the filter pass band is desirably set low, and to determine fast walking, the filter pass band is desirably set high.

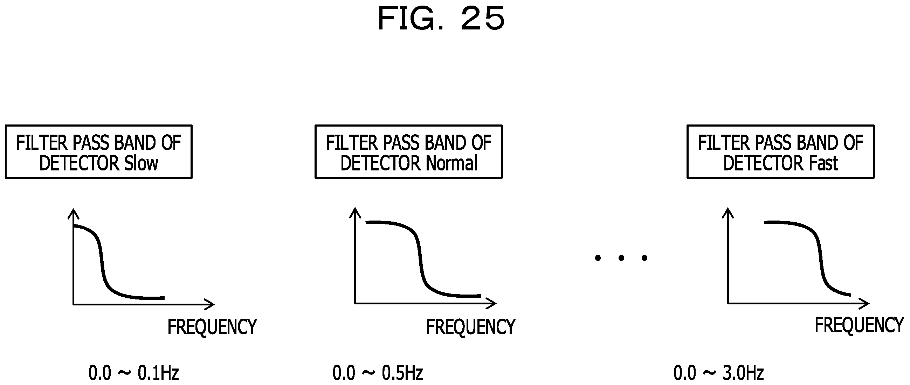

FIG. 25 includes diagrams each illustrating an example of a filter pass band. FIG. 25 illustrates a plurality of filter pass bands. This indicates that the filter pass bands differ from one another individually for the respective natural motion detectors. Thus, in step S220, one filter pass band is set.

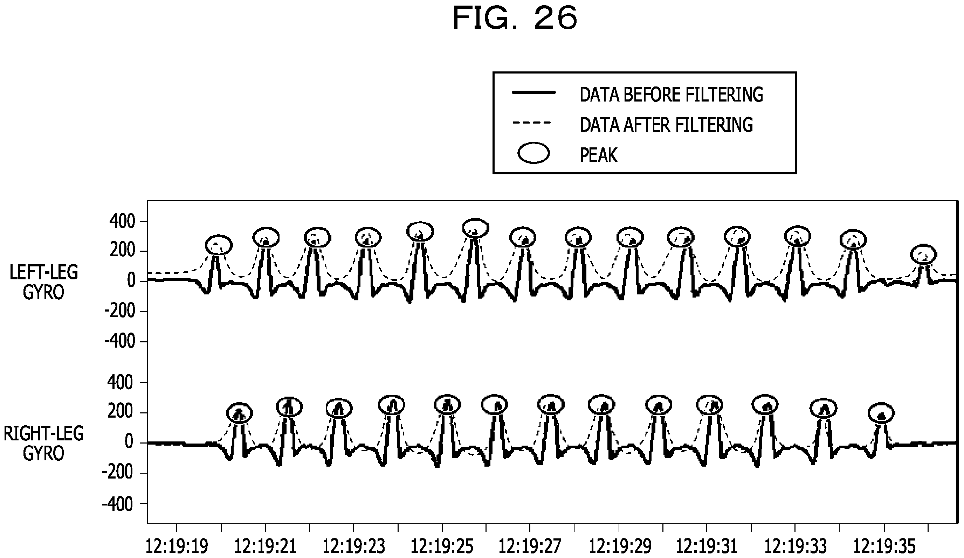

Then, the natural motion detection portion 115 detects a candidate motion (S230). For example, the natural motion detection portion 115 executes band-pass filtering on the sensor data of the target candidate duration, with the filter pass band set in step S220. Consequently, the sensor data is converted into data D in a predetermined frequency range. Further, the natural motion detection portion 115 performs peak detection on the filtered data D. During the peak detection, the natural motion detection portion 115 first searches for a portion of the data D with a derivative value being 0. The natural motion detection portion 115 determines that a peak occurs if a data value $D(t)$ at a portion of the data D with a derivative value being 0 exceeds a threshold TH_Amp that is one of the detection parameters, and sets t to a timing at which a candidate motion occurs. FIG. 26 illustrates an example of sensor data and peaks before and after filtering.

The natural motion detection portion 115 performs such processing on, for example, each of left-leg gyro and right-leg gyro. Since a left-right asymmetric motion may be possibly performed, the parameter list of the natural motion detectors and the threshold TH_Amp for the peak detection may be individually set for each of left and right.

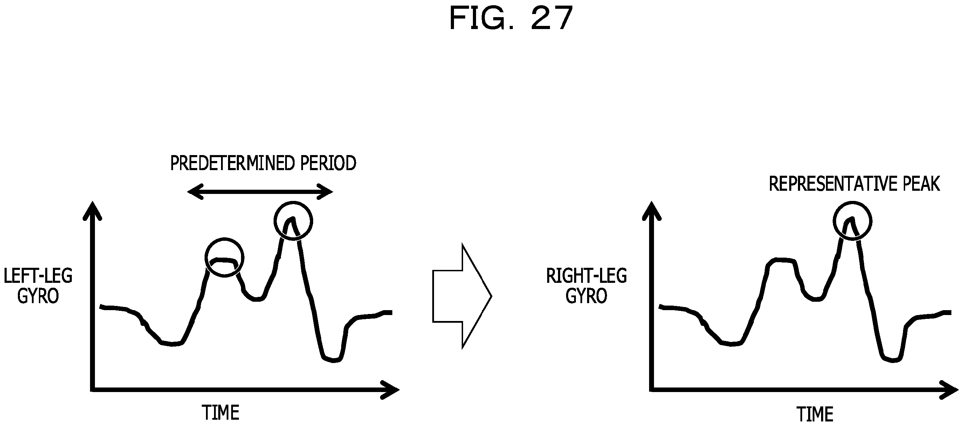

As illustrated in FIG. 27, when a plurality of peaks occur during a predetermined period TH_TimeDiff that is one of the detection parameters, the peaks may be grouped as one peak. In this case, the peak with the highest gyro value among the plurality of peaks serves as a representative peak.

Then, the inter-motion feature value extraction portion 116 specifies a motion range for each candidate motion (each peak) detected in the target candidate duration (S240). The motion range for the candidate motion represents a duration from the start point to the end point of the candidate motion.

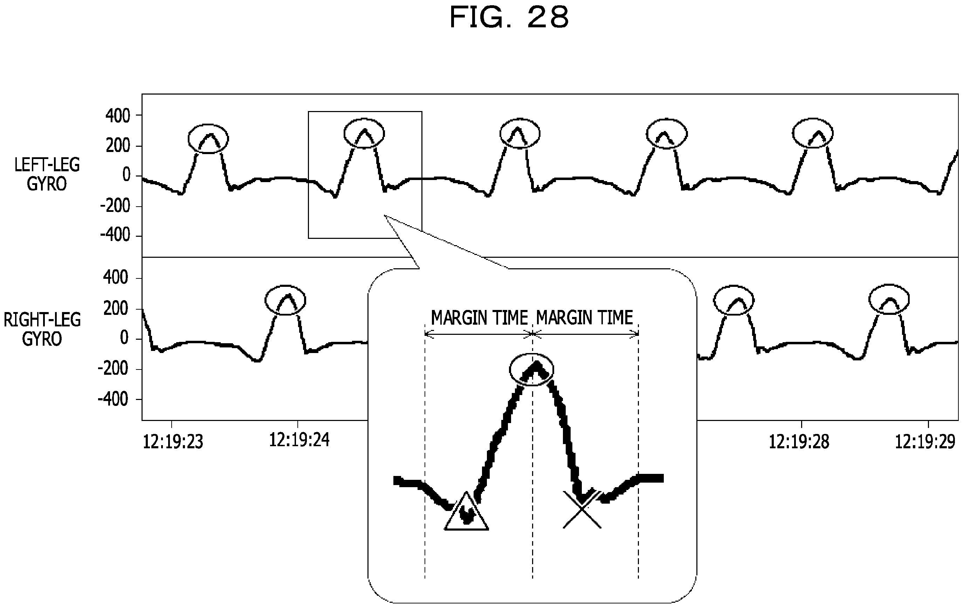

FIG. 28 is a diagram illustrating an example of a method of specifying a motion range. FIG. 28 illustrates an example in which a motion range is specified for the candidate motion second to the left. As illustrated in FIG. 28, the time point at which the gyro value is the minimum in the range from the peak occurrence point to a time point before a predetermined period MarginTime that is one of the detection parameters serves as the start point. The time point at which the gyro value is the minimum in the range from the peak occurrence point to a time point after the predetermined period MarginTime serves as the end point.

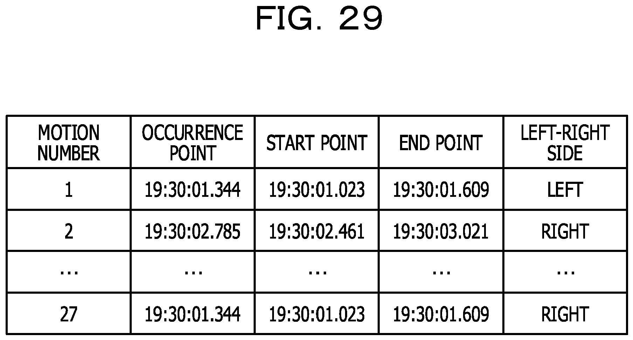

Since the motion range is specified for each candidate motion in the target candidate duration, information illustrated in FIG. 29 may be obtained for each candicate motion.

FIG. 29 is a diagram illustrating information obtained by specifying a motion range. In FIG. 29, motion number is an identification number of a candidate motion. Occurrence point is an occurrence time point of a candidate motion. Start point is a start time point of a candidate motion. End point is an end time point of a candidate motion. Left-right side is information indicating which one of left and right legs performs a candidate motion.

Then, the inter-motion feature value extraction portion 116 executes processing of extracting an inter-motion feature value from a candidate motion group in a target candidate duration (S250).

Then, the feature likelihood calculation portion 117 reads a likelihood calculation rule group corresponding to the target activity type from the rule storage unit 152 (S260).

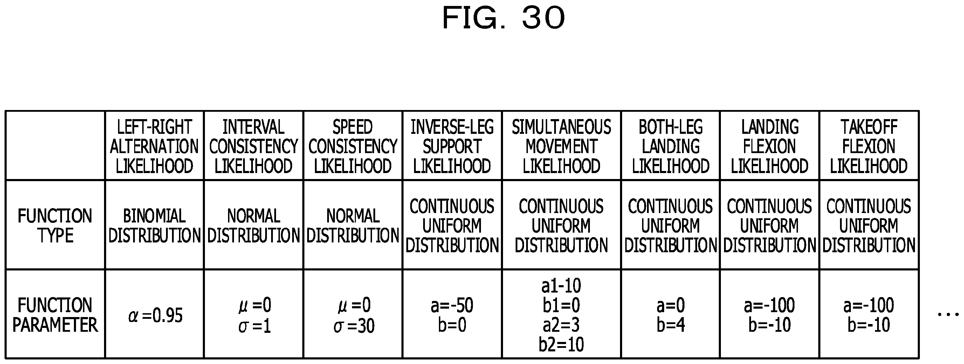

FIG. 30 is a diagram illustrating an example of a likelihood calculation rule group. FIG. 30 illustrates an example in which each likelihood calculation rule includes a function type and a function parameter. The function type indicates a type of function that is a likelihood calculation rule. The function parameter is a parameter that defines the function, foe example, the parameter illustrated in FIG. 17.

Then, the feature likelihood calculation portion 117 applies each likelihood calculation rule read in step S260 to each inter-motion feature value extracted in step S250, and hence calculates a feature likelihood (S270).

Then, the natural pattern likelihood calculation portion 118 calculates a natural pattern likelihood for the target candidate duration based on the feature likelihood calculated in step S270 (S280).

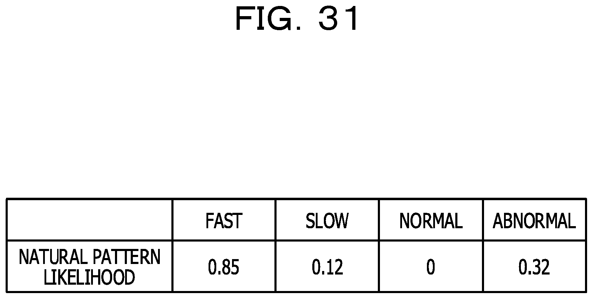

When steps S220 to S280 are executed on all natural motion detectors, as illustrated in FIG. 31, the natural pattern likelihood is calculated per natural motion detector, for the target activity type and the target candidate duration. Then, the detector selection portion 119 determines an activity duration and a detector for the target candidate duration (S290). For example, the detector selection portion 119 selects the maximum value from the natural pattern likelihoods for the respective natural motion detectors, and if the maximum value is larger than a threshold, determines that the target candidate duration is the activity duration of the target activity type. The detector selection portion 119 outputs (stores) the start time point and the end time point of the determined activity duration, and the identification information on the employed natural motion detector to (in) the determination result storage unit 15. The employed natural motion detector indicates the natural motion detector that is used for calculating the natural pattern likelihood relating to the maximum value larger than the threshold.

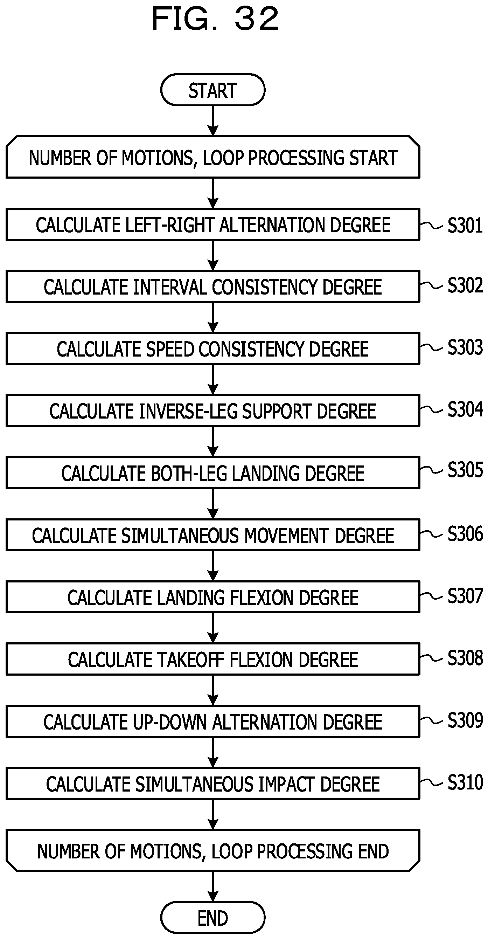

Subsequently, the details of step S250 will be described. FIG. 32 is a flowchart illustrating an example of a procedure for processing of extracting an inter-motion feature value.

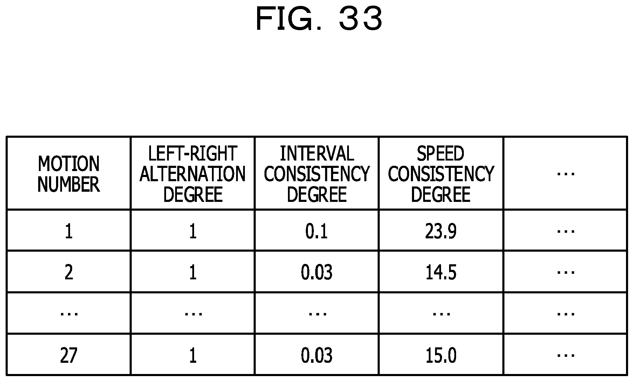

As illustrated in FIG. 32, the inter-motion feature value extraction portion 116 calculates, per candidate motion detected in the target candidate duration, the left-right alternation degree, interval consistency degree, speed consistency degree, inverse-leg support degree, both-leg landing degree, simultaneous movement degree, landing flexion degree, takeoff flexion degree, up-down alternation degree, and simultaneous impact degree (S301 to S310). Consequently, as illustrated in FIG. 33, respective inter-motion feature values are obtained per candidate motion. Note that the method of calculating each inter-motion feature value is as described above. Alternatively, one or some of inter-motion feature values may not be calculated in accordance with the target activity type or the collected sensor data.

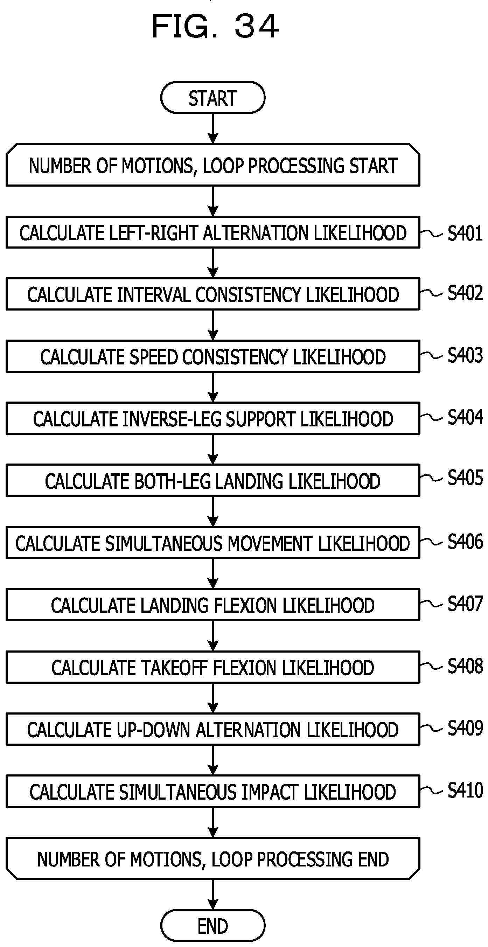

Subsequently, the details of step S270 in FIG. 23 will be described. FIG. 34 is a flowchart illustrating an example of a procedure for processing of calculating a feature likelihood.

The feature likelihood calculation portion 117 executes steps S401 to S410 per candidate motion detected in the target candidate duration. Hereinafter, the candidate motion serving as the processing target is referred to as "target motion".

In step S401, the feature likelihood calculation portion 117 calculates the left-right alternation likelihood of the target motion by applying the likelihood calculation rule of the left-right alternation likelihood to the left-right alternation degree of the target motion.

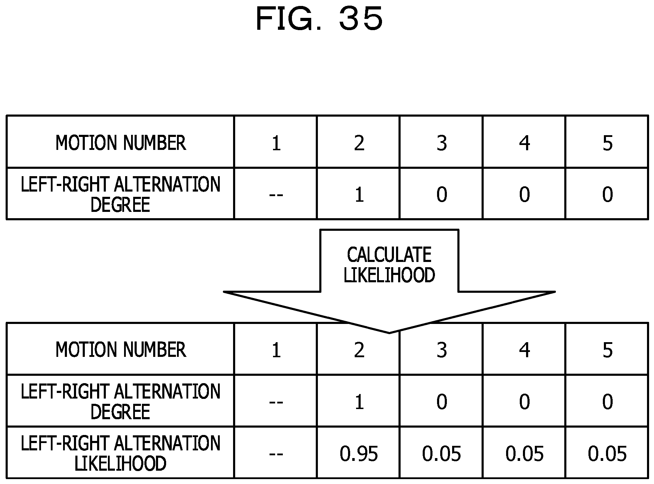

FIG. 35 is a diagram illustrating a calculation example of the left-right alternation likelihood. Referring to FIG. 30, since the function type of the likelihood calculation rule of the left-right alternation likelihood is a binomial distribution, the left-right alternation likelihood ($L(f_{lr})$) is calculated based on a probability function formula of a binomial distribution in FIG. 17. In this case, a which is a function parameter is 0.95. Thus, the left-right alternation likelihood of the candidate motion whose left-right alternation degree is "1" is 0.95, and the left-right alternation likelihood of the candidate motion whose left-right alternation degree is "0" is 0.05. FIG. 35 illustrates an example of calculating left-right alternation likelihoods for five candidate motions; however, a left-right alternation likelihood is calculated for one candidate motion by execution of single step S401.

Then, the feature likelihood calculation portion 117 calculates the interval consistency likelihood of the target motion by applying the likelihood calculation rule of the interval consistency likelihood to the interval consistency degree of the target motion (S402). The interval consistency likelihood ($L(f_{time})$) is calculated based on, for example, an expression as follows. Note that $f_{time}$ indicates an interval consistency degree. N indicates a normal distribution.

[Math. 1]

$$L(f_{time}) = N(f_{time} \mid \mu = 0, \sigma = 1)$$

Then, the feature likelihood calculation portion 117 calculates the speed consistency likelihood of the target motion by applying the likelihood calculation rule of the speed consistency likelihood to the speed consistency degree of the target motion (S403). The speed consistency likelihood ($L(f_{speed})$) is calculated based on, for example, an expression as follows. Note that $f_{speed}$ indicates a speed consistency degree.

[Math. 2]

$$L(f_{speed}) = N(f_{speed} \mid \mu = 0, \sigma = 20)$$

Then, the feature likelihood calculation portion 117 calculates the inverse-leg support likelihood of the target motion by applying the likelihood calculation rule of the inverse-leg support likelihood to the inverse-leg support degree of the target motion (S404). The inverse-leg support likelihood ($L(f_{inverse})$) is calculated based on, for example, an expression as follows. Note that $f_{inverse}$ indicates an inverse-leg support degree.

[Math. 3]

$$L(f_{inverse}) = \begin{cases} 0.02 & (-50 < f_{inverse} < 0) \\ 0 & \text{(other than the above)} \end{cases}$$

Then, the feature likelihood calculation portion 117 calculates the both-leg landing likelihood of the target motion by applying the likelihood calculation rule of the both-leg landing likelihood to the both-leg landing degree of the target motion (S405). The both-leg landing likelihood ($L(f_{stance})$) is calculated based on, for example, an expression as follows. Note that $f_{stance}$ indicates a both-leg landing degree.

[Math. 4]

$$L(f_{stance}) = \begin{cases} 0.25 & (0 < f_{stance} < 4) \\ 0 & \text{(other than the above)} \end{cases}$$

Then, the feature likelihood calculation portion 117 calculates the simultaneous movement likelihood of the target motion by applying the likelihood calculation rule of the simultaneous movement likelihood to the simultaneous movement degree of the target motion (S406). The simultaneous movement likelihoods ($L(f_{upper1}, f_{upper2})$) are calculated based on, for example, expressions as follows. Note that $f_{upper1}$ indicates a first feature of the simultaneous movement degree, and $f_{upper2}$ indicates a second feature of the simultaneous movement degree.

[Math. 5]

$$L(f_{upper1}, f_{upper2}) = L(f_{upper1}) \times L(f_{upper2})$$

$$L(f_{upper1}) = \begin{cases} 0.1 & (-10 < f_{upper1} < 0) \\ 0 & \text{(other than the above)} \end{cases}$$

$$L(f_{upper2}) = \begin{cases} 0.14 & (3 < f_{upper2} < 10) \\ 0 & \text{(other than the above)} \end{cases}$$

Then, the feature likelihood calculation portion 117 calculates the landing flexion likelihood of the target motion by applying the likelihood calculation rule of the landing flexion likelihood to the landing flexion degree of the target motion (S407). The landing flexion likelihood ($L(f_{heel-strike})$) is calculated based on, for example, an expression as follows. Note that $f_{heel-strike}$ indicates a landing flexion degree.

[Math. 6]

$$L(f_{heel-strike}) = \begin{cases} 0.01 & (-100 < f_{heel-strike} < -10) \\ 0 & \text{(other than the above)} \end{cases}$$

Then, the feature likelihood calculation portion 117 calculates the takeoff flexion likelihood of the target motion by applying the likelihood calculation rule of the takeoff flexion likelihood to the takeoff flexion degree of the target motion (S408). The takeoff flexion likelihood ($L(f_{toe-off})$) is calculated based on, for example, an expression as follows. Note that $f_{toe-off}$ indicates a takeoff flexion degree.
[Math. 7]

$$L(f_{toe-off}) = \begin{cases} 0.01 & (-100 < f_{toe-off} < -10) \\ 0 & \text{(other than the above)} \end{cases}$$

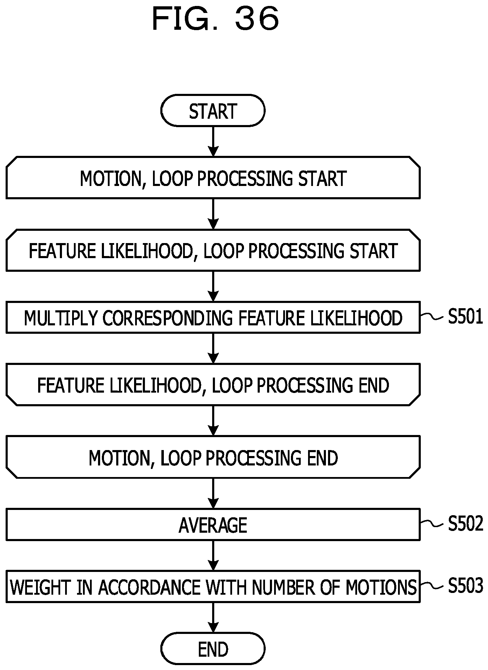

Then, the feature likelihood calculation portion 117 calculates the up-down alternation likelihood of the target motion by applying the likelihood calculation rule of the up-down alternation likelihood to the up-down alternation degree of the target motion (S409).
Then, the feature likelihood calculation portion 117 calculates the simultaneous impact likelihood of the target motion by applying the likelihood calculation rule of the simultaneous impact likelihood to the simultaneous impact degree of the target motion (S410).
Note that one or some of feature likelihoods may not be calculated in accordance with the target activity type or the collected sensor data.
Subsequently, the details of step S280 in FIG. 23 will be described. FIG. 36 is a flowchart illustrating an example of a procedure for processing of calculating natural pattern likelihood.
The natural pattern likelihood calculation portion 118 executes step S501 per candidate motion detected for the target candidate duration, and per feature likelihood calculated in step S270. Specifically, the natural pattern likelihood calculation portion 118 calculates the product of all feature likelihoods calculated for each candidate motion detected for the target candidate duration. Then, the natural pattern likelihood calculation portion 118 averages the calculation results based on the number of candidate motions and the number of feature likelihoods, and hence calculates a natural pattern likelihood (S502). Specifically, a natural pattern likelihood $L_{pattern}$ is calculated by an expression as follows.

[Math. 8]

$$L_{pattern} = \left[ \prod_{i=1}^{N} \prod_{j=1}^{M} L_{i,j} \right]^{\frac{1}{NM}}$$

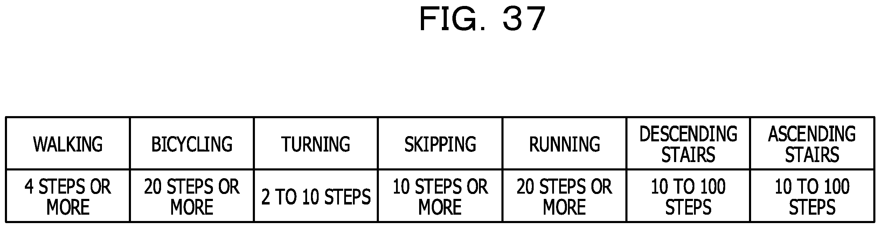

Note that i is a number of a feature likelihood, j is a number of a candidate motion, N is the number of feature likelihoods, and M is the number of candidate motions. Averaging is performed by raising the product of all feature likelihoods calculated for each candidate motion detected for the target candidate duration to the (1/NM)-th power.
Then, the natural pattern likelihood calculation portion 118 weights the calculation result of the natural pattern based on the number N of candidate motions (S503). For example, if the number N of candidate motions does not fall in a predetermined range that is set per activity type as illustrated in FIG. 37, the natural pattern likelihood may be set low. For example, in the case where the target activity type is "walking", if N is 4 or smaller, the duration is not recognized as a walking duration, and the natural pattern likelihood may be decreased.

When sensor data for a predetermined period or longer has been acquired, after all processing is completed, the activity duration determination unit 13 may obtain the distribution of occurrence frequencies of each activity type. If the distribution is unnatural, all processing may be re-executed by changing the threshold for the natural pattern likelihood for determining being the activity duration, or increasing or decreasing the types of natural motion detectors (parameter lists). In this case, "the occurrence frequency being unnatural" indicates a case where, when the number of times of "walking" per day is a predetermined value or smaller (five times or less), the number of times of occurrence of any one of specific activities other than "walking" ("running", "descending stairs", "ascending stairs", and "skipping") exceeds the number of times of occurrence of "walking", or exceeds a predetermined threshold (for example, skipping durations occur by 100 times per day).

When the occurrence frequency of a certain activity type is unnaturally high, the threshold for the natural pattern likelihood may be high, or the natural motion detectors may be decreased. On the other hand, when the occurrence frequency of a certain activity type is unnaturally low, the threshold for the natural pattern likelihood may be decreased, or the natural motion detectors may be increased.

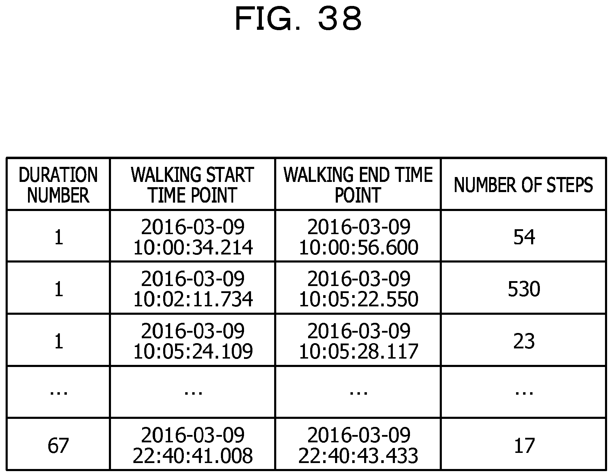

Note that, for example, when step S105 in FIG. 19 for "walking" is completed, the determination result storage unit 15 stores, for example, information as illustrated in FIG. 38.

FIG. 38 is a diagram illustrating an example of determination results of activity durations relating to "walking". FIG. 38 indicates the duration number, start time point, end time point, and number of steps for each activity duration confirmed such that the activity type is "walking". The number of steps is the number of candidate motions detected in the activity duration (candidate duration). Note that identification information on the employed natural motion detector may be associated with each activity duration and stored.

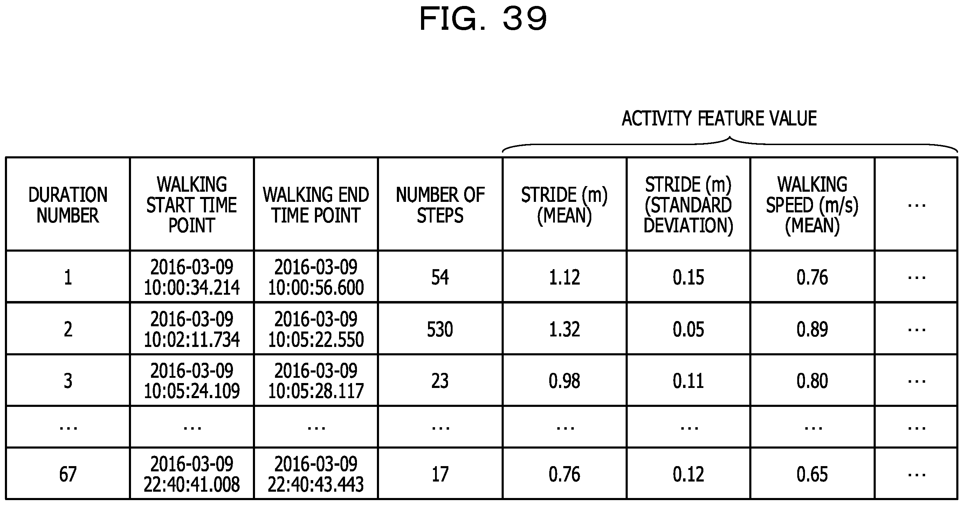

The data processing unit 14 may extract an activity feature value of each activity duration by using information on each activity duration (walking duration) stored in the determination result storage unit 15. As the activity feature value, for example, a stride, a walking speed, a walking duration, a cadence, an impact at landing, a both-leg landing duration, and the like, are extracted. The data processing unit 14 may additionally register the extracted activity feature values to the determination result storage unit 15. FIG. 39 illustrates an example in which the activity feature values are added to the determination results in FIG. 38. Note that the extraction methods of the activity feature values are described in detail in documents (1) to (3) as follows.

(1) Greene, "An adaptive gyroscope-based algorithm for temporal gait analysis", 2012
(2) Wu, "A robust step length estimation system for human gait using motion sensors", 2015
(3) Dobkin, "Reliability and Validity of Bilateral Ankle Accelerometer Algorithms for Activity Recognition and Walking Speed After Stroke", 2011

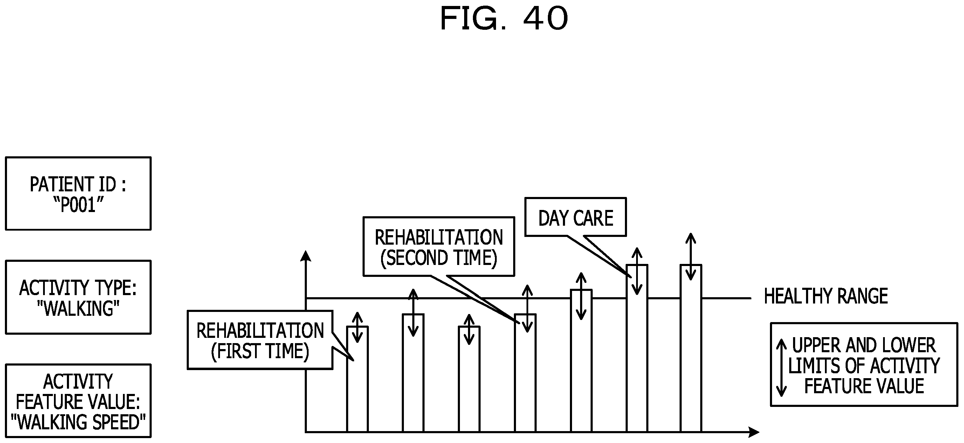

The visualization unit 21 visualizes (outputs) the activity feature values by using the output device 20. For example, when a target date range is designated by a user, the visualization unit 21 acquires the activity feature values in the designated date range from the determination result storage unit 15. Then, the visualization unit 21 calculates, for example, the acquired activity feature values as statistical values per date and visualizes the statistical values as illustrated in FIG. 40 to allow the transition of the way of daily activities to be recognizable. For visualization, for example, the range for reference values for health may be indicated, the timing of a reference event such as a medical treatment may be indicated, or the upper and lower limits of the inter-motion feature values may be indicated.

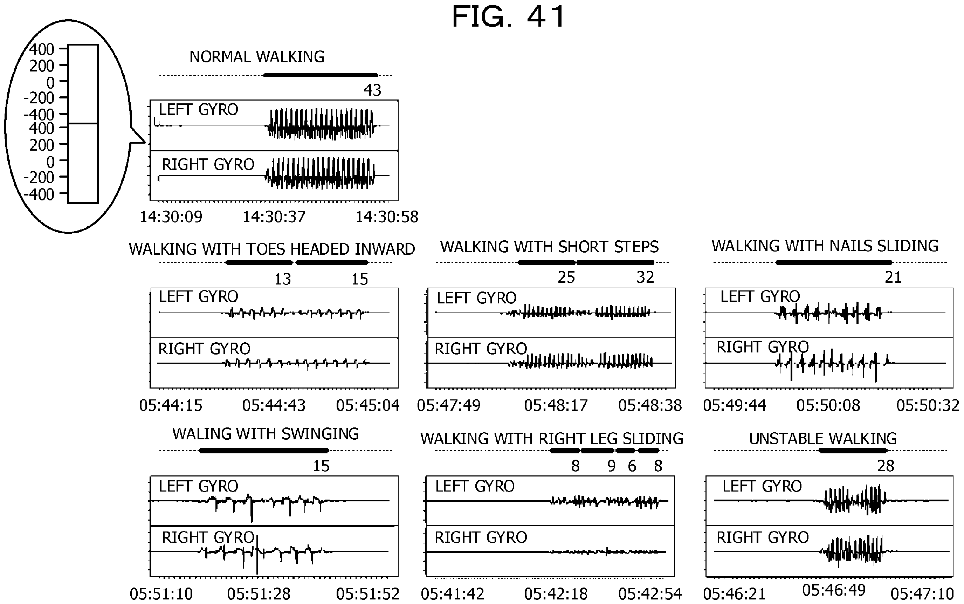

FIG. 41 illustrates the determination results of the activity durations of "walking" (walking durations) when this embodiment is applied to the sensor data acquired for a variety of ways of walking. FIG. 41 indicates durations determined as walking durations for each sensor data when normal walking, walking with toes headed inward, walking with short steps, walking with nails sliding, walking with swinging, walking with a right leg sliding, and unstable walking are performed, by using strips. Regarding FIG. 41, it is found that the walking durations are detected with high accuracy.

As described above, according to the first embodiment, a troublesome work for previously acquiring learning data is omitted, and the activity duration may be accurately detected in accordance with the way of movement of a patient. Even through the way of movement of the patient changes with time, the activity duration may be accurately detected by tracking the change.

Specifically, according to this embodiment, while it is desirable, in actual medical practice, (1) to (continuously) secure accuracy for any way of movement of the patient, and (2) to respond without a troublesome work, the requirements (1) and (2) may be satisfied.

According to this embodiment, since the natural motions are detected by the plurality of natural motion detectors, the possibility of an omission of detecting an activity duration may be reduced.

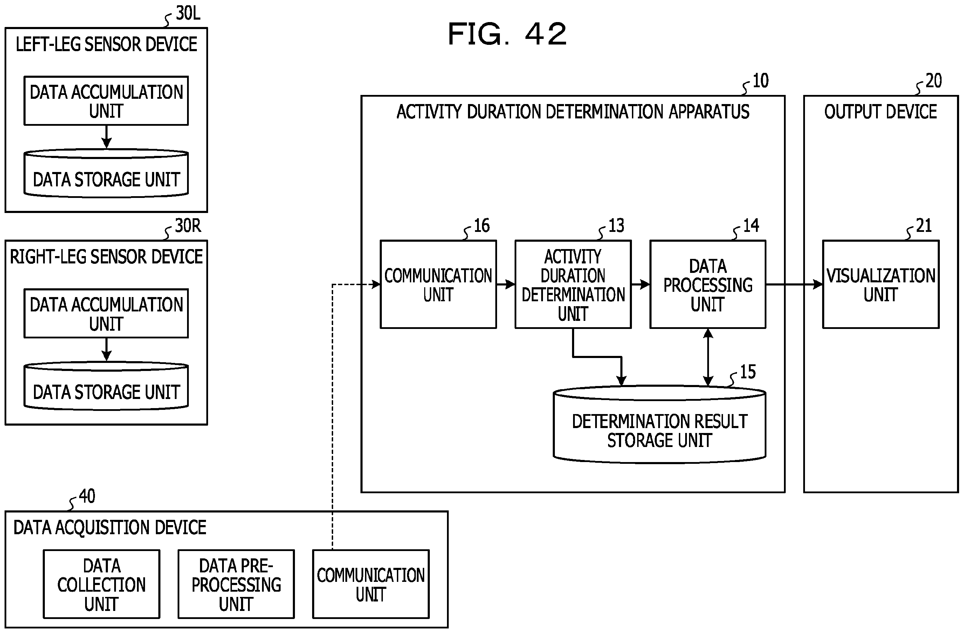

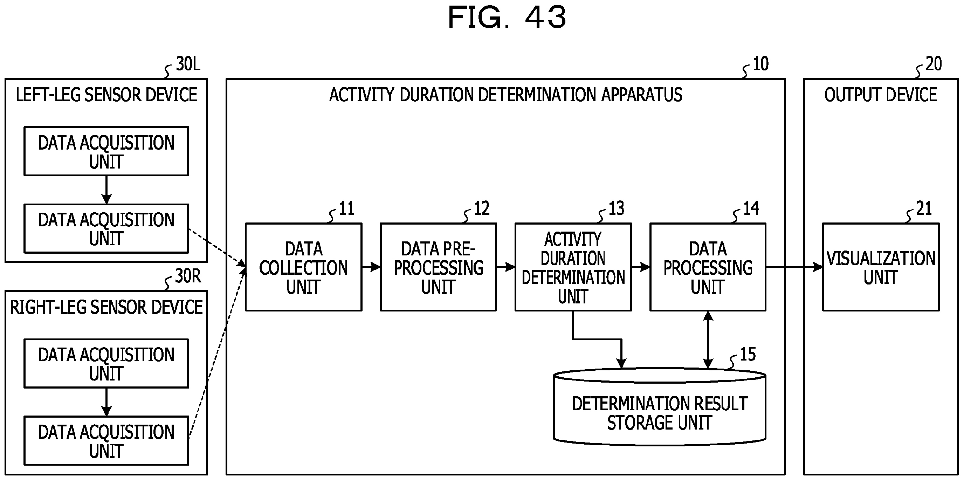

Note that, in the functional configuration illustrated in FIG. 2, the activity duration determination apparatus 10 may include the visualization unit 21. The functional configuration example illustrated in FIG. 2 may be changed to one illustrated in FIG. 42 or FIG. 43. FIG. 42 illustrates the configuration example in which a data acquisition device 40 executes collection of sensor data and pre-processing on the sensor data. In this case, the activity duration determination apparatus 10 may not include the data collection unit 11 and the data pre-processing unit 12, and may include a communication unit 16 for receiving the sensor data pre-processed by the data acquisition device 40.

FIG. 43 illustrates an example in which each sensor device does not include the data storage unit. Specifically, FIG. 43 illustrates a functional configuration example in which sensor data is collected from each sensor device in real time, and the procedure in FIG. 19 is executed in real time.

A second embodiment is described next. According to the second embodiment, only the points different from those of the first embodiment are described. The points not described according to the second embodiment may be similar to those according to the first embodiment.

According to the first embodiment, it is assumed that the candidate motion group in one candidate duration involves motions of the same activity type. Thus, if motions of a plurality of activity types are mixed in one candidate duration (for example, in a case where turning is performed before walking), accuracy of natural pattern likelihood may be deteriorated. Owing to this, according to the second embodiment, an example expected such that a plurality of activity types are mixed in a candidate duration is described.

According to the second embodiment, first, an activity duration of a target activity type (hereinafter, referred to as "target duration") and an activity duration of a non-target activity type (hereinafter, referred to as "non-target duration") are separated from each other by using the inter-motion feature value of each candidate motion, and then, a natural pattern likelihood is calculated for the target duration. A case where the target activity type is "walking" is described below.

First, candidate motions of a candidate motion group in a candidate duration are classified into a walking motion and a non-walking motion. Note that the walking motion is a candidate motion belonging to the activity type of "walking".

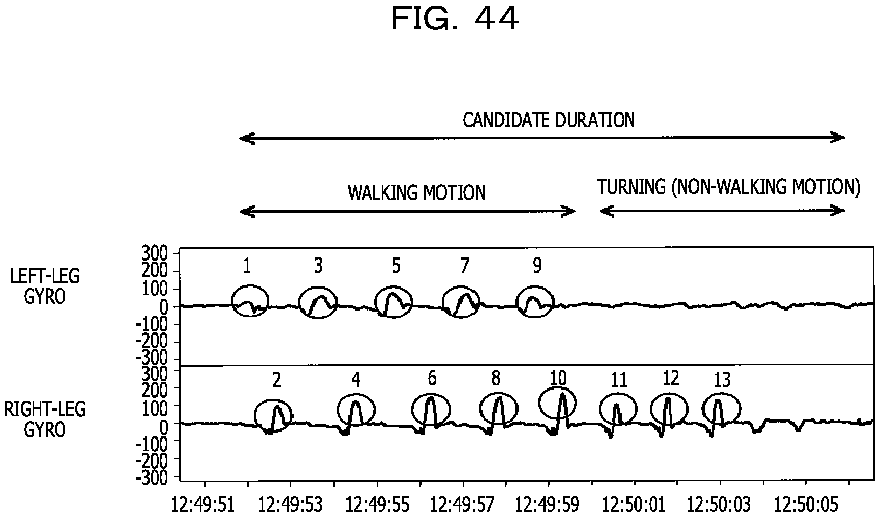

FIG. 44 is a diagram illustrating an example in which candidate motions of a candidate motion group are classified into a walking motion and a non-walking motion. FIG. 44 indicates gyro data in the sagittal plane direction of left and right legs when walking is performed and then turning is performed at the same position. The duration in which the series of motions is performed is extracted as one candidate duration. While 13 candidate motions are detected in that candidate duration, actually, candidate motions 1 to 10 are walking motions, and candidate motions 11 to 13 are non-walking motions.

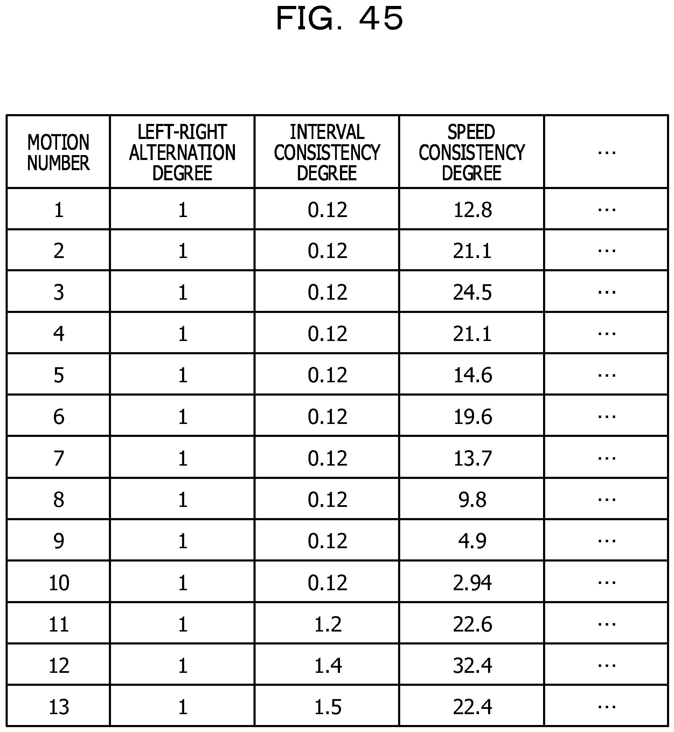

It is assumed that inter-motion feature values are extracted for the candidate motions 1 to 13 as illustrated in FIG. 45.

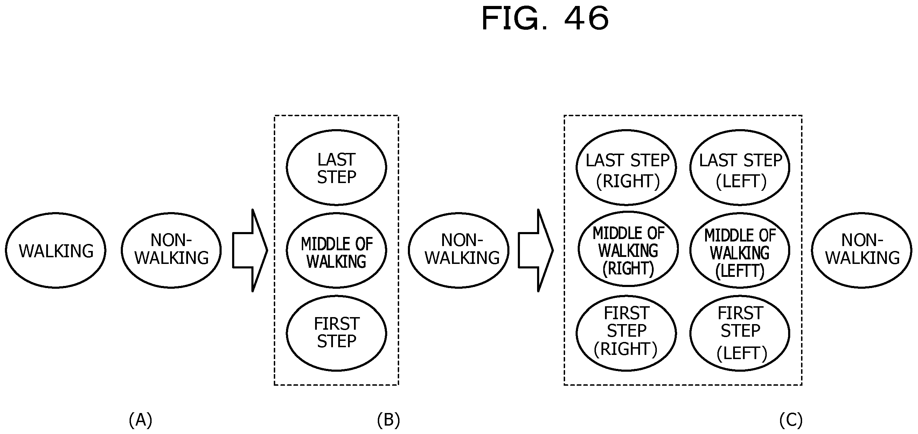

FIG. 46 is a diagram illustrating a strategy for classifying candidate motions of a candidate motion group into a walking motion and a non-walking motion.

For the candidate motion group, to allow the classification illustrated in FIG. 44, as illustrated in FIG. 46(A), one of at least two states of "walking motion" and "non-walking motion" is assigned to each candidate motion.

To recognize a walking motion further correctly, "walking motions" are classified into three states including "first step motion", "middle-of-walking motion", and "last step motion" as illustrated in FIG. 46(B), by taking into account a feature that the first step and the last step each make a half step unlike the second and later steps to a step immediately before the last step.

If the ways of movement of the left and right legs are asymmetric like sensor data as illustrated in FIG. 44, it is desirable to set different reference values of the inter-motion feature values for the left and right legs. Thus, as illustrated in FIG. 46(C), left "walking motion" is discriminated from right "walking motion". Consequently, walking motions are classified into six states.

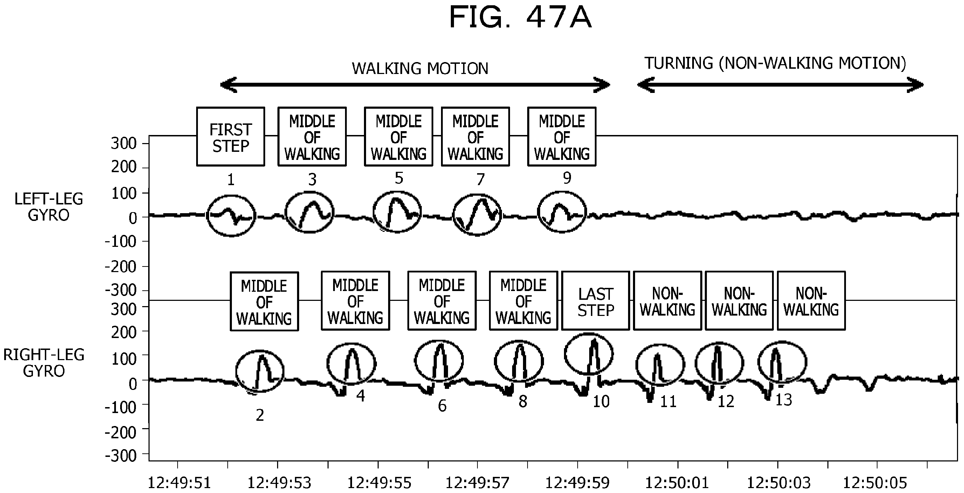

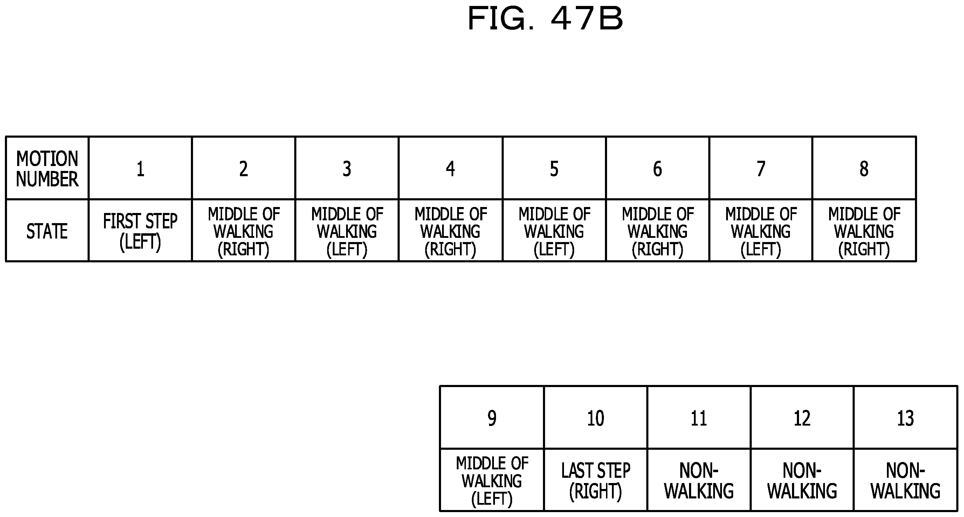

There are assumed seven states by adding the non-walking state to the six states relating to the walking motion. Then, one of these states is assigned to each candidate motion. FIGs. 47A and 47B illustrate an example in which one of the seven states is assigned to each candidate motion illustrated in FIG. 44. Consequently, a target duration and a non-target duration may be specified.

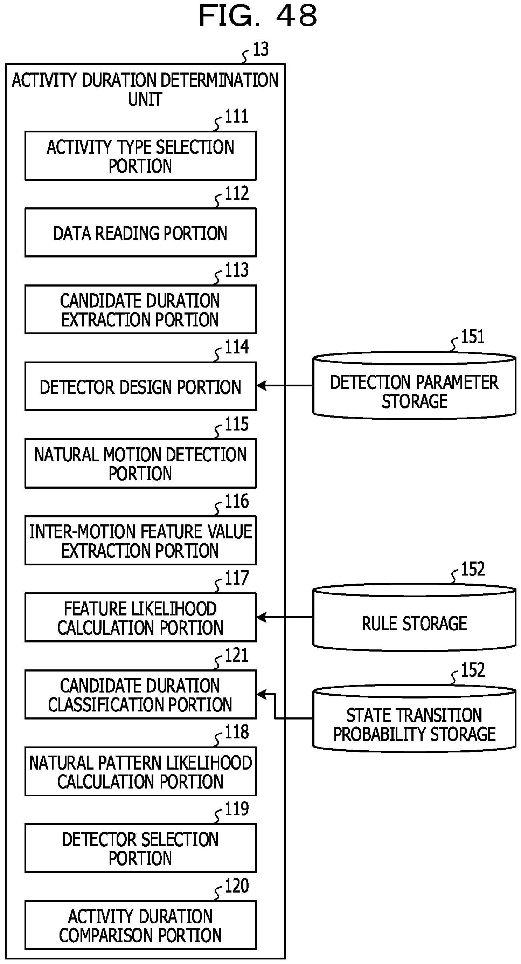

An activity duration determination apparatus 10 according to the second embodiment is described below in detail. FIG. 48 is a diagram illustrating a functional configuration example of an activity duration determination unit according to the second embodiment. In FIG. 48, the same reference sign is applied to the same part as one in FIG. 3, and the description thereof is omitted.

In FIG. 48, the activity duration determination unit 13 further includes a candidate duration classification portion 121. The candidate duration classification portion 121 uses a state transition probability storage unit 153. The state transition probability storage unit 153 may be implemented by using, for example, the auxiliary storage device 102, or a storage device or the like connectable to the activity duration determination apparatus 10 via a network.

The candidate duration classification portion 121 classifies candidate durations into a target duration and a non-target duration. The state transition probability storage unit 153 stores information on transition probabilities among the seven states. The information is used by the candidate duration classification portion 121.

The second embodiment differs from the first embodiment for the content of step S105 in FIG. 19.

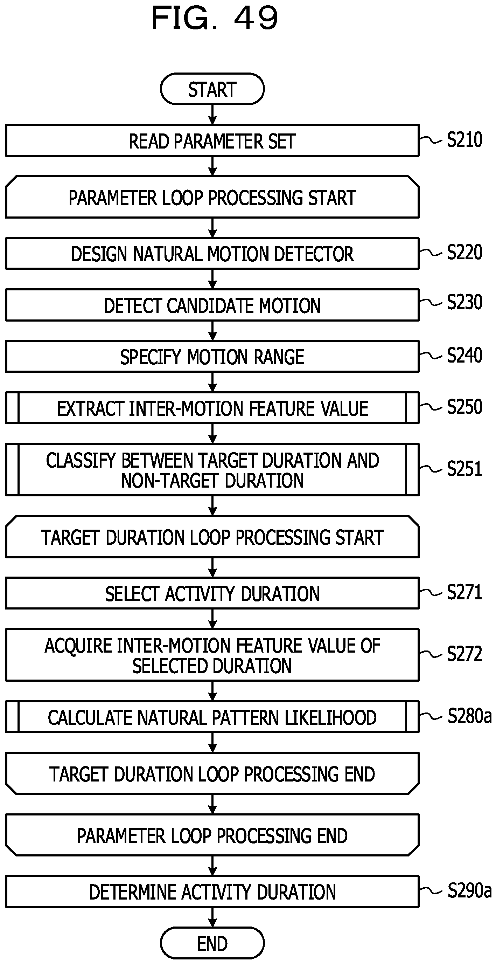

FIG. 49 is a flowchart illustrating an example of a procedure for activity duration determination processing according to the second embodiment. In FIG. 49, the same step number is applied to the same step as one in FIG. 23, and the description thereof is omitted.

In FIG. 49, step S251 is executed subsequently to step S250. In step S251, the activity duration determination unit 13 classifies target candidate durations into a target duration and a non-target duration. Specifically, a state is assigned to each candidate motion in a target candidate duration as described with reference FIGs. 47A and 47B, and thus, target candidate durations are classified into a target duration and a non-target duration.

Then, steps S271 to S280a are executed per target duration. For example, if a plurality of target durations are extracted in step S251 such as if "turning" is performed during a walking motion, step S271 to S280a are executed for each target duration. Specifically, a natural pattern likelihood is calculated per target duration.

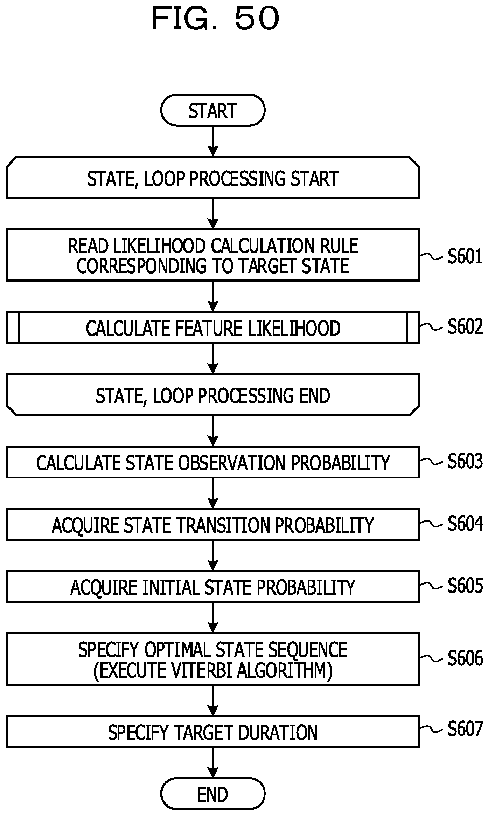

Subsequently, the details of step S251 will be described. FIG. 50 is a flowchart illustrating an example of a procedure for processing of classifying candidate durations.

First, steps S601 and S602 are executed per each of seven states. Hereinafter, a state being recognized as a processing target is referred to as "target state".

In step S601, the feature likelihood calculation portion 117 reads a likelihood calculation rule group corresponding to a target activity type and a target state from the rule storage unit 152. Specifically, according to the second embodiment, the likelihood calculation rule is defined per activity type and per state.

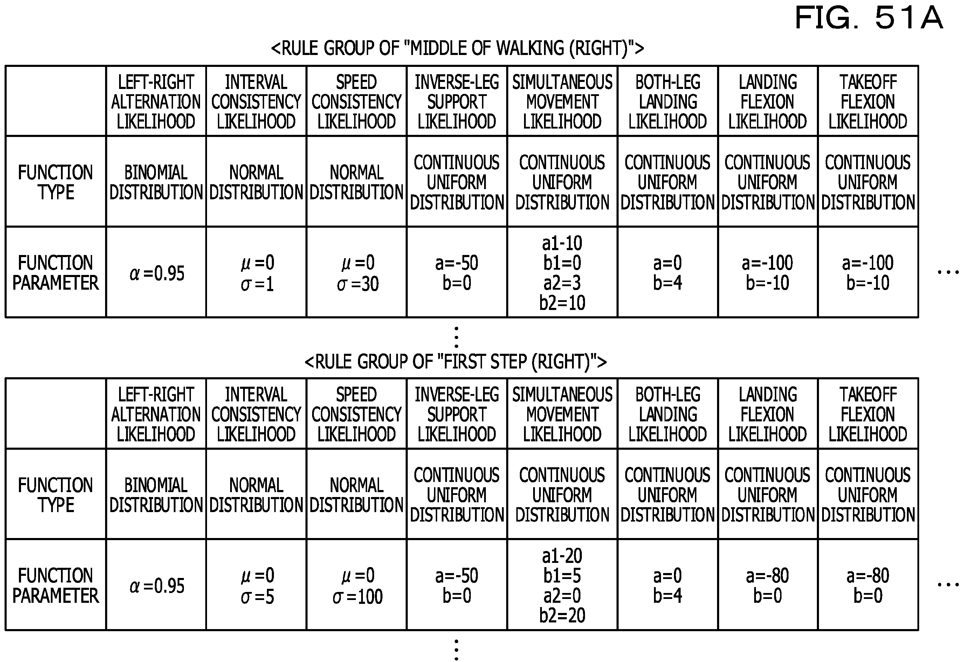

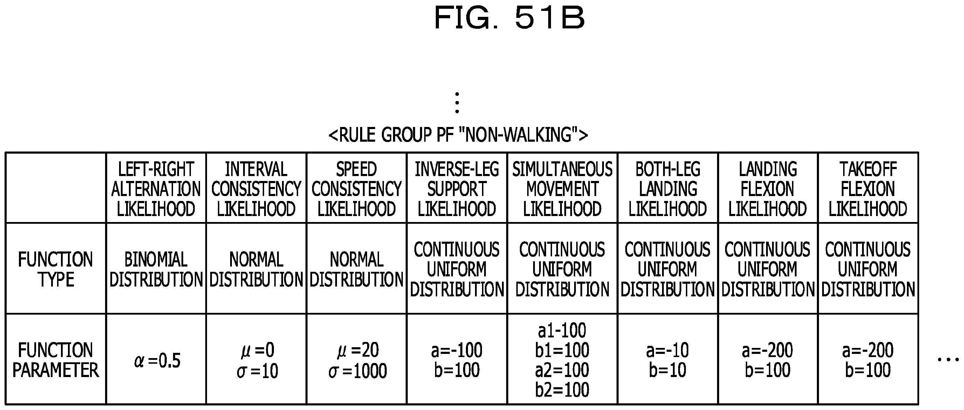

FIGs. 51A and 51B are a diagram illustrating likelihood calculation rules according to the second embodiment. FIGs. 51A and 51B indicate that the likelihood calculation rule group corresponding to "walking" is subdivided into likelihood calculation rule groups for the seven states.

Specifically, a likelihood calculation rule defines an optimal inter-motion feature value to obtain a likelihood per state, to assign a state to each candidate motion.

For example, for "middle of walking (right)" and "middle of walking (left)", one the same as the likelihood calculation rule according to the first embodiment may be employed. For "first step (right)", "first step (left)", "last step (right)", and "last step (left)", one obtained by changing the likelihood calculation rule corresponding to "middle of walking (right)" and "middle of walking (left)" is defined, by taking into account that a motion is not made before or after the first step or the last step and that only a half step is made by the first step or the last step.

For a non-walking state, "likelihood of non-walking" is defined. For a non-walking state, since it is difficult to expect an inter-motion feature value to be acquired, a rule group may be set so that a feature likelihood is uniform for any observed inter-motion feature value.

Then, the feature likelihood calculation portion 117 uses the read likelihood calculation rule group, executes the procedure in FIG. 34, and hence calculates each feature likelihood (S602).

When steps S601 and S602 are executed for the seven states, feature likelihoods are calculated for each of the seven states per candidate motion in the target candidate duration.

Then, the candidate duration classification portion 121 performs multiplication on the feature likelihood group calculated for the candidate motion and the state per candidate motion in the target candidate duration and per state (S603). The multiplication result of the feature likelihood group corresponds to a state observation probability according to hidden Markov model. According to the present invention, a probability distribution parameter of a likelihood calculation rule may be described as state observation probability information.

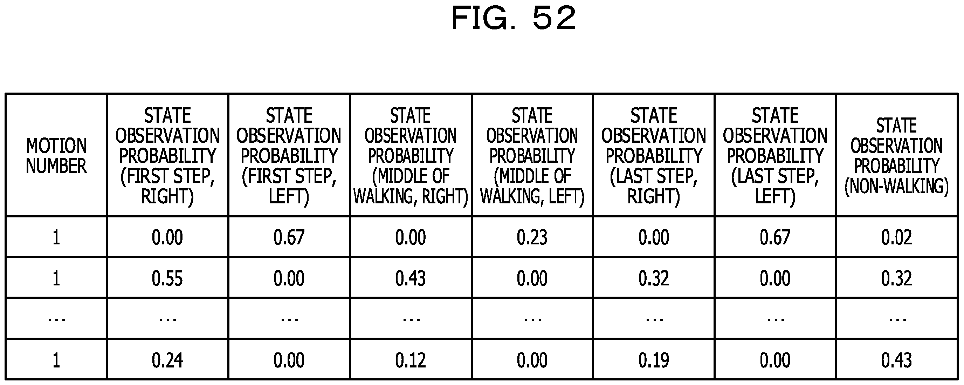

FIG. 52 is a diagram illustrating an example of calculation results of state observation probabilities according to hidden Markov model. FIG. 52 indicates calculation results of state observation probabilities of 13 candidate motions for each of the seven states.

Then, the candidate duration classification portion 121 acquires state transition probability information corresponding to the target activity type from the state transition probability storage 153 unit (S604).

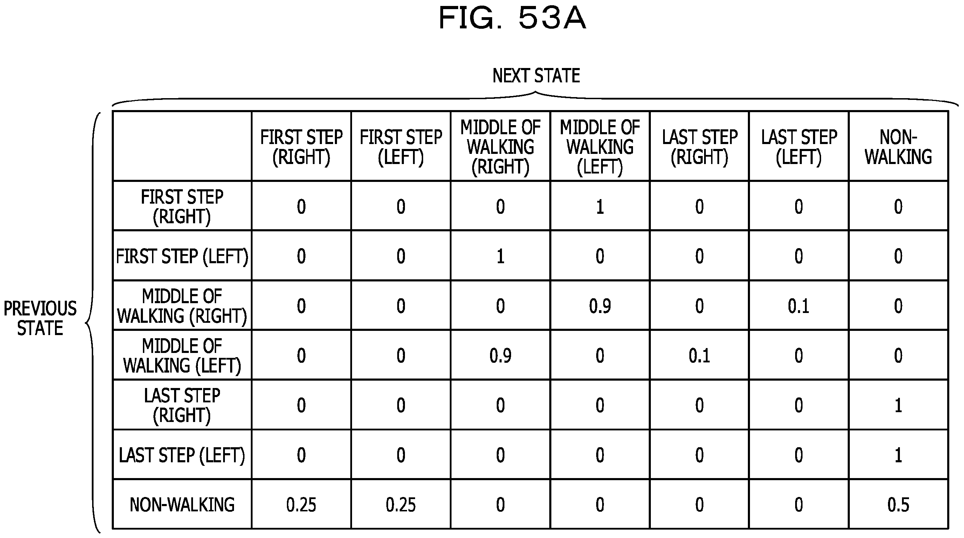

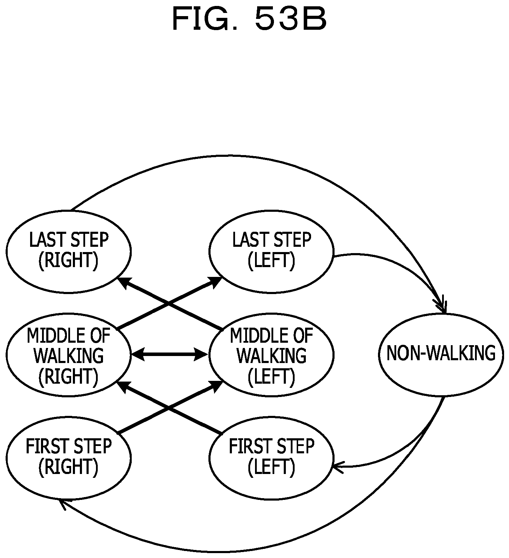

FIGs. 53A and 53B are a diagram illustrating a first example of state transition probability information. The table in FIGs. 53A and 53B, assuming that the state arranged in the row direction is the previous state, and the state arranged in the column direction is the next state, indicates probabilities of transition from the previous state to the next state. Note that FIGs. 53A and 53B illustrate, on the right side, a state transition diagram corresponding to the table in FIGs. 53A and 53B for reference.

When the state transition probability information is defined, to express "alternately stepping left and right legs during walking", the transition probability of transition to a state on the same side during walking may be set to 0 so as not to make the transition.

Then, the candidate duration classification portion 121 acquires initial state probability information corresponding to the target activity type from the state transition probability storage unit 153 (S605).

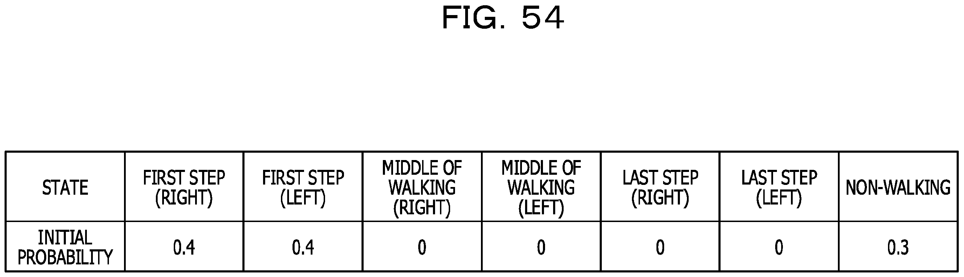

FIG. 54 is a diagram illustrating an example of initial state probability information. FIG. 54 illustrates an example in which an initial state is assumed to be one of "first step (right)", "first step (left)", and "non-walking", and an initial state probability being 0 in a state other than the above-mentioned states is defined.

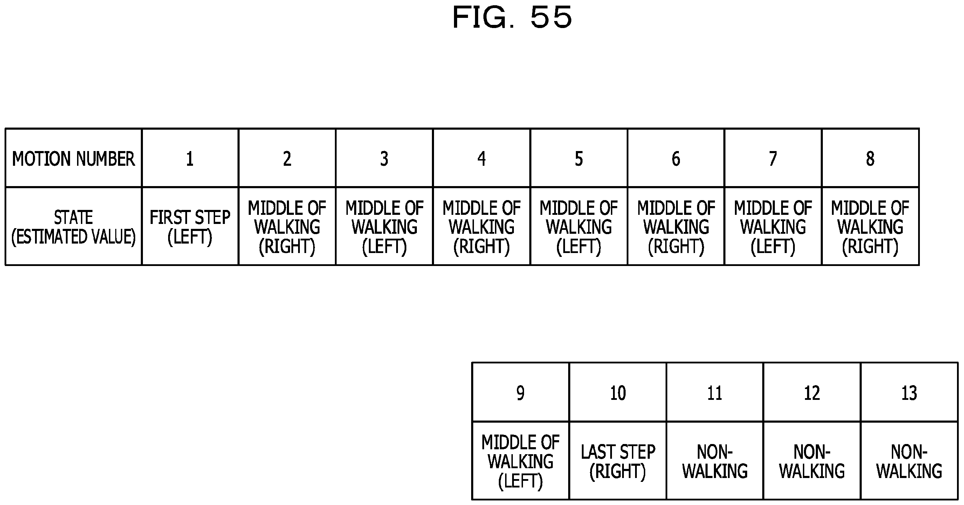

Then, the candidate duration classification portion 121 obtains an optimal state sequence based on Viterbi algorithm by using the state observation probability (FIG. 52), the state transition probability information (FIG. 53), and the initial state probability information (FIG. 54) for each state per candidate motion (S606). Consequently, information indicated in FIG. 55 is obtained.

FIG. 55 is a diagram illustrating an example of an optimal state sequence. As illustrated in FIG. 55, an estimated value for a state is assigned to each candidate motion.

Then, the candidate duration classification portion 121 specifies a duration from when a state included in a walking state is started to when the state is ended in the state sequence obtained in step S606, as a target duration (S607). In this case, a situation the number of steps (the number of candidate motions) exceeds a predetermined threshold (for example, 4) may be set as a condition for specification as a target duration.

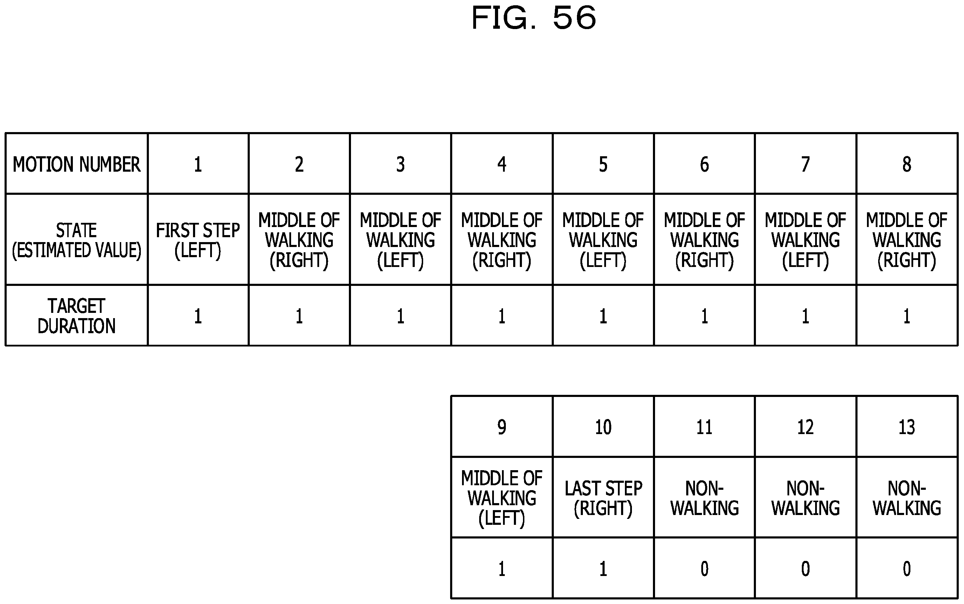

FIG. 56 is a diagram illustrating an example of specification results of a target duration. FIG. 56 adds a row of "target duration" to the table illustrated in FIG. 55. In the row, 1 is assigned to a candidate motion corresponding to a target duration, and 0 is assigned to a candidate motion corresponding to a non-target duration. Thus, in the example in FIG. 56, a motion number 1 corresponds to a start point of a walking duration, and a motion number 10 corresponds to an end point of the walking duration. The number of steps (the number of candidate motions) from the start point to the end point are 10, which are specified as a target duration.

In step S280a in FIG. 49, an inter-motion feature value in the target duration is extracted to obtain a natural pattern likelihood in the target duration. For example, regarding the specification results of the target duration illustrated in FIG. 56, inter-motion feature values are extracted per candidate motion from the motion number 1 to the motion number 10 in FIG. 45.

Each extracted inter-motion feature value is converted into a feature likelihood by the same method as one according to the first embodiment, and a natural pattern likelihood is calculated by the same method as one according to the first embodiment (that is, based on Math. 8 (Expression 8)).

Alternatively, to take into account a state transition probability, hidden Markov model is applied, and the logarithm of a model likelihood of a state sequence in FIG. 55 may be obtained as a natural pattern likelihood.

Still alternatively, a value (corresponding to a model likelihood per step) obtained by dividing the logarithm of the model likelihood by the number of steps (the number of candidate motions) may be obtained as a natural pattern likelihood.

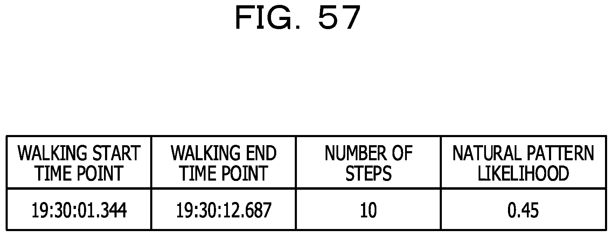

The detector selection portion 119 stores the calculation results of the natural pattern likelihood together with the number of steps while treating a start time point of a peak expressing the start of walking as "walking start time point" and an end time point of a peak expressing the end of walking as "walking end time point", in the determination result storage unit 15 in the format illustrated in FIG. 57.

The above-described processing is executed per parameter list (per natural motion detector). According to this embodiment, a walking duration and a natural pattern likelihood are calculated for the four types of parameter lists including "Fast", "Slow", "Normal", and "Abnormal".

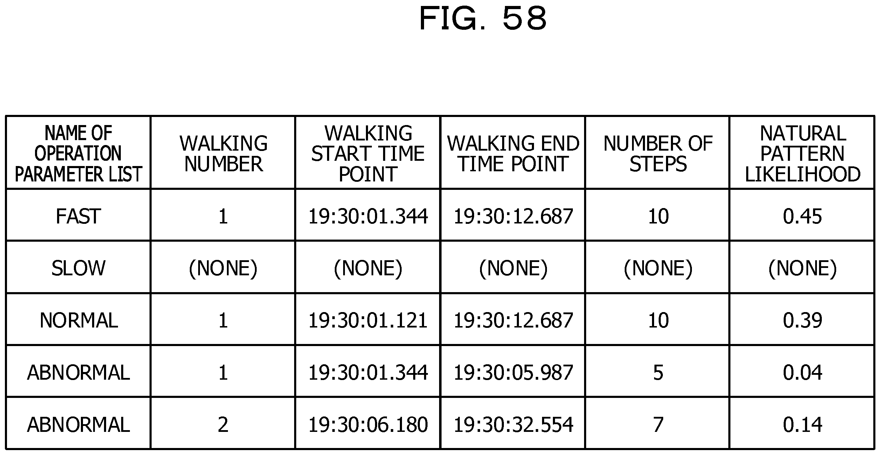

FIG. 58 is a diagram illustrating an example of determination results of activity durations based on all natural motion detectors for one candidate duration according the second embodiment. In the example in FIG. 58, a walking duration is not detected for "Slow" at all, and two walking durations are detected in a target candidate duration for "Abnormal".

In step S290a in FIG. 49, the detector selection portion 119 basically executes processing similar to one in step S290 in FIG. 23. However, for a natural motion detector that detects a plurality of walking durations, the mean of natural pattern likelihoods in the plurality of walking durations serves as an evaluation value. Alternatively, instead of a simple mean, a weighted mean obtained by weighting a natural pattern likelihood using a walking duration or the number of steps may serve as an evaluation value.

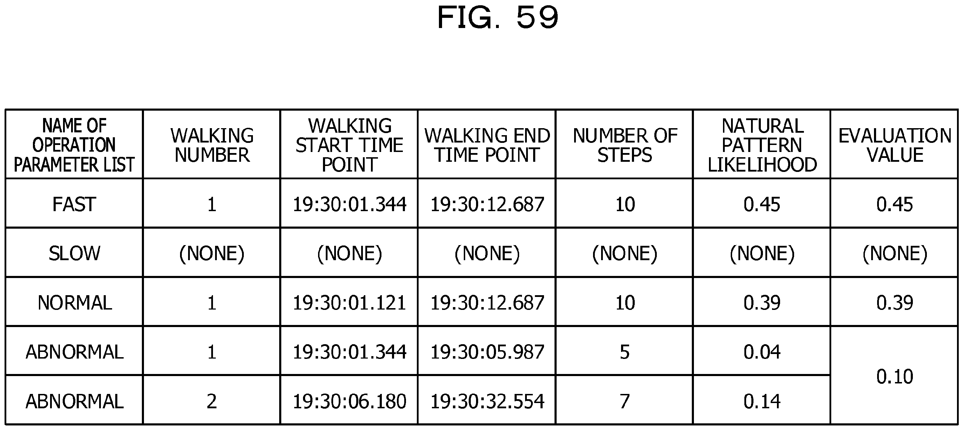

FIG. 59 is a diagram illustrating an example of evaluation values of determination results of activity durations based on all natural motion detectors for one candidate duration. In FIG. 59, a weighted mean obtained by weighting two natural pattern likelihoods using the number of steps serves as an evaluation value for "Abnormal" by which two walking durations are detected. For "Fast" and "Normal", natural pattern likelihoods serve as evaluation values without being changed.

In this case, the detector selection portion 119 employs "Fast" with the maximum evaluation value. If the evaluation value exceeds a threshold for the natural pattern likelihood, the detector selection portion 119 outputs the start time point and the end time point of "Fast" as the start time point and the end time point of "walking".

While the case where the target activity type is "walking" has been described above, the second embodiment may be similarly applied to other activity types. However, in the case of "ascending stairs", "descending stairs", "running", and "skipping", walking may be highly possibly performed before and after such a motion.

Hence, a walking state may be added as a state of hidden Markov model, and then state transition may be defined.

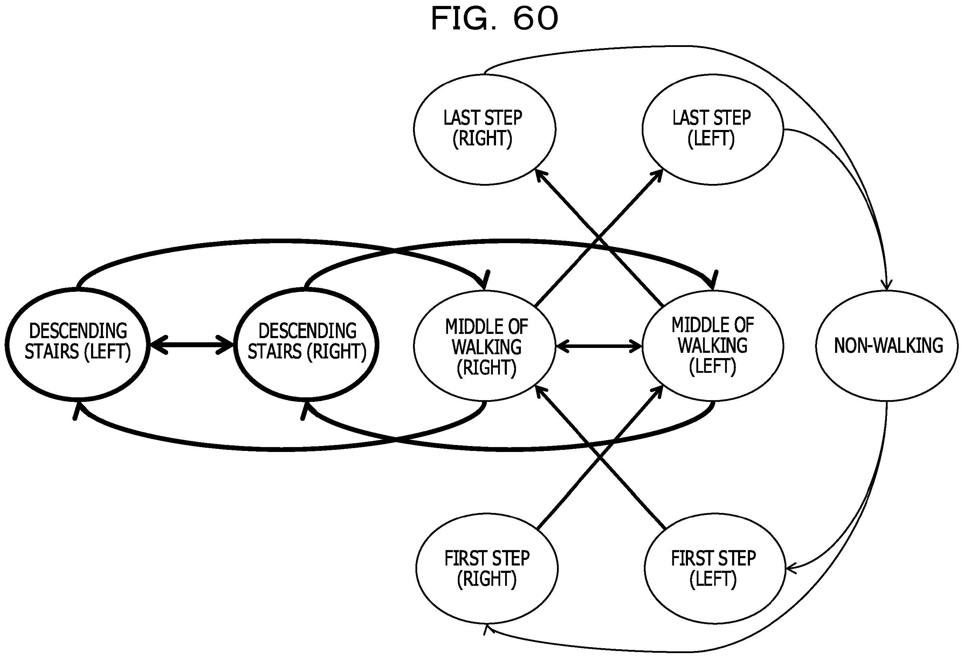

FIG. 60 is a diagram illustrating an example of state transition of "descending stairs". FIG. 60 illustrates a state transition diagram for transition from a walking state to "a descending stairs state".

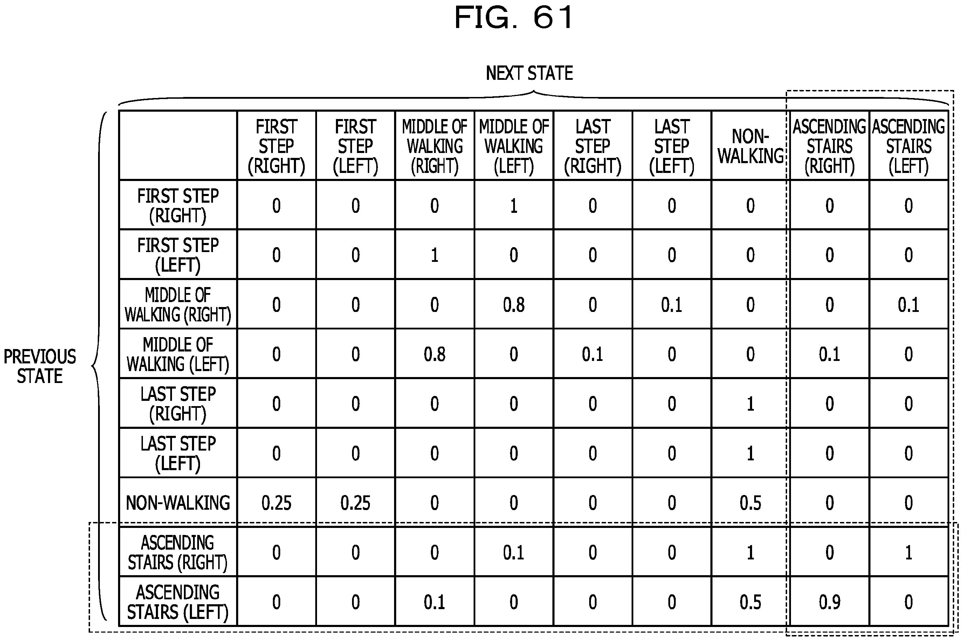

State transition probability information may be defined as illustrated in FIG. 61, based on such a state transition diagram.

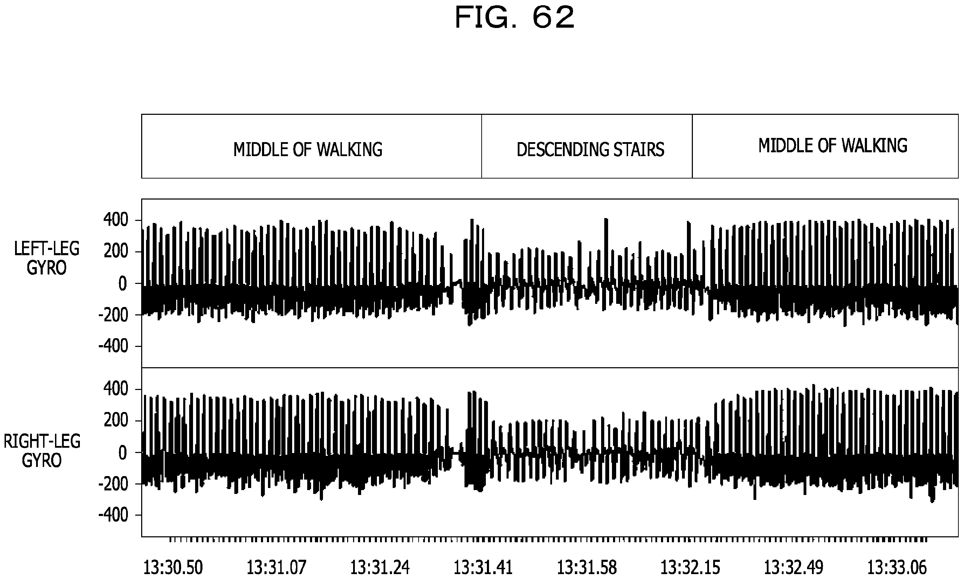

The way of viewing FIG. 61 is similar to FIGs. 53A and 53B. However, in FIG. 61, rows and columns relating to "descending stairs (right)" and "descending stairs (left)" are added. FIG. 61 indicates that the state of "middle of walking" transitions to the state of "descending stairs" by a 10% probability, and after the state transitions to the state of "descending stairs", the state stays in the state of "descending stairs" by a 90% probability. FIG. 62 illustrates a result example in which the state transition probability information in FIG. 61 is used and a state is assigned to a candidate motion group.

Note that state transition probability information similar to that of "descending stairs" may be used also for "ascending stairs", "running", and "skipping". Specifically, "descending stairs state" in the state transition probability information in FIG. 61 may be replaced with "ascending stair state", "running state", or "skipping state".

In the above description, the probability distribution parameter of the likelihood calculation rule (FIGs. 51A and 51B) and the state transition probability information (FIGs. 53A and 53B, FIG. 61) are previously set information.

However, if sensor data with a confirmed activity duration exists, the candidate duration classification portion 121 may execute processing of using the sensor data, using Baum-Welch algorithm or the like, learning each of parameters (probability distribution parameter of likelihood calculation rule, and state transition probability information) of hidden Markov model, and adding the learning results to the rule storage 152 and the state transition probability storage unit 153.

In this case, the likelihood calculation rule and the state transition probability information (hereinafter, referred to as "parameter group of hidden Markov model") serving as application targets are stored by a plurality of sets. Processing results when a natural pattern likelihood is calculated based on each parameter group of hidden Markov model and a maximum natural pattern likelihood is obtained may be employed.

However, when learning is repeated and the number of sets of parameter groups of hidden Markov model increases, the processing load increases.

Thus, the candidate duration classification portion 121 may add a parameter group only if a parameter group that is not similar to any of already-stored parameter groups has been learned.

For example, the candidate duration classification portion 121 may calculate a similarity sim(P, Q) based on the following expression for a newly learned parameter group P and all existing parameter groups $Q_1, Q_2, ..., Q_N$.

[Math. 9]

$$sim(P,Q) = \sum_{i=1}^{N} \left( \frac{|p_i - q_i|}{\eta_i} \right)^2$$

$p_i$: parameter value of i-th type in parameter group P

$q_i$: parameter value of i-th type in parameter group Q

$\eta_i$: normalization term of parameter of i-th type

The candidate duration classification portion 121 may add the newly learned parameter group P to the rule storage 152 and the state transition probability storage 153 if the similarity sim(P, Q) is equal to or smaller than a threshold.

As described above, according to the second embodiment, an activity duration may be accurately detected even if a plurality of activity types are mixed in one candidate duration.

A third embodiment is described next. According to the third embodiment, only the points different from those of the first and second embodiments are described. The points not described according to the third embodiment may be similar to those according to the first and second embodiments.

According to the third embodiment, a case of using a sensor device that measures plantar pressure data of both legs instead of sensor devices that measures motion sensor data of both legs is described.

Note that if plantar pressures are obtained, since a large change in pressure occurs at landing, a natural motion may be detected with relatively high accuracy.

If only sensor data expressing plantar pressures of both legs is obtained, (1) extraction of candidate duration, (2) detection of natural motion, (3) extraction of inter-motion feature value, and (4) calculation of natural pattern likelihood are executed, similarly to the case where motion sensor data is obtained (the first embodiment). (1), (2), and (3) execute processing specific to the plantar pressures, whereas (4) executes common processing.

(1) Extraction of Candidate Duration

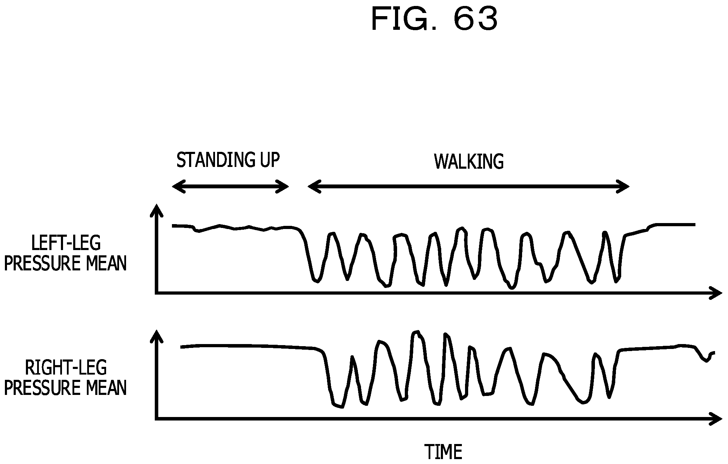

As illustrated in FIG. 63, the mean pressures of the plantar pressures of both legs are high at standing up; however, the mean pressures are low while a leg swinging motion is performed. In this case, correlation between the left and right mean pressures is negative.

Thus, similarly to the case where motion sensor data is obtained, a mean pressure P(t) (or a total sum pressure) of the entire region of a sole in a window with a constant width is calculated for each leg at each time point while the window is shifted, and a window in which a correlation coefficient of "mean pressure $P_{left}(t)$ of left leg" and "mean pressure $P_{right}(t)$ of right leg" is equal to or smaller than a threshold is recognized as a candidate window. Continuous candidate windows are coupled to each other and a candidate duration is extracted.

(2) Detection of Natural Motion

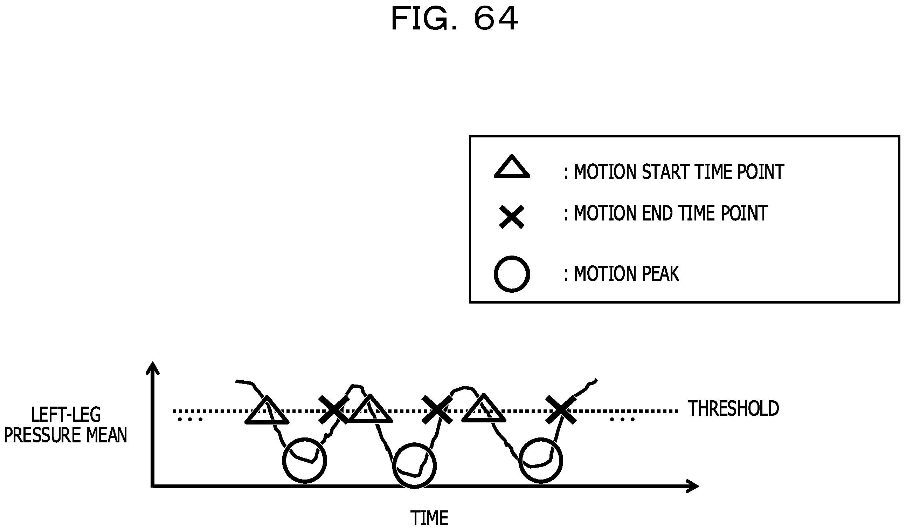

In a candidate duration, it is recognized that a leg swinging motion occurs if the mean pressure is below the threshold, a time point at which the mean pressure becomes below the threshold first is treated as a motion start time point, and a time point at which the mean pressure becomes above the threshold last is treated as a motion end time point. The lowest point of the mean pressure is treated as a motion peak time point. The above detection is executed for both left and right legs. FIG. 64 illustrates a detection example of a natural motion based on plantar pressure data.

(3) Extraction of Inter-motion Feature Value

The left-right alternation degree, interval consistency degree, and both-leg landing degree are extracted similarly to those according to the first embodiment.
The speed consistency degree is calculated, by taking into account that the speed consistency degree correlates with the walking speed and the impact of heel strike, such that the maximum value of the mean pressure at a point close to a landing time point is set as a speed feature and a proper reference value is provided.
The inverse-leg support degree is a time-average mean pressure of an inverse leg during motion.
The landing flexion degree is "a variation of a mean pressure of a sole front region" from the end of a motion to after a threshold time point. Since a heel is landed at landing, a toe region is lifted as a knee is bent, and the mean pressure of the sole front region markedly changes.
The takeoff flexion degree is "a variation of a mean pressure of a sole rear region" from the start of a motion to before a threshold time point. Since a toe is lifted at taking off, a heel region is lifted as a knee is bent before taking off, and the mean pressure of the sole rear region slightly changes.

(4) Calculation of Natural Pattern Likelihood

Calculation of a natural pattern likelihood is performed similarly to the first embodiment. However, 1 is assigned to a feature likelihood for a lacking inter-motion feature value.
A fourth embodiment is described next. According to the fourth embodiment, only the points different from those of the first and second embodiments are described. The points not described according to the fourth embodiment may be similar to those according to the first and second embodiments.
According to the fourth embodiment, a case of using a sensor device that measures sensor data expressing a motion of an upper body is described.
If only sensor data expressing a motion of an upper body is obtained, (1) extraction of candidate duration, (2) detection of natural motion, (3) extraction of inter-motion feature value, and (4) calculation of natural pattern likelihood are executed, similarly to the case where motion sensor data is obtained (the first embodiment). Note that (1), (2), and (3) execute processing specific to the upper body motion, whereas (4) executes common processing. Processing of separating candidate motions into left and right candidate motions is executed in (3).

(1) Extraction of Candidate Duration

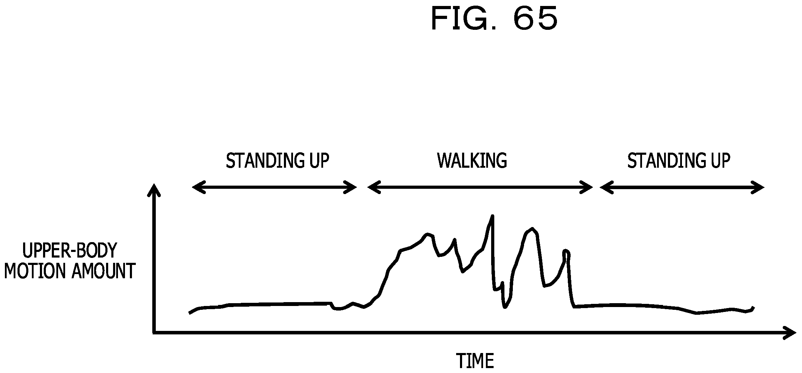

FIG. 65 illustrates an example of sensor data indicating a motion of an upper body. Similarly to the case where motion sensor data is obtained, an upper body motion amount (for example, a composite value of the squares of gyro or acceleration of an upper body along all axes) P(t) in a window with a constant width is calculated at each time point while the window is shifted, and a window in which a mean and a dispersion of motion amounts in the window are thresholds or larger are recognized as a candidate window. Continuous candidate windows are coupled to each other and a candidate duration is extracted.

(2) Detection of Natural Motion

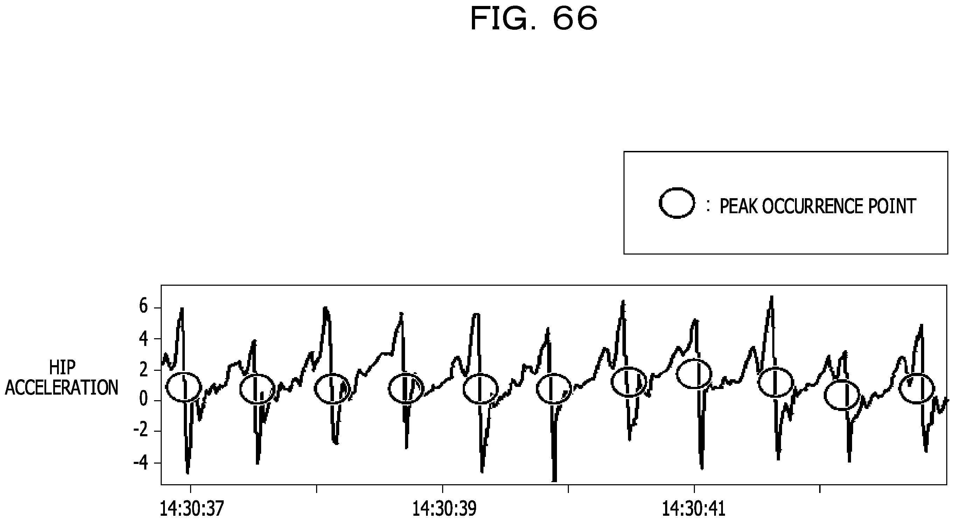

Data X(t) expressing an amplitude in the up-down direction (for example, acceleration in the up-down direction) is extracted from upper body motion data in a candidate duration (FIG. 66), and is converted into data D(t) = X(t) - X(t-1) expressed by a variation from a preceding measurement time point. Peak detection is performed while a band-pass filter is applied to the variation data D(t). It is determined that a motion occurs if a peak exceeds a threshold. The occurrence point of natural motion detection may be a peak or a point before or after the peak.

(3) Extraction of Inter-motion Feature Value

Before an inter-motion feature value is extracted, occurring motions are classified into a left leg motion or a right leg motion. Motions may be simply alternately assigned to "right leg, left leg, right leg, ..." in the order of occurrence. Alternatively, "right leg motion" may be determined if the gravity center direction is inclined to the left at landing, and "left leg motion" may be determined if the gravity center direction is inclined to the right at landing.

The left-right alternation degree and interval consistency degree are extracted similarly to the case where motion sensor data for both legs is obtained.

The speed consistency degree is calculated, by taking into account that the speed consistency degree correlates with the walking speed and the impact of heel strike, such that the maximum value of the variation at a point close to a peak is set as a speed feature and with a proper reference value is provided.

The simultaneous movement degree is extracted similarly to the first embodiment.

The inverse-leg support degree, both-leg landing degree, landing flexion degree, and takeoff flexion degree are lacking values.

(4) Calculation of Natural Pattern Likelihood

Calculation of a natural pattern likelihood is performed similarly to the first embodiment. However, 1 is assigned to a feature likelihood for a lacking inter-motion feature value.

A fifth embodiment is described next. According to the fifth embodiment, only the points different from those of the first embodiment are described. The points not described according to the fifth embodiment may be similar to those according to the first embodiment.

According to the fifth embodiment, "a likelihood that a candidate motion group is a natural motion corresponding to a target activity type" is added as an evaluation reference for a candidate group (candidate motion group) of natural motions. The likelihood is called "natural motion likelihood" according to the fifth embodiment.

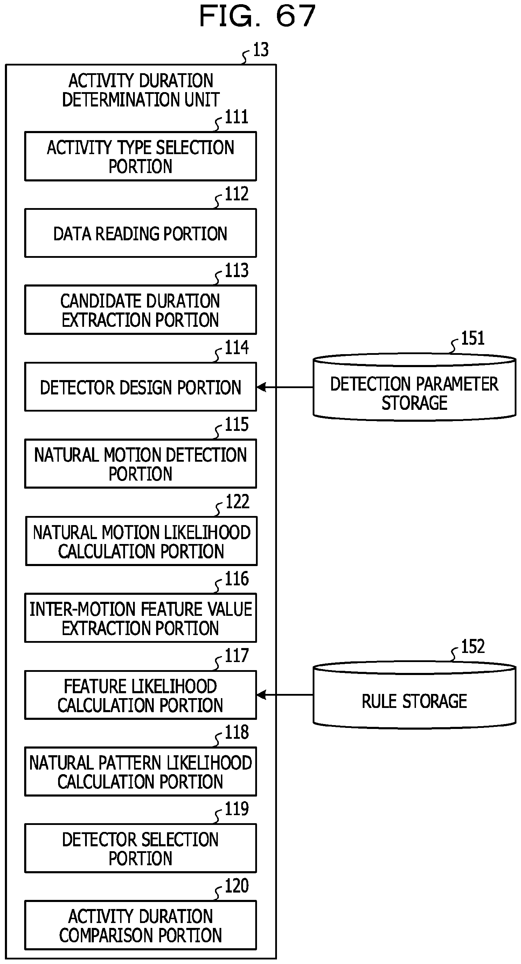

FIG. 67 is a diagram illustrating a functional configuration example of an activity duration determination unit according to the fifth embodiment. In FIG. 67, the same reference sign is applied to the same part as one in FIG. 3, and the description thereof is omitted.

In FIG. 67, the activity duration determination unit 13 further includes a natural motion likelihood calculation portion 122. The natural motion likelihood calculation portion 122 calculates a natural motion likelihood for each candidate motion group.

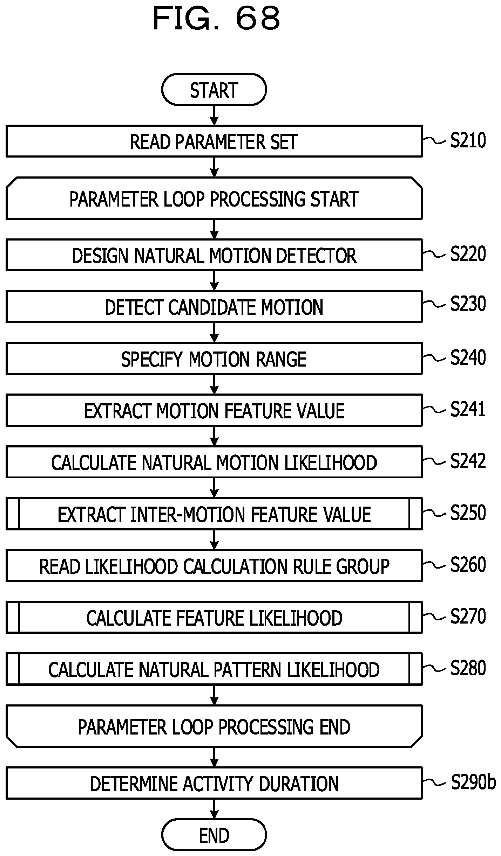

FIG. 68 is a flowchart illustrating an example of a procedure for activity duration determination processing according to the fifth embodiment. In FIG. 68, the same step number is applied to the same step as one in FIG. 23, and the description thereof is omitted. In FIG. 68, steps S241 and S242 are executed subsequently to step S240.

In step S241, the natural motion likelihood calculation portion 122 extracts a motion feature value of a natural motion corresponding to a target activity type, for each candidate motion in a target candidate duration. The motion feature value is an index indicating a feature of each of four types of natural motions, and is discriminated from an inter-motion feature value that is a feature value between candidate motions.

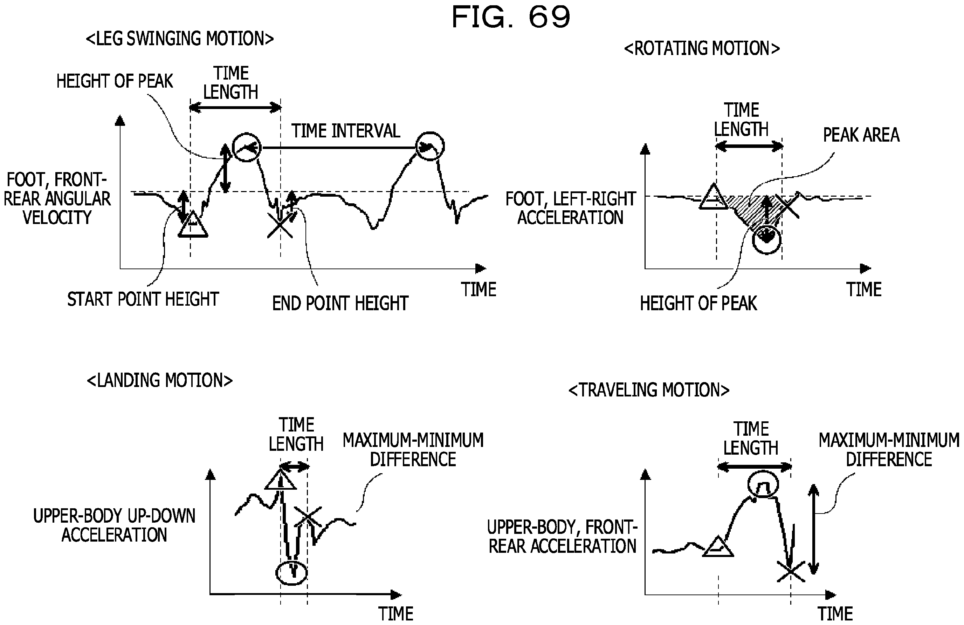

FIG. 69 includes diagrams each illustrating an example of a motion feature value of each natural motion. Note that, in FIG. 69, sign △ indicates a start point. Sign O indicates a peak. Sign × indicates an end point.

Motion feature values of a leg swinging motion are five features including (1) time interval, (2) time length, (3) height of start point, (4) height of end point, and (5) height of peak. (1) Time interval is a time interval between a peak of the candidate motion and a peak of the next candidate motion. Time length is a time interval from the start point to the end point of the candidate motion. (3) Height of start point is a signal value at the start point. (4) Height of end point is a signal value at the end point. (5) Height of peak is a signal value at a peak.

Motion feature values of a rotating motion are three features including (1) time length, (2) height of peak, and (3) area of peak. (1) Time length is a time interval from the start point to the end point. (2) Height of peak is a signal value at a peak. (3) Area of peak is an integral value of signal values from the start point to the end point.

Motion feature values of a landing motion are two features including (1) maximum-minimum difference and (2) time length. (1) Maximum-minimum difference is a difference between the maximum value and the minimum value of signals in a motion range. (2) Time length is a time interval from the start point to the end point.

Motion feature values of a traveling motion are two features including (1) maximum-minimum difference and (2) time length. (1) Maximum-minimum difference is a difference between the maximum value and the minimum value of signals in a motion range. (2) Time length is a time interval from the start point to the end point.

Note that a natural motion corresponding to a target motion is as illustrated in FIG. 5. For example, information indicated in FIG. 5 may be previously stored in the auxiliary storage device 102. For example, when motion sensor data for both ankles is acquired and if a target activity type is "walking", a natural motion corresponding to the target activity type (hereinafter, referred to as "target natural motion") is a leg swinging motion and a landing motion. The natural motion likelihood calculation portion 122 calculates a natural motion likelihood for one or both of these.

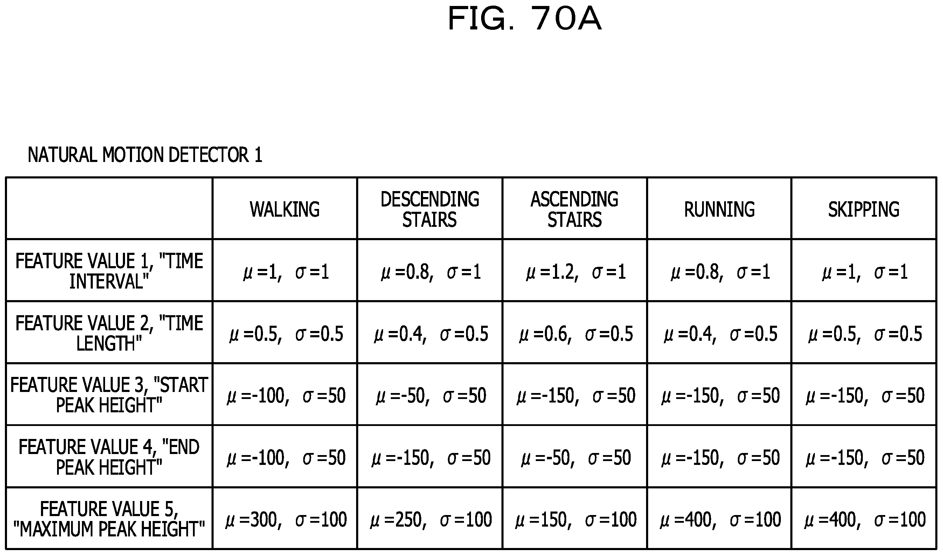

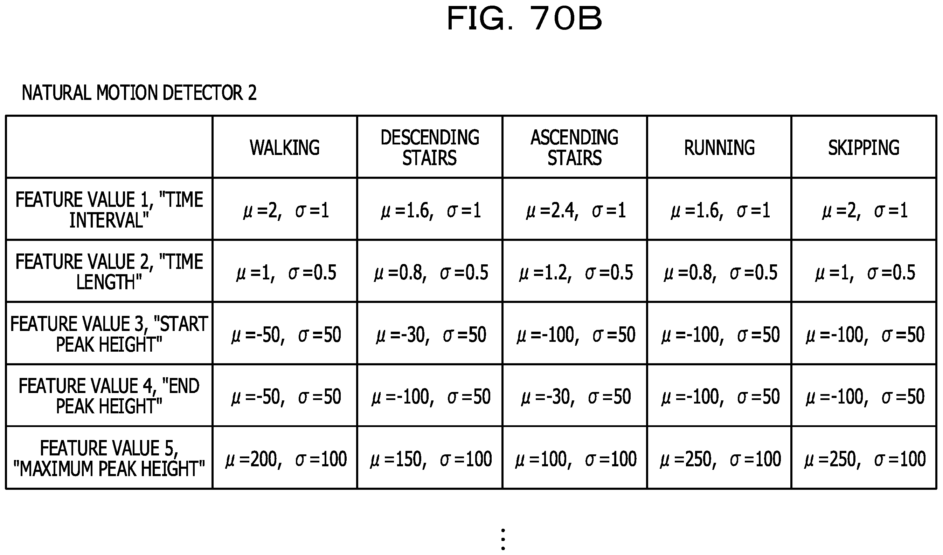

First, the natural motion likelihood calculation portion 122 calculates a likelihood element by using a probability distribution containing a predetermined parameter per candidate motion in the target candidate duration. The likelihood element is an index that is calculated for one candidate motion and one motion feature value, and is a component of a natural motion likelihood. A method of calculating the likelihood element is described later. The shape of a probability distribution may be one of a binomial distribution, a multinomial distribution, a normal distribution, and a continuous uniform distribution, for example, as illustrated in FIG. 17 similarly to the case of obtaining a feature likelihood. These basically represent "the degree of closeness to a previously set reference value parameter" of an observed motion feature value, or "whether falling in a previously set range" of the observed motion feature value. The reference value parameter is set per activity type and per type of natural motion. Further, the reference value parameter may be set per type of natural motion detector. FIGs. 70A and 70B illustrate an example of a reference value parameter set for a leg swinging motion per type of natural motion detector.

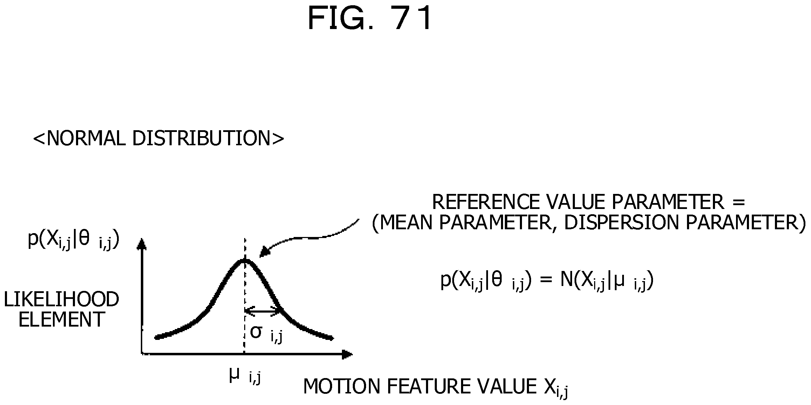

For example, a likelihood element of a motion feature value i according to a normal distribution for a j-th candidate motion in a target candidate duration is calculated by using an expression indicated in FIG. 71. In FIG. 71, x denotes a motion feature value, i denotes a number of a motion feature value, and $p(x_{i,j}|\theta_i)$ denotes a likelihood element.

The natural motion likelihood calculation portion 122 calculates a likelihood element per motion feature value of a target natural motion, for each candidate motion in a target candidate duration. If target natural motions are a leg swinging motion and a landing motion, a likelihood element relating to one of the leg swinging motion and the landing motion may be calculated, or likelihood elements relating to both may be calculated.

Then, the natural motion likelihood calculation portion 122 calculates a natural motion likelihood for a candidate motion group in a target candidate duration (S242). A natural motion likelihood $L_{action}$ is calculated by using the following expression.

[Math. 10]

$$L_{action} = \left[ \prod_{i=1}^{N} \prod_{j=1}^{M} p(x_{i,j} \mid \theta_{i,j}) \right]^{\frac{1}{NM}}$$

Note that i is a number of a motion feature value, j is a number of a candidate motion, N is the number of motion feature values, and M is the number of candidate motions. For example, a natural motion likelihood relating to a leg swinging motion is N = 5.

Specifically, the natural motion likelihood is calculated by the product of all motion feature values of a target natural motion calculated for all candidate motions in a target candidate duration.

Note that if a plurality of target natural motions exist, a plurality of natural motion likelihoods may be calculated, or a natural motion likelihood relating to one of target natural motions may be calculated.

In step S290b in FIG. 68, the detector selection portion 119 determines an activity duration also in consideration of a natural motion likelihood for a target candidate duration. For example, when a natural motion likelihood calculated for a candidate motion group in a target candidate duration is $L_{action}$, and a natural pattern likelihood is $L_{pattern}$, a value L obtained by multiplying these values, $L = L_{action} \times L_{pattern}$ serves as an evaluation value. The evaluation value P is calculated per natural motion detector. If the maximum value among evaluation values P of respective natural motion detectors exceeds a threshold, the detector selection portion 119 selects a candidate duration as an activity duration of a target activity type.

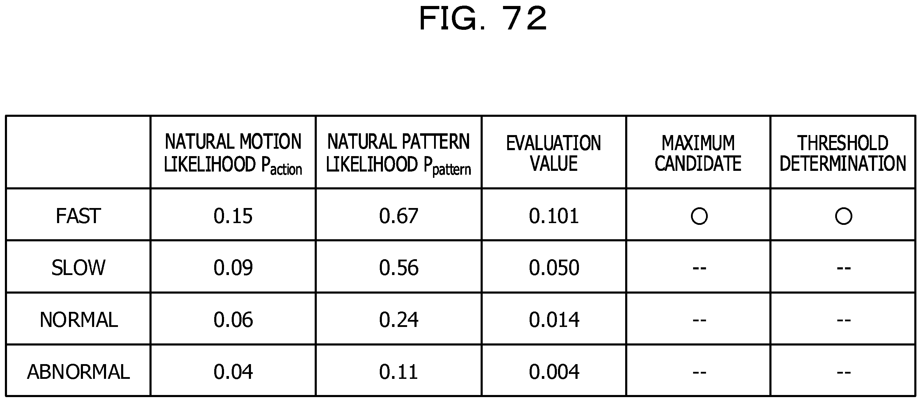

For example, when an evaluation value is calculated as indicated in FIG. 72, since the evaluation value of "Fast" is the maximum, the processing result of "Fast" is employed if the evaluation value exceeds a threshold.

As described above, an activity duration may be determined also in consideration of a likelihood that a candidate motion group is a natural motion corresponding to a target activity type. Thus, an improvement in accuracy of a determined duration of an activity duration may be expected.

A sixth embodiment is described next. According to the sixth embodiment, only the points different from those of the above-described embodiments are described. The points not described according to the sixth embodiment may be similar to those according to the above-described embodiments.

Taking into account according to the sixth embodiment is a possibility that an activity duration is not detected at all by any of a plurality of natural motion detectors based on parameter lists of previously set detection parameters.

When the activity duration determination unit 13 detects an activity duration from sensor data for a predetermined period or longer and a distribution of occurrence frequencies of each activity type is obtained, if a distribution of occurrence frequencies is unnatural, such as if walking is not detected at all, the detector design portion 114 plots a plurality of natural pattern likelihoods calculated until step S107 on coordinates having each detection parameter constituting a parameter list as a dimension. The detector design portion 114 determines a direction in which the natural pattern likelihood increases based on the plotted result, and determines a value of each detection parameter in that direction. The natural motion detection portion 115 designs a natural motion detector based on the determined value. Thereafter, step S230 and later steps in FIG. 23 may be executed based on the natural motion detector.

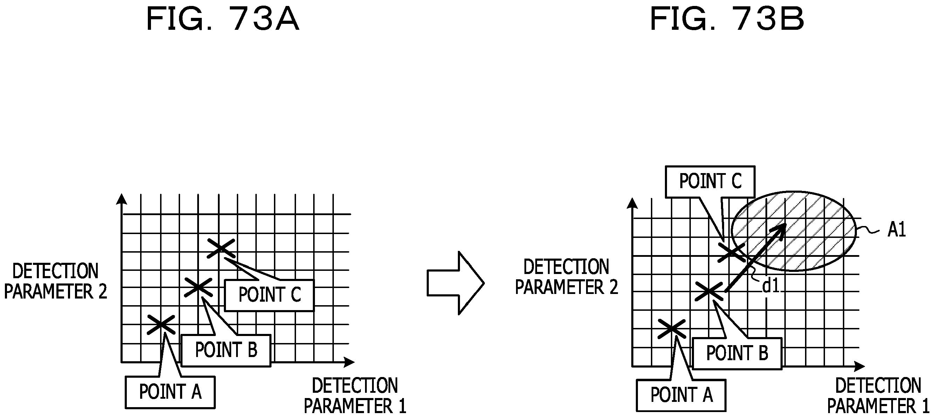

For example, FIGs. 73A and 73B illustrate examples in which two detection parameters constitute a parameter list for the convenience of description (note that, according to this embodiment, five detection parameters constitute a parameter list as illustrated in FIG. 24). FIG. 73A illustrates an example in which a point A, a point B, and a point C that are natural pattern likelihoods calculated based on three parameter lists (that is, three natural motion detectors) are plotted for one candidate section on a coordinate system having two detection parameters as dimensions. FIG. 73B illustrates an example in which a direction d1 in which the natural pattern likelihood increases is determined based on the point A, point B, and point C, and a value corresponding to a region A1 existing at a leading position in the direction d1 is re-set to each detection parameter (that is, a new detection parameter is generated).

As described above, according to the sixth embodiment, a possibility that an activity duration is successfully detected may be increased.

Note that, in each of the above-described embodiments, the activity duration determination unit 13 is an example of an information processing apparatus. The candidate duration extraction portion 113 is an example of a specification unit. The detector design portion 114 is an example of a design unit. The natural motion detection portion 115 is an example of a detection unit. The natural pattern likelihood calculation portion 118 is an example of a first calculation unit. The inter-motion feature value extraction portion 116 is an example of a first extraction unit. The feature likelihood calculation portion 117 is an example of a conversion unit. The candidate duration classification portion 121 is an example of a classification unit. The activity duration comparison portion 120 is an example of a determination unit. The natural motion likelihood calculation portion 122 is an example of a second calculation unit.


Reference Signs List

| 10 | activity duration determination apparatus |
| 11 | data collection unit |
| 12 | data pre-processing unit |
| 13 | activity duration determination unit |
| 14 | data processing unit |
| 15 | determination result storage unit |
| 16 | communication unit |
| 20 | output device |
| 21 | visualization unit |
| 30L | left-leg sensor device |
| 30R | right-leg sensor device |
| 30U | upper-body sensor device |
| 40 | data acquisition device |
| 100 | drive device |
| 101 | recording medium |
| 102 | auxiliary storage device |
| 103 | memory device |
| 104 | CPU |
| 105 | interface device |
| 111 | activity type selection portion |
| 112 | data reading portion |
| 113 | candidate duration extraction portion |
| 114 | detector design portion |
| 115 | natural motion detection portion |
| 116 | inter-motion feature value extraction portion |
| 117 | feature likelihood calculation portion |

118    natural pattern likelihood calculation portion
119    detector selection portion
120    activity duration comparison portion
121    candidate duration classification portion
122    natural motion likelihood calculation portion
151    detection parameter storage unit
152    rule storage unit
153    state transition probability storage
B    bus

**Claims**

1. An information processing apparatus, comprising:

a specification unit (113) configured to extract a duration of a predetermined activity based on sensor data indicating a motion of at least one region of a body in time series;
a detection unit (115) configured to detect a motion group in the duration based on the sensor data;
a first extraction unit (116) configured to extract a feature value between a first motion and a second motion in the detected motion group;
a conversion unit (117) configured to convert the extracted feature value into an index indicating a degree by which the feature value is likely the predetermined activity; and
a first calculation unit (118) configured to calculate and output, based on the index converted by the conversion unit, a likelihood that the motion group is the predetermined activity,
wherein the first extraction unit (116) is configured to extract, as the feature value, one of degrees including
a degree by which speed features or time features of motions are similar to one another between the first and second motions,
a degree by which regions to which motions belong are alternate between the first and second motions,
a degree by which occurrence time points of motions are simultaneous between the first and second motions, and
a degree by which motions occur in a specific order between the first and second motions.

2. The information processing apparatus according to claim 1,
wherein the detection unit (115) is configured to detect a peak of a predetermined value or larger from data in which sensor data indicating a predetermined physical amount in a predetermined axis direction or in a predetermined plane of at least one joint is converted into data in a predetermined frequency range.

3. The information processing apparatus according to claim 1 or 2,
wherein the detection unit (115) is configured to apply a plurality of conditions for detecting the motion group to the sensor data, and detect the motion group per each of the conditions, and
wherein the first calculation unit (118) is configured to calculate, per each of the conditions, based on the feature of the motion group detected based on the condition, the likelihood that the motion group is the predetermined activity.

4. The information processing apparatus according to claim 3, comprising:
a design unit (114) configured to specify an occurrence frequency distribution of the activity from sensor data for a predetermined period or longer, and when the occurrence frequency distribution does not satisfy a predetermined condition, design a new condition based on a plurality of the likelihoods individually calculated for the plurality of conditions.

5. The information processing apparatus according to claim 1, comprising:

a second calculation unit (122) configured to extract, per motion constituting the detected motion group, a feature value of each motion, convert the extracted feature value into an index indicating a degree by which the feature value is likely the predetermined activity, and calculate a second likelihood based on the converted index,
wherein the first calculation unit (118) is configured to calculate the likelihood by adding the second likelihood.

6. The information processing apparatus according to any one of claims 1 to 5, comprising:

a classification unit (121) configured to assign a plurality of first states of the predetermined activity or a second state of an activity other than the predetermined activity to each motion constituting the detected motion group based on state transition probability information and state observation probability information relating to the first states and the second state, and classify the duration specified by the specification unit (113) into a first duration relating to a motion to which the first states are assigned and a second duration relating to a motion to which the second state is assigned,

wherein the first calculation unit (118) is configured to calculate, based on a feature of a motion group relating to the first duration included in the detected motion group, the likelihood that the motion group is the predetermined activity.

7. The information processing apparatus according to claim 6,
wherein the classification unit (121) is configured to form a state group by adding states which differ from one another individually for regions, form a state group by adding a state indicating start of the predetermined activity, a state indicating end of the predetermined activity, and a state indicating not being the predetermined activity, form a state group by adding a state relating to an activity which differs from the predetermined activity, and assign one of states included in the formed state groups to each motion constituting the detected motion group.

8. The information processing apparatus according to claim 6 or 7,
wherein the classification unit (121) is configured to learn the state transition probability information and the state observation probability information based on sensor data for the specified duration, and classify the specified duration into the first duration and the second duration based on the state transition probability information and the state observation probability information which have been learned.

9. The information processing apparatus according to claim 8,
wherein the classification unit (121) is configured to use the state transition probability information and the state observation probability information which have been learned, in accordance with similarity between the state transition probability information and the state observation probability information which have been learned, and the state transition probability information and the state observation probability information which exist.

10. The information processing apparatus according to any one of claims 1 to 9,
wherein the first calculation unit (118) is configured to calculate, per each of a plurality of types of activities, based on a feature of a detected motion group, a likelihood that the motion group is a motion group relating to the respective types of the activities, and
wherein the information processing apparatus comprises a determination unit configured to determine a type of an activity in the duration based on a combination of the types of the activities and a likelihood per each of the types of the activities.

11. An information processing method executed by a computer, comprising: extracting a duration of a predetermined activity based on sensor data indicating a motion of at least one region of a body in time series; detecting a motion group in the duration based on the sensor data; extracting a feature value between a first motion and a second motion in the detected motion group; converting the extracted feature value into an index indicating a degree by which the feature value is likely the predetermined activity; and calculating and outputting, based on the index converted in the processing of converting, a likelihood that the motion group is the predetermined activity,
wherein the processing of extracting comprises extracting, as the feature value, one of degrees including a degree by which speed features or time features of motions are similar to one another between the first and second motions,
a degree by which regions to which motions belong are alternate between the first and second motions,
a degree by which occurrence time points of motions are simultaneous between the first and second motions, and
a degree by which motions occur in a specific order between the first and second motions.

12. The information processing method according to claim 11,
wherein the processing of detecting comprises detecting a peak of a predetermined value or larger from data in which sensor data indicating a predetermined physical amount in a predetermined axis direction or in a predetermined plane of at least one joint is converted into data in a predetermined frequency range.

13. An information processing program that causes a computer to execute the method of claim 11 or claim 12.

**Patentansprüche**

1. Informationsverarbeitungsvorrichtung, umfassend:

   eine Spezifikationseinheit (113), die dazu ausgelegt ist, eine Dauer einer vorbestimmten Aktivität basierend auf Sensordaten zu extrahieren, die eine Bewegung mindestens einer Region eines Körpers in Zeitreihen anzeigen;
   eine Erkennungseinheit (115), die dazu ausgelegt ist, während der Dauer eine Bewegungsgruppe basierend auf den Sensordaten zu erkennen;
   eine erste Extraktionseinheit (116), die dazu ausgelegt ist, zwischen einer ersten Bewegung und einer zweiten Bewegung in der erkannten Bewegungsgruppe einen Merkmalswert zu extrahieren;
   eine Umwandlungseinheit (117), die dazu ausgelegt ist, den extrahierten Merkmalswert in einen Index umzuwandeln, der einen Grad anzeigt, zu dem der Merkmalswert wahrscheinlich die vorbestimmte Aktivität ist; und
   eine erste Berechnungseinheit (118), die dazu ausgelegt ist, basierend auf dem von der Umwandlungs-einheit umgewandelten Index eine Wahrscheinlichkeit dafür, dass die Bewegungsgruppe die vorbestimmte Aktivität ist, zu berechnen und auszugeben;
   wobei die erste Extraktionseinheit (116) dazu ausgelegt ist, als den Merkmalswert einen von Graden zu extrahieren, die aufweisen
   einen Grad, zu dem Geschwindigkeitsmerkmale oder Zeitmerkmale von Bewegungen zwischen der ersten und der zweiten Bewegung einander ähnlich sind,
   einen Grad, zu dem Regionen, zu denen Bewegungen gehören, zwischen der ersten und der zweiten Bewegung wechseln,
   einen Grad, zu dem Auftrittszeitpunkte von Bewegungen gleichzeitig zwischen der ersten und der zweiten Bewegung sind, und
   einen Grad, zu dem Bewegungen in einer spezifischen Reihenfolge zwischen der ersten und der zweiten Bewegung auftreten.

2. Informationsverarbeitungsvorrichtung nach Anspruch 1,
   wobei die Erkennungseinheit (115) dazu ausgelegt ist, eine Spitze eines vorbestimmten Werts, oder größer, aus Daten zu erkennen, in denen Sensordaten, die eine vorbestimmte physikalische Größe in einer vorbestimmten Achsenrichtung oder in einer vorbestimmten Ebene mindestens eines Gelenks anzeigen, in Daten in einem vorbestimmten Frequenzbereich umgewandelt sind.

3. Informationsverarbeitungsvorrichtung nach Anspruch 1 oder 2,
   wobei die Erkennungseinheit (115) dazu ausgelegt ist, mehrere Bedingungen zum Erkennen der Bewegungsgruppe auf die Sensordaten anzuwenden, und die Bewegungsgruppe für jede der Bedingungen zu erkennen, und
   wobei die erste Berechnungseinheit (118) dazu ausgelegt ist, für jede der Bedingungen, basierend auf dem Merkmal der Bewegungsgruppe, die basierend auf der Bedingung erkannt wird, die Wahrscheinlichkeit dafür zu berechnen, dass die Bewegungsgruppe die vorbestimmte Aktivität ist.

4. Informationsverarbeitungsvorrichtung nach Anspruch 3, umfassend:
   eine Entwurfseinheit (114), die dazu ausgelegt ist, eine Auftrittshäufigkeitsverteilung der Aktivität aus Sensordaten für einen vorbestimmten Zeitraum oder länger zu spezifizieren, und wenn die Auftrittshäufigkeits-verteilung eine vorbestimmte Bedingung nicht erfüllt, einen neue Bedingung basierend auf mehreren der Wahrscheinlichkeiten, die individuell für die mehreren Bedingungen berechnet wurden, zu entwerfen.

5. Informationsverarbeitungsvorrichtung nach Anspruch 1, umfassend:

   eine zweite Berechnungseinheit (122), die dazu ausgelegt ist, pro Bewegung, die die erkannte Bewegungs-gruppe bildet, einen Merkmalswert jeder Bewegung zu extrahieren, den extrahierten Merkmalswert in einen Index umzuwandeln, der einen Grad anzeigt, zu dem der Merkmalswert wahrscheinlich die vorbestimmte Aktivität ist, und eine zweite Wahrscheinlichkeit basierend auf dem umgewandelten Index zu berechnen,
   wobei die erste Berechnungseinheit (118) dazu ausgelegt ist, die Wahrscheinlichkeit durch Addieren der zweiten Wahrscheinlichkeit zu berechnen.

6. Informationsverarbeitungsvorrichtung nach einem der Ansprüche 1 bis 5, umfassend:

   eine Klassifizierungseinheit (121), die dazu ausgelegt ist, mehrere erste Zustände der vorbestimmten Aktivität oder einen zweiten Zustand einer anderen Aktivität als der vorbestimmten Aktivität jeder Bewegung zuzuweisen, die die erkannte Bewegungsgruppe bildet, basierend auf Zustands-Übergangswahrscheinlichkeits-Informationen und Zustands-Beobachtungswahrscheinlichkeits-Informationen, die sich auf die ersten Zustände und den zweiten Zustand beziehen, und die durch die Spezifikationseinheit (113) spezifizierte Dauer in eine erste Dauer, die sich auf eine Bewegung bezieht, der die ersten Zustände zugewiesen sind, und eine zweite Dauer, die sich auf eine Bewegung bezieht, der der zweite Zustand zugewiesen ist, zu klassifizieren, wobei die erste Berechnungseinheit (118) dazu ausgelegt ist, basierend auf einem Merkmal einer Bewegungsgruppe, die sich auf die erste in der erkannten Bewegungsgruppe enthaltene Dauer bezieht, die Wahrscheinlichkeit dafür zu berechnen, dass die Bewegungsgruppe die vorbestimmte Aktivität ist.

7. Informationsverarbeitungsvorrichtung nach Anspruch 6,
wobei die Klassifizierungseinheit (121) dazu ausgelegt ist, eine Zustandsgruppe zu bilden durch Addieren von Zuständen, die sich für Regionen individuell voneinander unterscheiden, eine Zustandsgruppe zu bilden durch Addieren eines Zustands, der einen Beginn der vorbestimmten Aktivität anzeigt, eines Zustands, der ein Ende der vorbestimmten Aktivität anzeigt, und eines Zustands, der anzeigt, nicht die vorbestimmte Aktivität zu sein, eine Zustandsgruppe zu bilden zu durch Addieren eines Zustands, der sich auf eine Aktivität bezieht, die sich von der vorbestimmten Aktivität unterscheidet, und jeder Bewegung, die die erkannte Bewegungsgruppe bildet, einen von Zuständen, die in den gebildeten Zustandsgruppen enthalten sind, zuzuweisen.

8. Informationsverarbeitungsvorrichtung nach Anspruch 6 oder 7,
wobei die Klassifizierungseinheit (121) dazu ausgelegt ist, die Zustands-Übergangswahrscheinlichkeits-Informationen und die Zustands-Beobachtungswahrscheinlichkeits-Informationen basierend auf Sensordaten für die spezifizierte Dauer zu lernen, und die spezifizierte Dauer basierend auf den Zustands-Übergangswahrscheinlichkeits-Informationen und den Zustands-Beobachtungswahrscheinlichkeits-Informationen, die gelernt wurden, in die erste Dauer und die zweite Dauer zu klassifizieren.

9. Informationsverarbeitungsvorrichtung nach Anspruch 8,
wobei die Klassifizierungseinheit (121) dazu ausgelegt ist, die Zustands-Übergangswahrscheinlichkeits-Informationen und die Zustands-Beobachtungswahrscheinlichkeits-Informationen, die gelernt wurden, gemäß einer Ähnlichkeit zwischen den Zustands-Übergangswahrscheinlichkeits-Informationen und den Zustands-Beobachtungswahrscheinlichkeits-Informationen, die gelernt wurden, und den Zustands-Übergangswahrscheinlichkeits-Informationen und den Zustands-Beobachtungswahrscheinlichkeits-Informationen, die existieren, zu verwenden.

10. Informationsverarbeitungsvorrichtung nach einem der Ansprüche 1 bis 9,
wobei die erste Berechnungseinheit (118) dazu ausgelegt ist, für jede von mehreren Typen von Aktivitäten basierend auf einem Merkmal einer erkannten Bewegungsgruppe eine Wahrscheinlichkeit dafür zu berechnen, dass die Bewegungsgruppe eine Bewegungsgruppe ist, die sich auf die jeweiligen Typen der Aktivitäten bezieht, und wobei die Informationsverarbeitungsvorrichtung eine Bestimmungseinheit umfasst, die dazu ausgelegt ist, einen Typ einer Aktivität in der Dauer basierend auf einer Kombination der Typen der Aktivitäten und einer Wahrscheinlichkeit für jeden der Typen der Aktivitäten zu bestimmen.

11. Informationsverarbeitungsverfahren, das von einem Computer ausgeführt wird, umfassend:

Extrahieren einer Dauer einer vorbestimmten Aktivität basierend auf Sensordaten, die eine Bewegung mindestens einer Region eines Körpers in Zeitreihen anzeigen;
Erkennen einer Bewegungsgruppe während der Dauer basierend auf den Sensordaten;
Extrahieren eines Merkmalswerts zwischen einer ersten Bewegung und einer zweiten Bewegung in der erkannten Bewegungsgruppe;
Umwandeln des extrahierten Merkmalswerts in einen Index, der einen Grad anzeigt, zu dem der Merkmalswert wahrscheinlich die vorbestimmte Aktivität ist; und
Berechnen und Ausgeben einer Wahrscheinlichkeit, dass die Bewegungsgruppe die vorbestimmte Aktivität ist, basierend auf dem Index, der bei der Verarbeitung der Umwandlung umgewandelt wird;
wobei die Verarbeitung des Extrahierens ein Extrahieren eines von Graden umfasst, die aufweisen
einen Grad, zu dem Geschwindigkeitsmerkmale oder Zeitmerkmale von Bewegungen zwischen der ersten und der zweiten Bewegung einander ähnlich sind,
einen Grad, zu dem Regionen, zu denen Bewegungen gehören, zwischen der ersten und der zweiten Bewegung wechseln,
einen Grad, zu dem Auftrittszeitpunkte von Bewegungen gleichzeitig zwischen der ersten und der zweiten Bewegung sind, und

einen Grad, zu dem Bewegungen in einer bestimmten Reihenfolge zwischen der ersten und der zweiten Bewegung auftreten.

**12.** Informationsverarbeitungsverfahren nach Anspruch 11, wobei die Verarbeitung eines Erkennens ein Erkennen eines Peaks eines vorbestimmten Werts, oder größer, aus Daten umfasst, in denen Sensordaten, die eine vorbestimmte physikalische Größe in einer vorbestimmten Achsenrichtung oder in einer vorbestimmten Ebene mindestens eines Gelenks anzeigen, in Daten in einem vorbestimmten Frequenzbereich umgewandelt werden.

**13.** Informationsverarbeitungsprogramm, das bewirkt, dass ein Computer das Verfahren nach Anspruch 11 oder Anspruch 12 ausführt.

**Revendications**

**1.** Appareil de traitement d'informations, comprenant :

une unité de spécification (113) configurée de manière à extraire une durée d'une activité prédéterminée, sur la base de données de capteurs indiquant un mouvement d'au moins une région d'un corps dans une série temporelle ;
une unité de détection (115) configurée de manière à détecter un groupe de mouvements sur la durée, sur la base des données de capteurs ;
une première unité d'extraction (116) configurée de manière à extraire une valeur de caractéristique entre un premier mouvement et un second mouvement dans le groupe de mouvements détecté ;
une unité de conversion (117) configurée de manière à convertir la valeur de caractéristique extraite en un index indiquant un degré dans lequel la valeur de caractéristique est susceptible d'être l'activité prédéterminée ; et
une première unité de calcul (118) configurée de manière à calculer et fournir en sortie, sur la base de l'index converti par l'unité de conversion, une probabilité que le groupe de mouvements soit l'activité prédéterminée ;
dans lequel la première unité d'extraction (116) est configurée de manière à extraire, en tant que la valeur de caractéristique, l'un parmi les degrés incluant :

un degré de similarité mutuelle de caractéristiques de vitesse ou de caractéristiques temporelles de mouvements, entre les premier et second mouvements ;
un degré d'alternance de régions auxquelles des mouvements appartiennent, entre les premier et second mouvements ;
un degré de simultanéité d'instants d'occurrence de mouvements, entre les premier et second mouvements ; et
un degré d'occurrence de mouvements dans un ordre spécifique entre les premier et second mouvements.

**2.** Appareil de traitement d'informations selon la revendication 1,
dans lequel l'unité de détection (115) est configurée de manière à détecter un pic d'une valeur prédéterminée, ou d'une valeur supérieure à celle-ci, à partir de données dans lesquelles des données de capteurs indiquant une quantité physique prédéterminée dans une direction d'axe prédéterminée ou dans un plan prédéterminé d'au moins une articulation sont converties en des données dans une gamme de fréquences prédéterminée.

**3.** Appareil de traitement d'informations selon la revendication 1 ou 2,
dans lequel l'unité de détection (115) est configurée de manière à appliquer une pluralité de conditions pour détecter le groupe de mouvements, aux données de capteurs, et à détecter le groupe de mouvements pour chacune des conditions ; et
dans lequel la première unité de calcul (118) est configurée de manière à calculer, pour chacune des conditions, sur la base de la caractéristique du groupe de mouvements détecté sur la base de la condition, la probabilité que le groupe de mouvements soit l'activité prédéterminée.

**4.** Appareil de traitement d'informations selon la revendication 3, comprenant :
une unité de conception (114) configurée de manière à spécifier une distribution de fréquence d'occurrence de l'activité, à partir de données de capteurs, pour une période prédéterminée, ou une période plus longue que celle-ci, et lorsque la distribution de fréquence d'occurrence ne satisfait pas une condition prédéterminée, à concevoir une nouvelle condition sur la base d'une pluralité de probabilités calculées individuellement pour la pluralité de conditions.

5. Appareil de traitement d'informations selon la revendication 1, comprenant :

une seconde unité de calcul (122) configurée de manière à extraire, pour chaque mouvement constituant le groupe de mouvements détecté, une valeur de caractéristique de chaque mouvement, à convertir la valeur de caractéristique extraite en un index indiquant un degré dans lequel la valeur de caractéristique est susceptible d'être l'activité prédéterminée, et à calculer une seconde probabilité sur la base de l'index converti ;
dans lequel la première unité de calcul (118) est configurée de manière à calculer la probabilité en ajoutant la seconde probabilité.

6. Appareil de traitement d'informations selon l'une quelconque des revendications 1 à 5, comprenant :

une unité de classification (121) configurée de manière à affecter une pluralité de premiers états de l'activité prédéterminée, ou un second état d'une activité distincte de l'activité prédéterminée, à chaque mouvement constituant le groupe de mouvements détecté, sur la base d'informations de probabilité de transition d'état et d'informations de probabilité d'observation d'état connexes aux premiers états et au second état, et à classer la durée spécifiée par l'unité de spécification (113), en une première durée connexe à un mouvement auquel les premiers états sont affectés, et une seconde durée connexe à un mouvement auquel le second état est affecté ;
dans lequel la première unité de calcul (118) est configurée de manière à calculer, sur la base d'une caractéristique d'un groupe de mouvements connexe à la première durée, incluse dans le groupe de mouvements détecté, la probabilité que le groupe de mouvements soit l'activité prédéterminée.

7. Appareil de traitement d'informations selon la revendication 6,
dans lequel l'unité de classification (121) est configurée de manière à former un groupe d'états en ajoutant des états qui diffèrent les uns des autres, individuellement, pour des régions, à former un groupe d'états en ajoutant un état indiquant le début de l'activité prédéterminée, un état indiquant la fin de l'activité prédéterminée, et un état indiquant qu'il ne s'agit pas de l'activité prédéterminée, à former un groupe d'états en ajoutant un état connexe à une activité qui diffère de l'activité prédéterminée, et à affecter l'un parmi des états inclus dans les groupes d'états formés à chaque mouvement constituant le groupe de mouvements détecté.

8. Appareil de traitement d'informations selon la revendication 6 ou 7,
dans lequel l'unité de classification (121) est configurée de manière à apprendre les informations de probabilité de transition d'état et les informations de probabilité d'observation d'état, sur la base de données de capteurs pour la durée spécifiée, et à classer la durée spécifiée dans la première durée et la seconde durée, sur la base des informations de probabilité de transition d'état et des informations de probabilité d'observation d'état qui ont été apprises.

9. Appareil de traitement d'informations selon la revendication 8,
dans lequel l'unité de classification (121) est configurée de manière à utiliser les informations de probabilité de transition d'état et les informations de probabilité d'observation d'état qui ont été apprises, conformément à une similarité entre les informations de probabilité de transition d'état et informations de probabilité d'observation d'état qui ont été apprises, et les informations de probabilité de transition d'état et informations de probabilité d'observation d'état qui existent.

10. Appareil de traitement d'informations selon l'une quelconque des revendications 1 à 9,
dans lequel la première unité de calcul (118) est configurée de manière à calculer, pour chaque type d'une pluralité de types d'activités, sur la base d'une caractéristique d'un groupe de mouvements détecté, une probabilité que le groupe de mouvements soit un groupe de mouvements connexe aux types respectifs des activités ; et
dans lequel l'appareil de traitement d'informations comprend une unité de détermination configurée de manière à déterminer un type d'une activité sur la durée, sur la base d'une combinaison des types des activités et d'une probabilité pour chacun des types des activités.

11. Procédé de traitement d'informations exécuté par un ordinateur, le procédé comprenant les étapes ci-dessous consistant à :

extraire une durée d'une activité prédéterminée, sur la base de données de capteurs indiquant un mouvement d'au moins une région d'un corps dans une série temporelle ;

détecter un groupe de mouvements sur la durée, sur la base des données de capteurs ;

extraire une valeur de caractéristique entre un premier mouvement et un second mouvement dans le groupe de mouvements détecté ;

convertir la valeur de caractéristique extraite en un index indiquant un degré dans lequel la valeur de caractéristique est susceptible d'être l'activité prédéterminée ; et

calculer et fournir en sortie, sur la base de l'index converti à l'étape de conversion, une probabilité que le groupe de mouvements soit l'activité prédéterminée ;

dans lequel l'étape d'extraction consiste à extraire, en tant que la valeur de caractéristique, l'un parmi des degrés incluant :

un degré de similarité mutuelle de caractéristiques de vitesse ou de caractéristiques temporelles de mouvements, entre les premier et second mouvements ;

un degré d'alternance de régions auxquelles des mouvements appartiennent, entre les premier et second mouvements ;

un degré de simultanéité d'instants d'occurrence de mouvements, entre les premier et second mouvements ; et

un degré d'occurrence de mouvements dans un ordre spécifique entre les premier et second mouvements.

12. Procédé de traitement d'informations selon la revendication 11,
dans lequel l'étape de détection consiste à détecter un pic d'une valeur prédéterminée, ou d'une valeur supérieure à celle-ci, à partir de données dans lesquelles des données de capteurs indiquant une quantité physique prédéterminée dans une direction d'axe prédéterminée ou dans un plan prédéterminé d'au moins une articulation sont converties en des données dans une gamme de fréquences prédéterminée.

13. Programme de traitement d'informations qui amène un ordinateur à exécuter le procédé selon la revendication 11 ou 12.

# FIG. 1

10

```
        ┌──────────────┐        ┌──────────────┐
        │   104        │        │   105        │
        │              │        │  INTERFACE   │
        │     CPU      │        │   DEVICE     │
        └──────┬───────┘        └──────┬───────┘
               │                       │
  B ───────────┴───────────────────────┴──────────────────
     │                      │                   │
  ┌──┴─────────┐    ┌───────┴──────┐    ┌────────┴─────┐
  │   100      │    │   102        │    │   103        │
  │            │    │  AUXILIARY   │    │              │
  │DRIVE DEVICE│    │  STORAGE     │    │   MEMORY     │
  │            │    │  DEVICE      │    │   DEVICE     │
  └──┬─────────┘    └──────────────┘    └──────────────┘
     │
  ┌──┴─────────┐
  │   101      │
  │ RECORDING  │
  │  MEDIUM    │
  └────────────┘
```

# FIG. 2

| LEFT-LEG SENSOR DEVICE (30L) | ACTIVITY DURATION DETERMINATION APPARATUS (10) | OUTPUT DEVICE (20) |

**LEFT-LEG SENSOR DEVICE** — 30L
- DATA ACQUISITION UNIT → DATA STORAGE UNIT

**RIGHT-LEG SENSOR DEVICE** — 30R
- DATA ACQUISITION UNIT → DATA STORAGE UNIT

**ACTIVITY DURATION DETERMINATION APPARATUS** — 10
- DATA COLLECTION UNIT (11) → DATA PRE-PROCESSING UNIT (12) → ACTIVITY DURATION DETERMINATION UNIT (13) → DATA PROCESSING UNIT (14) → VISUALIZATION UNIT (21)
- DETERMINATION RESULT STORAGE UNIT (15)

**OUTPUT DEVICE** — 20
- VISUALIZATION UNIT (21)

EP 3 459 453 B1

# FIG. 3

13

## ACTIVITY DURATION DETERMINATION UNIT

### 111
ACTIVITY TYPE SELECTION PORTION

### 112
DATA READING PORTION

### 113
CANDIDATE DURATION EXTRACTION PORTION

### 114
DETECTOR DESIGN PORTION ◄——— 151 DETECTION PARAMETER STORAGE

### 115
NATURAL MOTION DETECTION PORTION

### 116
INTER-MOTION FEATURE VALUE EXTRACTION PORTION

### 117
FEATURE LIKELIHOOD CALCULATION PORTION ◄——— 152 RULE STORAGE

### 118
NATURAL PATTERN LIKELIHOOD CALCULATION PORTION

### 119
DETECTOR SELECTION PORTION

### 120
ACTIVITY DURATION COMPARISON PORTION

# FIG. 4

## FIG. 5

| BODY REGION | WALKING | RUNNING | DESCENDING STAIRS | ASCENDING STAIRS | SKIPPING | BICYCLING | TURNING |
|---|---|---|---|---|---|---|---|
| LEG | LEG SWINGING MOTION LANDING MOTION | SAME AS LEFT | SAME AS LEFT | SAME AS LEFT | SAME AS LEFT | ONLY LEG SWINGING MOTION | ROTATING MOTION |
| UPPER BODY | TRAVELING MOTION LANDING MOTION | SAME AS LEFT | SAME AS LEFT | SAME AS LEFT | SAME AS LEFT | ONLY TRAVELING MOTION | ROTATING MOTION |
| FOOT | LANDING MOTION | SAME AS LEFT | SAME AS LEFT | SAME AS LEFT | SAME AS LEFT | - | ROTATING MOTION |

EP 3 459 453 B1

# FIG. 6

EP 3 459 453 B1

# FIG. 7

LEFT-LEG GYRO
400 200 0 -200 -400

RIGHT-LEG GYRO
400 200 0 -200 -400

T2

T1

10:51:13    10:51:14    10:51:15

EXAMPLE OF INTERVAL CONSISTENCY DEGREE

$$f_{time}(n) = |T(n) - \mu_T|$$

T(n): TIME INTERVAL OF N-TH MOTION

$\mu_T$: TIME INTERVAL REFERENCE VALUE

EP 3 459 453 B1

# FIG. 8

EP 3 459 453 B1

HEIGHT OF PEAK

LEFT-LEG GYRO

RIGHT-LEG GYRO

10:51:13    10:51:14    10:51:15

EXAMPLE OF SPEED CONSISTENCY DEGREE

$$f_{speed}(n) = |V(n) - \mu_V|$$

V(n): SPEED FEATURE OF N-TH MOTION

$\mu_V$: SPEED REFERENCE VALUE

# FIG. 9

LEFT-LEG
GYRO — CURRENT MOTION

— NEIGHBORHOOD RANGE

RIGHT-LEG
GYRO — GYRO VALUE OF INVERSE LEG

END POINT OF    12:19:28    START POINT OF
PRECEDING MOTION             SUCCEEDING MOTION

EXAMPLE OF INVERSE-LEG
SUPPORT DEGREE

$$f_{inverse}(n) = \underset{t \in U}{mean}(gyro_{inverse}(t))$$

gyro_{inverse}(t): GYRO VALUE OF INVERSE LEG

U: NEIGHBORHOOD RANGE

EP 3 459 453 B1

# FIG. 10

BOTH-LEG LANDING DEGREE

LEFT-LEG GYRO
400 200 0 -200 -400

RIGHT-LEG GYRO
400 200 0 -200 -400

10:51:13    10:51:14    10:51:15

EXAMPLE OF BOTH-LEG LANDING DEGREE

$$\int_{Stance} (n) = T_{S(n+1)} - T_e(n)$$

$T_e(n)$: END TIME POINT OF N-TH MOTION

$T_s(n+1)$: START TIME POINT OF (n+1)-TH MOTION

EP 3 459 453 B1

# FIG. 11

OCCURRENCE POINT $T_1$ OF LANDING MOTION

LEFT-LEG GYRO

400 200 0 -200 -400

HIP ACCELERATION

14 12 10 8 6

MAXIMUM VALUE OF ADVANCEMENT AMOUNT NEAR LEG SWINGING TIME POINT $T_2$ OF INVERSE LEG

$[T_{2-\tau} , T_{2+\tau}]$

MINIMUM VALUE OF ADVANCEMENT AMOUNT NEAR LANDING TIME POINT $T_1$

$[T_{1-\varepsilon} , T_{1+\varepsilon}]$

EXAMPLE OF SIMULTANEOUS MOVEMENT DEGREE

$$f_{upper1} = \min_{t\in[T_1-\varepsilon, T_1+\varepsilon]} g_{waist}(t)$$

$$f_{upper2} = \max_{t\in[T_2-\tau, T_2+\tau]} g_{waist}(t)$$

EP 3 459 453 B1

# FIG. 12

# FIG. 13

# FIG. 14

# FIG. 15

# FIG. 16

| ACTIVITY TYPE | INTERVAL CONSISTENCY LIKELIHOOD | SPEED CONSISTENCY LIKELIHOOD | LEFT-RIGHT ALTERNATION LIKELIHOOD | UP-DOWN ALTERNATION LIKELIHOOD | INVERSE-LEG SUPPORT LIKELIHOOD | SIMULTANEOUS IMPACT LIKELIHOOD | SIMULTANEOUS MOVEMENT LIKELIHOOD | BOTH-LEG LANDING LIKELIHOOD | LANDING FLEXION LIKELIHOOD | TAKEOFF FLEXION LIKELIHOOD |
|---|---|---|---|---|---|---|---|---|---|---|
| WALKING | ○ | ○ | ○ | ○ | ○ | ○ | ○ | ○ | ○ | ○ |
| RUNNING | ○ | ○ | ○ | ○ | ○ | ○ | ○ | ◎ | ○ | ○ |
| DESCENDING STAIRS | ○ | ○ | ○ | ○ | ○ | ○ | ○ | ○ | ○ | ◎ |
| ASCENDING STAIRS | ○ | ○ | ○ | ○ | ○ | ○ | ○ | ○ | ◎ | ◎ |
| BICYCLING | ○ | ○ | ○ | × | ○ | × | × | × | × | × |
| TURNING | × | × | ○ | × | ○ | × | × | × | × | × |
| SKIPPING | ○ | ○ | ○ | ○ | ◎ | ○ | ○ | ○ | ○ | ○ |

EP 3 459 453 B1

# FIG. 17

(1) IF INTER-MOTION FEATURE VALUE IS DISCRETE VALUE

<BINOMIAL DISTRIBUTION>

FEATURE LIKELIHOOD

INTER-MOTION FEATURE VALUE

$$p(x) = \begin{cases} \alpha & (x=1) \\ 1-\alpha & (x=0) \end{cases}$$

<MULTINOMIAL DISTRIBUTION>

FEATURE LIKELIHOOD

INTER-MOTION FEATURE VALUE

$$p(x) = \begin{cases} \alpha 1 & (x=0) \\ \alpha 2 & (x=1) \\ \vdots \\ \alpha N & (x=N-1) \end{cases}$$

(2) IF INTER-MOTION FEATURE VALUE IS CONTINUOUS VALUE

<NORMAL DISTRIBUTION>

FEATURE LIKELIHOOD

DISPERSION PARAMETER $\sigma$

MIEAN PARAMETER $\mu$

INTER-MOTION FEATURE VALUE

$$p(x) = N(x| \mu, \sigma)$$

<CONTINUOUS UNIFORM DISTRIBUTION>

FEATURE LIKELIHOOD

INTER-MOTION FEATURE VALUE

$$p(x) = \begin{cases} \dfrac{1}{b-\alpha} & (\alpha < x < b) \\ 0 & \text{(OTHER THAN THE ABOVE)} \end{cases}$$

EP 3 459 453 B1

# FIG. 18

| SIMILARITY | ALTERNATION | SIMULTANEITY | ORDERING |
|---|---|---|---|
| INTERVAL CONSISTENCY LIKELIHOOD<br><br>SPEED CONSISTENCY LIKELIHOOD | LEFT-RIGHT ALTERNATION LIKELIHOOD<br><br>UP-DOWN ALTERNATION LIKELIHOOD | SIMULTANEOUS IMPACT LIKELIHOOD<br><br>SIMULTANEOUS MOVEMENT LIKELIHOOD<br><br>INVERSE-LEG SUPPORT LIKELIHOOD | BOTH-LEG LANDING LIKELIHOOD<br><br>LANDING FLEXION LIKELIHOOD<br><br>TAKEOFF FLEXION LIKELIHOOD |

# FIG. 19

```
                    START

            SELECT ACTIVITY TYPE              ~ S101

            READ SENSOR DATA                  ~ S102

         EXTRACT CANDIDATE DURATION           ~ S103

   CANDIDATE DURATION LOOP PROCESSING START

         SELECT CANDIDATE DURATION            ~ S104

      ACTIVITY DURATION DETERMINATION         ~ S105
               PROCESSING

    CANDIDATE DURATION LOOP PROCESSING END
                                        S106
   NO
            LAST ACTIVITY TYPE?
                      YES
   COMPARISON PROCESSING AMONG PLURAL          ~ S107
            ACTIVITY TYPES

                    END
```

# FIG. 20

| WALKING | BICYCLING | TURNING | SKIPPING | ASCENDING STAIRS | DESCENDING STAIRS | RUNNING |
|---------|-----------|---------|----------|------------------|-------------------|---------|
| TRUE | TRUE | TRUE | FALSE | TRUE | TRUE | FALSE |

EP 3 459 453 B1

# FIG. 21

# FIG. 22

EP 3 459 453 B1

| CANDIDATE DURATION | WALKING | BICYCLING | ... | RUNNING | CONFIRMATION RESULT |
|---|---|---|---|---|---|
| 1 | ◯ (0.67) | × | ... | ◯ (0.51) | WALKING |
| 2 | × | × | ... | × | NO ACTIVITY |
| ... | ... | ... | ... | ... | ... |

# FIG. 23

START

READ PARAMETER SET ～ S210

PARAMETER LOOP PROCESSING START

DESIGN NATURAL MOTION DETECTOR ～ S220

DETECT CANDIDATE MOTION ～ S230

SPECIFY MOTION RANGE ～ S240

EXTRACT INTER-MOTION FEATURE VALUE ～ S250

READ LIKELIHOOD CALCULATION RULE GROUP ～ S260

CALCULATE FEATURE LIKELIHOOD ～ S270

CALCULATE NATURAL PATTERN LIKELIHOOD ～ S280

PARAMETER LOOP PROCESSING END

DETERMINE ACTIVITY DURATION ～ S290

END

# FIG. 24

|  | TH_TimeDiff | TH_Amp | CutOffParam | MarginTime | Dimparam |
|---|---|---|---|---|---|
| FAST | 0.15 | 80 | 6 | 0.5 | 5 |
| SLOW | 0.5 | 35 | 3 | 0.7 | 3 |
| NORMAL | 0.3 | 50 | 5 | 0.6 | 4 |
| ABNORMAL | 0.7 | 20 | 1 | 0.7 | 2 |

# FIG. 25

| FILTER PASS BAND OF DETECTOR Slow | FILTER PASS BAND OF DETECTOR Normal | FILTER PASS BAND OF DETECTOR Fast |
|---|---|---|

FREQUENCY     FREQUENCY     FREQUENCY

0.0 ～ 0.1Hz     0.0 ～ 0.5Hz     0.0 ～ 3.0Hz

EP 3 459 453 B1

# FIG. 26

| | |
|---|---|
| —— | DATA BEFORE FILTERING |
| ---- | DATA AFTER FILTERING |
| ⬭ | PEAK |

LEFT-LEG GYRO

RIGHT-LEG GYRO

12:19:19   12:19:21   12:19:23   12:19:25   12:19:27   12:19:29   12:19:31   12:19:33   12:19:35

# FIG. 27

EP 3 459 453 B1

FIG. 28

# FIG. 29

| MOTION NUMBER | OCCURRENCE POINT | START POINT | END POINT | LEFT-RIGHT SIDE |
|---|---|---|---|---|
| 1 | 19:30:01.344 | 19:30:01.023 | 19:30:01.609 | LEFT |
| 2 | 19:30:02.785 | 19:30:02.461 | 19:30:03.021 | RIGHT |
| … | … | … | … | … |
| 27 | 19:30:01.344 | 19:30:01.023 | 19:30:01.609 | RIGHT |

# FIG. 30

| | LEFT-RIGHT ALTERNATION LIKELIHOOD | INTERVAL CONSISTENCY LIKELIHOOD | SPEED CONSISTENCY LIKELIHOOD | INVERSE-LEG SUPPORT LIKELIHOOD | SIMULTANEOUS MOVEMENT LIKELIHOOD | BOTH-LEG LANDING LIKELIHOOD | LANDING FLEXION LIKELIHOOD | TAKEOFF FLEXION LIKELIHOOD | |
|---|---|---|---|---|---|---|---|---|---|
| FUNCTION TYPE | BINOMIAL DISTRIBUTION | NORMAL DISTRIBUTION | NORMAL DISTRIBUTION | CONTINUOUS UNIFORM DISTRIBUTION | CONTINUOUS UNIFORM DISTRIBUTION | CONTINUOUS UNIFORM DISTRIBUTION | CONTINUOUS UNIFORM DISTRIBUTION | CONTINUOUS UNIFORM DISTRIBUTION | |
| FUNCTION PARAMETER | $\alpha = 0.95$ | $\mu = 0$ $\sigma = 1$ | $\mu = 0$ $\sigma = 30$ | a=-50 b=0 | a1-10 b1=0 a2=3 b2=10 | a=0 b=4 | a=-100 b=-10 | a=-100 b=-10 | ... |

EP 3 459 453 B1

# FIG. 31

| | FAST | SLOW | NORMAL | ABNORMAL |
|---|---|---|---|---|
| NATURAL PATTERN LIKELIHOOD | 0.85 | 0.12 | 0 | 0.32 |

# FIG. 32

START

NUMBER OF MOTIONS, LOOP PROCESSING START

CALCULATE LEFT-RIGHT ALTERNATION DEGREE — S301

CALCULATE INTERVAL CONSISTENCY DEGREE — S302

CALCULATE SPEED CONSISTENCY DEGREE — S303

CALCULATE INVERSE-LEG SUPPORT DEGREE — S304

CALCULATE BOTH-LEG LANDING DEGREE — S305

CALCULATE SIMULTANEOUS MOVEMENT DEGREE — S306

CALCULATE LANDING FLEXION DEGREE — S307

CALCULATE TAKEOFF FLEXION DEGREE — S308

CALCULATE UP-DOWN ALTERNATION DEGREE — S309

CALCULATE SIMULTANEOUS IMPACT DEGREE — S310

NUMBER OF MOTIONS, LOOP PROCESSING END

END

# FIG. 33

| MOTION NUMBER | LEFT-RIGHT ALTERNATION DEGREE | INTERVAL CONSISTENCY DEGREE | SPEED CONSISTENCY DEGREE | · · · |
|---|---|---|---|---|
| 1 | 1 | 0.1 | 23.9 | · · · |
| 2 | 1 | 0.03 | 14.5 | · · · |
| · · · | · · · | · · · | · · · | · · · |
| 27 | 1 | 0.03 | 15.0 | · · · |

# FIG. 34

START

NUMBER OF MOTIONS, LOOP PROCESSING START

CALCULATE LEFT-RIGHT ALTERNATION LIKELIHOOD — S401

CALCULATE INTERVAL CONSISTENCY LIKELIHOOD — S402

CALCULATE SPEED CONSISTENCY LIKELIHOOD — S403

CALCULATE INVERSE-LEG SUPPORT LIKELIHOOD — S404

CALCULATE BOTH-LEG LANDING LIKELIHOOD — S405

CALCULATE SIMULTANEOUS MOVEMENT LIKELIHOOD — S406

CALCULATE LANDING FLEXION LIKELIHOOD — S407

CALCULATE TAKEOFF FLEXION LIKELIHOOD — S408

CALCULATE UP-DOWN ALTERNATION LIKELIHOOD — S409

CALCULATE SIMULTANEOUS IMPACT LIKELIHOOD — S410

NUMBER OF MOTIONS, LOOP PROCESSING END

END

# FIG. 35

| MOTION NUMBER | 1 | 2 | 3 | 4 | 5 |
|---|---|---|---|---|---|
| LEFT-RIGHT ALTERNATION DEGREE | -- | 1 | 0 | 0 | 0 |

CALCULATE LIKELIHOOD

| MOTION NUMBER | 1 | 2 | 3 | 4 | 5 |
|---|---|---|---|---|---|
| LEFT-RIGHT ALTERNATION DEGREE | -- | 1 | 0 | 0 | 0 |
| LEFT-RIGHT ALTERNATION LIKELIHOOD | -- | 0.95 | 0.05 | 0.05 | 0.05 |

# FIG. 36

```
                    ┌─────────────┐
                    │    START    │
                    └──────┬──────┘
                           │
                           ▼
        ┌──────────────────────────────────────┐
        │    MOTION, LOOP PROCESSING START      │
        └──────────────────┬───────────────────┘
                           │
                           ▼
        ┌──────────────────────────────────────┐
        │ FEATURE LIKELIHOOD, LOOP PROCESSING START │
        └──────────────────┬───────────────────┘
                           │
                           ▼
        ┌──────────────────────────────────────┐
        │ MULTIPLY CORRESPONDING FEATURE LIKELIHOOD │──── S501
        └──────────────────┬───────────────────┘
                           │
                           ▼
        ┌──────────────────────────────────────┐
        │ FEATURE LIKELIHOOD, LOOP PROCESSING END │
        └──────────────────┬───────────────────┘
                           │
                           ▼
        ┌──────────────────────────────────────┐
        │     MOTION, LOOP PROCESSING END       │
        └──────────────────┬───────────────────┘
                           │
                           ▼
        ┌──────────────────────────────────────┐
        │              AVERAGE                  │──── S502
        └──────────────────┬───────────────────┘
                           │
                           ▼
        ┌──────────────────────────────────────┐
        │ WEIGHT IN ACCORDANCE WITH NUMBER OF MOTIONS │──── S503
        └──────────────────┬───────────────────┘
                           │
                           ▼
                    ┌─────────────┐
                    │     END     │
                    └─────────────┘
```

# FIG. 37

| WALKING | BICYCLING | TURNING | SKIPPING | RUNNING | DESCENDING STAIRS | ASCENDING STAIRS |
|---|---|---|---|---|---|---|
| 4 STEPS OR MORE | 20 STEPS OR MORE | 2 TO 10 STEPS | 10 STEPS OR MORE | 20 STEPS OR MORE | 10 TO 100 STEPS | 10 TO 100 STEPS |

EP 3 459 453 B1

# FIG. 38

| DURATION NUMBER | WALKING START TIME POINT | WALKING END TIME POINT | NUMBER OF STEPS |
|---|---|---|---|
| 1 | 2016-03-09 10:00:34.214 | 2016-03-09 10:00:56.600 | 54 |
| 1 | 2016-03-09 10:02:11.734 | 2016-03-09 10:05:22.550 | 530 |
| 1 | 2016-03-09 10:05:24.109 | 2016-03-09 10:05:28.117 | 23 |
| … | … | … | … |
| 67 | 2016-03-09 22:40:41.008 | 2016-03-09 22:40:43.433 | 17 |

## FIG. 39

ACTIVITY FEATURE VALUE

| DURATION NUMBER | WALKING START TIME POINT | WALKING END TIME POINT | NUMBER OF STEPS | STRIDE (m) (MEAN) | STRIDE (m) (STANDARD DEVIATION) | WALKING SPEED (m/s) (MEAN) | ... |
|---|---|---|---|---|---|---|---|
| 1 | 2016-03-09 10:00:34.214 | 2016-03-09 10:00:56.600 | 54 | 1.12 | 0.15 | 0.76 | ... |
| 2 | 2016-03-09 10:02:11.734 | 2016-03-09 10:05:22.550 | 530 | 1.32 | 0.05 | 0.89 | ... |
| 3 | 2016-03-09 10:05:24.109 | 2016-03-09 10:05:28.117 | 23 | 0.98 | 0.11 | 0.80 | ... |
| ... | ... | ... | ... | ... | ... | ... | ... |
| 67 | 2016-03-09 22:40:41.008 | 2016-03-09 22:40:43.443 | 17 | 0.76 | 0.12 | 0.65 | ... |

EP 3 459 453 B1

# FIG. 40

PATIENT ID : "P001"

ACTIVITY TYPE: "WALKING"

ACTIVITY FEATURE VALUE: "WALKING SPEED"

REHABILITATION (FIRST TIME)

REHABILITATION (SECOND TIME)

DAY CARE

HEALTHY RANGE

UPPER AND LOWER LIMITS OF ACTIVITY FEATURE VALUE

EP 3 459 453 B1

# FIG. 41

NORMAL WALKING

43

LEFT GYRO

RIGHT GYRO

14:30:09    14:30:37    14:30:58

WALKING WITH TOES HEADED INWARD

13    15

LEFT GYRO

RIGHT GYRO

05:44:15    05:44:43    05:45:04

WALKING WITH SHORT STEPS

25    32

LEFT GYRO

RIGHT GYRO

05:47:49    05:48:17    05:48:38

WALKING WITH NAILS SLIDING

21

LEFT GYRO

RIGHT GYRO

05:49:44    05:50:08    05:50:32

WALING WITH SWINGING

15

LEFT GYRO

RIGHT GYRO

05:51:10    05:51:28    05:51:52

WALKING WITH RIGHT LEG SLIDING

8    9    6    8

LEFT GYRO

RIGHT GYRO

05:41:42    05:42:18    05:42:54

UNSTABLE WALKING

28

LEFT GYRO

RIGHT GYRO

05:46:21    05:46:49    05:47:10

EP 3 459 453 B1

FIG. 42

EP 3 459 453 B1

# FIG. 43

OUTPUT DEVICE — 20

VISUALIZATION UNIT — 21

ACTIVITY DURATION DETERMINATION APPARATUS — 10

DATA COLLECTION UNIT — 11

DATA PRE-PROCESSING UNIT — 12

ACTIVITY DURATION DETERMINATION UNIT — 13

DATA PROCESSING UNIT — 14

DETERMINATION RESULT STORAGE UNIT — 15

LEFT-LEG SENSOR DEVICE — 30L

DATA ACQUISITION UNIT

DATA ACQUISITION UNIT

RIGHT-LEG SENSOR DEVICE — 30R

DATA ACQUISITION UNIT

DATA ACQUISITION UNIT

# FIG. 44

CANDIDATE DURATION

WALKING MOTION    TURNING (NON-WALKING MOTION)

LEFT-LEG GYRO

RIGHT-LEG GYRO

EP 3 459 453 B1

# FIG. 45

| MOTION NUMBER | LEFT-RIGHT ALTERNATION DEGREE | INTERVAL CONSISTENCY DEGREE | SPEED CONSISTENCY DEGREE | ... |
|---|---|---|---|---|
| 1 | 1 | 0.12 | 12.8 | ... |
| 2 | 1 | 0.12 | 21.1 | ... |
| 3 | 1 | 0.12 | 24.5 | ... |
| 4 | 1 | 0.12 | 21.1 | ... |
| 5 | 1 | 0.12 | 14.6 | ... |
| 6 | 1 | 0.12 | 19.6 | ... |
| 7 | 1 | 0.12 | 13.7 | ... |
| 8 | 1 | 0.12 | 9.8 | ... |
| 9 | 1 | 0.12 | 4.9 | ... |
| 10 | 1 | 0.12 | 2.94 | ... |
| 11 | 1 | 1.2 | 22.6 | ... |
| 12 | 1 | 1.4 | 32.4 | ... |
| 13 | 1 | 1.5 | 22.4 | ... |

# FIG. 46

(A)　　　　　(B)　　　　　(C)

# FIG. 47A

# FIG. 47B

| MOTION NUMBER | 1 | 2 | 3 | 4 | 5 | 6 | 7 | 8 |
|---|---|---|---|---|---|---|---|---|
| STATE | FIRST STEP (LEFT) | MIDDLE OF WALKING (RIGHT) | MIDDLE OF WALKING (LEFT) | MIDDLE OF WALKING (RIGHT) | MIDDLE OF WALKING (LEFT) | MIDDLE OF WALKING (RIGHT) | MIDDLE OF WALKING (LEFT) | MIDDLE OF WALKING (RIGHT) |

| 9 | 10 | 11 | 12 | 13 |
|---|---|---|---|---|
| MIDDLE OF WALKING (LEFT) | LAST STEP (RIGHT) | NON-WALKING | NON-WALKING | NON-WALKING |

EP 3 459 453 B1

# FIG. 48

ACTIVITY DURATION DETERMINATION
UNIT — 13

ACTIVITY TYPE SELECTION
PORTION — 111

DATA READING PORTION — 112

CANDIDATE DURATION
EXTRACTION PORTION — 113

DETECTOR DESIGN PORTION — 114 ◄— DETECTION PARAMETER
STORAGE — 151

NATURAL MOTION DETECTION
PORTION — 115

INTER-MOTION FEATURE VALUE
EXTRACTION PORTION — 116

FEATURE LIKELIHOOD
CALCULATION PORTION — 117 ◄— RULE STORAGE — 152

CANDIDATE DURATION
CLASSIFICATION PORTION — 121 ◄— STATE TRANSITION
PROBABILITY STORAGE — 152

NATURAL PATTERN LIKELIHOOD
CALCULATION PORTION — 118

DETECTOR SELECTION
PORTION — 119

ACTIVITY DURATION
COMPARISON PORTION — 120

# FIG. 49

START

READ PARAMETER SET $\quad$ S210

PARAMETER LOOP PROCESSING START

DESIGN NATURAL MOTION DETECTOR $\quad$ S220

DETECT CANDIDATE MOTION $\quad$ S230

SPECIFY MOTION RANGE $\quad$ S240

EXTRACT INTER-MOTION FEATURE VALUE $\quad$ S250

CLASSIFY BETWEEN TARGET DURATION AND NON-TARGET DURATION $\quad$ S251

TARGET DURATION LOOP PROCESSING START

SELECT ACTIVITY DURATION $\quad$ S271

ACQUIRE INTER-MOTION FEATURE VALUE OF SELECTED DURATION $\quad$ S272

CALCULATE NATURAL PATTERN LIKELIHOOD $\quad$ S280a

TARGET DURATION LOOP PROCESSING END

PARAMETER LOOP PROCESSING END

DETERMINE ACTIVITY DURATION $\quad$ S290a

END

# FIG. 50

```
           ┌─────────────┐
           │    START    │
           └─────────────┘
                  │
                  ▼
   ┌──────────────────────────────────┐
   │    STATE, LOOP PROCESSING START  │
   └──────────────────────────────────┘
                  │
                  ▼
   ┌──────────────────────────────────┐
   │  READ LIKELIHOOD CALCULATION RULE │──── S601
   │   CORRESPONDING TO TARGET STATE   │
   └──────────────────────────────────┘
                  │
                  ▼
   ┌──────────────────────────────────┐
   │     CALCULATE FEATURE LIKELIHOOD  │──── S602
   └──────────────────────────────────┘
                  │
                  ▼
   ┌──────────────────────────────────┐
   │     STATE, LOOP PROCESSING END    │
   └──────────────────────────────────┘
                  │
                  ▼
   ┌──────────────────────────────────┐
   │  CALCULATE STATE OBSERVATION PROBABILITY │──── S603
   └──────────────────────────────────┘
                  │
                  ▼
   ┌──────────────────────────────────┐
   │  ACQUIRE STATE TRANSITION PROBABILITY │──── S604
   └──────────────────────────────────┘
                  │
                  ▼
   ┌──────────────────────────────────┐
   │   ACQUIRE INITIAL STATE PROBABILITY │──── S605
   └──────────────────────────────────┘
                  │
                  ▼
   ┌──────────────────────────────────┐
   │    SPECIFY OPTIMAL STATE SEQUENCE │──── S606
   │     (EXECUTE VITERBI ALGORITHM)   │
   └──────────────────────────────────┘
                  │
                  ▼
   ┌──────────────────────────────────┐
   │      SPECIFY TARGET DURATION      │──── S607
   └──────────────────────────────────┘
                  │
                  ▼
           ┌─────────────┐
           │     END     │
           └─────────────┘
```

## FIG. 51A

### <RULE GROUP OF "MIDDLE OF WALKING (RIGHT)">

|  | LEFT-RIGHT ALTERNATION LIKELIHOOD | INTERVAL CONSISTENCY LIKELIHOOD | SPEED CONSISTENCY LIKELIHOOD | INVERSE-LEG SUPPORT LIKELIHOOD | SIMULTANEOUS MOVEMENT LIKELIHOOD | BOTH-LEG LANDING LIKELIHOOD | LANDING FLEXION LIKELIHOOD | TAKEOFF FLEXION LIKELIHOOD |
|---|---|---|---|---|---|---|---|---|
| FUNCTION TYPE | BINOMIAL DISTRIBUTION | NORMAL DISTRIBUTION | NORMAL DISTRIBUTION | CONTINUOUS UNIFORM DISTRIBUTION | CONTINUOUS UNIFORM DISTRIBUTION | CONTINUOUS UNIFORM DISTRIBUTION | CONTINUOUS UNIFORM DISTRIBUTION | CONTINUOUS UNIFORM DISTRIBUTION |
| FUNCTION PARAMETER | $\alpha = 0.95$ | $\mu = 0$ <br> $\sigma = 1$ | $\mu = 0$ <br> $\sigma = 30$ | a=-50 <br> b=0 | a1-10 <br> b1=0 <br> a2=3 <br> b2=10 | a=0 <br> b=4 | a=-100 <br> b=-10 | a=-100 <br> b=-10 |

### <RULE GROUP OF "FIRST STEP (RIGHT)">

|  | LEFT-RIGHT ALTERNATION LIKELIHOOD | INTERVAL CONSISTENCY LIKELIHOOD | SPEED CONSISTENCY LIKELIHOOD | INVERSE-LEG SUPPORT LIKELIHOOD | SIMULTANEOUS MOVEMENT LIKELIHOOD | BOTH-LEG LANDING LIKELIHOOD | LANDING FLEXION LIKELIHOOD | TAKEOFF FLEXION LIKELIHOOD |
|---|---|---|---|---|---|---|---|---|
| FUNCTION TYPE | BINOMIAL DISTRIBUTION | NORMAL DISTRIBUTION | NORMAL DISTRIBUTION | CONTINUOUS UNIFORM DISTRIBUTION | CONTINUOUS UNIFORM DISTRIBUTION | CONTINUOUS UNIFORM DISTRIBUTION | CONTINUOUS UNIFORM DISTRIBUTION | CONTINUOUS UNIFORM DISTRIBUTION |
| FUNCTION PARAMETER | $\alpha = 0.95$ | $\mu = 0$ <br> $\sigma = 5$ | $\mu = 0$ <br> $\sigma = 100$ | a=-50 <br> b=0 | a1-20 <br> b1=5 <br> a2=0 <br> b2=20 | a=0 <br> b=4 | a=-80 <br> b=0 | a=-80 <br> b=0 |

EP 3 459 453 B1

## FIG. 51B

⋮

<RULE GROUP PF "NON-WALKING">

| | LEFT-RIGHT ALTERNATION LIKELIHOOD | INTERVAL CONSISTENCY LIKELIHOOD | SPEED CONSISTENCY LIKELIHOOD | INVERSE-LEG SUPPORT LIKELIHOOD | SIMULTANEOUS MOVEMENT LIKELIHOOD | BOTH-LEG LANDING LIKELIHOOD | LANDING FLEXION LIKELIHOOD | TAKEOFF FLEXION LIKELIHOOD | |
|---|---|---|---|---|---|---|---|---|---|
| FUNCTION TYPE | BINOMIAL DISTRIBUTION | NORMAL DISTRIBUTION | NORMAL DISTRIBUTION | CONTINUOUS UNIFORM DISTRIBUTION | CONTINUOUS UNIFORM DISTRIBUTION | CONTINUOUS UNIFORM DISTRIBUTION | CONTINUOUS UNIFORM DISTRIBUTION | CONTINUOUS UNIFORM DISTRIBUTION | |
| FUNCTION PARAMETER | $\alpha = 0.5$ | $\mu = 0$ $\sigma = 10$ | $\mu = 20$ $\sigma = 1000$ | a=-100 b=100 | a1-100 b1=100 a2=100 b2=100 | a=-10 b=10 | a=-200 b=100 | a=-200 b=100 | ... |

EP 3 459 453 B1

## FIG. 52

| MOTION NUMBER | STATE OBSERVATION PROBABILITY (FIRST STEP, RIGHT) | STATE OBSERVATION PROBABILITY (FIRST STEP, LEFT) | STATE OBSERVATION PROBABILITY (MIDDLE OF WALKING, RIGHT) | STATE OBSERVATION PROBABILITY (MIDDLE OF WALKING, LEFT) | STATE OBSERVATION PROBABILITY (LAST STEP, RIGHT) | STATE OBSERVATION PROBABILITY (LAST STEP, LEFT) | STATE OBSERVATION PROBABILITY (NON-WALKING) |
|---|---|---|---|---|---|---|---|
| 1 | 0.00 | 0.67 | 0.00 | 0.23 | 0.00 | 0.67 | 0.02 |
| 1 | 0.55 | 0.00 | 0.43 | 0.00 | 0.32 | 0.00 | 0.32 |
| ... | ... | ... | ... | ... | ... | ... | ... |
| 1 | 0.24 | 0.00 | 0.12 | 0.00 | 0.19 | 0.00 | 0.43 |

EP 3 459 453 B1

# FIG. 53A

|  | NEXT STATE | | | | | | |
|---|---|---|---|---|---|---|---|
|  | FIRST STEP (RIGHT) | FIRST STEP (LEFT) | MIDDLE OF WALKING (RIGHT) | MIDDLE OF WALKING (LEFT) | LAST STEP (RIGHT) | LAST STEP (LEFT) | NON-WALKING |
| FIRST STEP (RIGHT) | 0 | 0 | 0 | 1 | 0 | 0 | 0 |
| FIRST STEP (LEFT) | 0 | 0 | 1 | 0 | 0 | 0 | 0 |
| MIDDLE OF WALKING (RIGHT) | 0 | 0 | 0 | 0.9 | 0 | 0.1 | 0 |
| MIDDLE OF WALKING (LEFT) | 0 | 0 | 0.9 | 0 | 0.1 | 0 | 0 |
| LAST STEP (RIGHT) | 0 | 0 | 0 | 0 | 0 | 0 | 1 |
| LAST STEP (LEFT) | 0 | 0 | 0 | 0 | 0 | 0 | 1 |
| NON-WALKING | 0.25 | 0.25 | 0 | 0 | 0 | 0 | 0.5 |

PREVIOUS STATE

# FIG. 53B

FIG. 54

EP 3 459 453 B1

| STATE | FIRST STEP (RIGHT) | FIRST STEP (LEFT) | MIDDLE OF WALKING (RIGHT) | MIDDLE OF WALKING (LEFT) | LAST STEP (RIGHT) | LAST STEP (LEFT) | NON-WALKING |
|---|---|---|---|---|---|---|---|
| INITIAL PROBABILITY | 0.4 | 0.4 | 0 | 0 | 0 | 0 | 0.3 |

# FIG. 55

| MOTION NUMBER | 1 | 2 | 3 | 4 | 5 | 6 | 7 | 8 |
|---|---|---|---|---|---|---|---|---|
| STATE (ESTIMATED VALUE) | FIRST STEP (LEFT) | MIDDLE OF WALKING (RIGHT) | MIDDLE OF WALKING (LEFT) | MIDDLE OF WALKING (RIGHT) | MIDDLE OF WALKING (LEFT) | MIDDLE OF WALKING (RIGHT) | MIDDLE OF WALKING (LEFT) | MIDDLE OF WALKING (RIGHT) |

| 9 | 10 | 11 | 12 | 13 |
|---|---|---|---|---|
| MIDDLE OF WALKING (LEFT) | LAST STEP (RIGHT) | NON-WALKING | NON-WALKING | NON-WALKING |

# FIG. 56

| MOTION NUMBER | 1 | 2 | 3 | 4 | 5 | 6 | 7 | 8 |
|---|---|---|---|---|---|---|---|---|
| STATE (ESTIMATED VALUE) | FIRST STEP (LEFT) | MIDDLE OF WALKING (RIGHT) | MIDDLE OF WALKING (LEFT) | MIDDLE OF WALKING (RIGHT) | MIDDLE OF WALKING (LEFT) | MIDDLE OF WALKING (RIGHT) | MIDDLE OF WALKING (LEFT) | MIDDLE OF WALKING (RIGHT) |
| TARGET DURATION | 1 | 1 | 1 | 1 | 1 | 1 | 1 | 1 |

| 9 | 10 | 11 | 12 | 13 |
|---|---|---|---|---|
| MIDDLE OF WALKING (LEFT) | LAST STEP (RIGHT) | NON-WALKING | NON-WALKING | NON-WALKING |
| 1 | 1 | 0 | 0 | 0 |

EP 3 459 453 B1

# FIG. 57

| WALKING START TIME POINT | WALKING END TIME POINT | NUMBER OF STEPS | NATURAL PATTERN LIKELIHOOD |
|---|---|---|---|
| 19:30:01.344 | 19:30:12.687 | 10 | 0.45 |

# FIG. 58

| NAME OF OPERATION PARAMETER LIST | WALKING NUMBER | WALKING START TIME POINT | WALKING END TIME POINT | NUMBER OF STEPS | NATURAL PATTERN LIKELIHOOD |
|---|---|---|---|---|---|
| FAST | 1 | 19:30:01.344 | 19:30:12.687 | 10 | 0.45 |
| SLOW | (NONE) | (NONE) | (NONE) | (NONE) | (NONE) |
| NORMAL | 1 | 19:30:01.121 | 19:30:12.687 | 10 | 0.39 |
| ABNORMAL | 1 | 19:30:01.344 | 19:30:05.987 | 5 | 0.04 |
| ABNORMAL | 2 | 19:30:06.180 | 19:30:32.554 | 7 | 0.14 |

EP 3 459 453 B1

## FIG. 59

| NAME OF OPERATION PARAMETER LIST | WALKING NUMBER | WALKING START TIME POINT | WALKING END TIME POINT | NUMBER OF STEPS | NATURAL PATTERN LIKELIHOOD | EVALUATION VALUE |
|---|---|---|---|---|---|---|
| FAST | 1 | 19:30:01.344 | 19:30:12.687 | 10 | 0.45 | 0.45 |
| SLOW | (NONE) | (NONE) | (NONE) | (NONE) | (NONE) | (NONE) |
| NORMAL | 1 | 19:30:01.121 | 19:30:12.687 | 10 | 0.39 | 0.39 |
| ABNORMAL | 1 | 19:30:01.344 | 19:30:05.987 | 5 | 0.04 | 0.10 |
| ABNORMAL | 2 | 19:30:06.180 | 19:30:32.554 | 7 | 0.14 | |

EP 3 459 453 B1

# FIG. 60

EP 3 459 453 B1

## FIG. 61

EP 3 459 453 B1

|  | NEXT STATE | | | | | | | ASCENDING STAIRS | |
|---|---|---|---|---|---|---|---|---|---|
| PREVIOUS STATE | FIRST STEP (RIGHT) | FIRST STEP (LEFT) | MIDDLE OF WALKING (RIGHT) | MIDDLE OF WALKING (LEFT) | LAST STEP (RIGHT) | LAST STEP (LEFT) | NON-WALKING | ASCENDING STAIRS (RIGHT) | ASCENDING STAIRS (LEFT) |
| FIRST STEP (RIGHT) | 0 | 0 | 0 | 1 | 0 | 0 | 0 | 0 | 0 |
| FIRST STEP (LEFT) | 0 | 0 | 1 | 0 | 0 | 0 | 0 | 0 | 0 |
| MIDDLE OF WALKING (RIGHT) | 0 | 0 | 0 | 0.8 | 0 | 0.1 | 0 | 0 | 0.1 |
| MIDDLE OF WALKING (LEFT) | 0 | 0 | 0.8 | 0 | 0.1 | 0 | 0 | 0.1 | 0 |
| LAST STEP (RIGHT) | 0 | 0 | 0 | 0 | 0 | 0 | 1 | 0 | 0 |
| LAST STEP (LEFT) | 0 | 0 | 0 | 0 | 0 | 0 | 1 | 0 | 0 |
| NON-WALKING | 0.25 | 0.25 | 0 | 0 | 0 | 0 | 0.5 | 0 | 0 |
| ASCENDING STAIRS (RIGHT) | 0 | 0 | 0 | 0.1 | 0 | 0 | 1 | 0 | 1 |
| ASCENDING STAIRS (LEFT) | 0 | 0 | 0.1 | 0 | 0 | 0 | 0.5 | 0.9 | 0 |

# FIG. 62

| MIDDLE OF WALKING | DESCENDING STAIRS | MIDDLE OF WALKING |
|:---:|:---:|:---:|

LEFT-LEG GYRO

RIGHT-LEG GYRO

13:30.50    13:31.07    13:31.24    13:31.41    13:31.58    13:32.15    13:32.49    13:33.06

EP 3 459 453 B1

FIG. 63

# FIG. 64

| | |
|---|---|
| △ | : MOTION START TIME POINT |
| ✕ | : MOTION END TIME POINT |
| ○ | : MOTION PEAK |

LEFT-LEG
PRESSURE MEAN

THRESHOLD

TIME

EP 3 459 453 B1

# FIG. 65

STANDING UP | WALKING | STANDING UP

UPPER-BODY
MOTION AMOUNT

TIME

EP 3 459 453 B1

# FIG. 66

⬭ : PEAK OCCURRENCE POINT

HIP
ACCELERATION

14:30:37    14:30:39    14:30:41

EP 3 459 453 B1

# FIG. 67

# FIG. 68

START

READ PARAMETER SET ～ S210

PARAMETER LOOP PROCESSING START

DESIGN NATURAL MOTION DETECTOR ～ S220

DETECT CANDIDATE MOTION ～ S230

SPECIFY MOTION RANGE ～ S240

EXTRACT MOTION FEATURE VALUE ～ S241

CALCULATE NATURAL MOTION LIKELIHOOD ～ S242

EXTRACT INTER-MOTION FEATURE VALUE ～ S250

READ LIKELIHOOD CALCULATION RULE GROUP ～ S260

CALCULATE FEATURE LIKELIHOOD ～ S270

CALCULATE NATURAL PATTERN LIKELIHOOD ～ S280

PARAMETER LOOP PROCESSING END

DETERMINE ACTIVITY DURATION ～ S290b

END

# FIG. 69

<LEG SWINGING MOTION>

HEIGHT OF PEAK

TIME LENGTH

TIME INTERVAL

FOOT, FRONT-REAR ANGULAR VELOCITY

TIME

START POINT HEIGHT

END POINT HEIGHT

<ROTATING MOTION>

TIME LENGTH

PEAK AREA

FOOT, LEFT-RIGHT ACCELERATION

TIME

HEIGHT OF PEAK

<LANDING MOTION>

TIME LENGTH

MAXIMUM-MINIMUM DIFFERENCE

UPPER-BODY UP-DOWN ACCELERATION

TIME

<TRAVELING MOTION>

TIME LENGTH

MAXIMUM-MINIMUM DIFFERENCE

UPPER-BODY, FRONT-REAR ACCELERATION

TIME

EP 3 459 453 B1

# FIG. 70A

NATURAL MOTION DETECTOR 1

| | WALKING | DESCENDING STAIRS | ASCENDING STAIRS | RUNNING | SKIPPING |
|---|---|---|---|---|---|
| FEATURE VALUE 1, "TIME INTERVAL" | $\mu=1,\ \sigma=1$ | $\mu=0.8,\ \sigma=1$ | $\mu=1.2,\ \sigma=1$ | $\mu=0.8,\ \sigma=1$ | $\mu=1,\ \sigma=1$ |
| FEATURE VALUE 2, "TIME LENGTH" | $\mu=0.5,\ \sigma=0.5$ | $\mu=0.4,\ \sigma=0.5$ | $\mu=0.6,\ \sigma=0.5$ | $\mu=0.4,\ \sigma=0.5$ | $\mu=0.5,\ \sigma=0.5$ |
| FEATURE VALUE 3, "START PEAK HEIGHT" | $\mu=-100,\ \sigma=50$ | $\mu=-50,\ \sigma=50$ | $\mu=-150,\ \sigma=50$ | $\mu=-150,\ \sigma=50$ | $\mu=-150,\ \sigma=50$ |
| FEATURE VALUE 4, "END PEAK HEIGHT" | $\mu=-100,\ \sigma=50$ | $\mu=-150,\ \sigma=50$ | $\mu=-50,\ \sigma=50$ | $\mu=-150,\ \sigma=50$ | $\mu=-150,\ \sigma=50$ |
| FEATURE VALUE 5, "MAXIMUM PEAK HEIGHT" | $\mu=300,\ \sigma=100$ | $\mu=250,\ \sigma=100$ | $\mu=150,\ \sigma=100$ | $\mu=400,\ \sigma=100$ | $\mu=400,\ \sigma=100$ |

EP 3 459 453 B1

# FIG. 70B

NATURAL MOTION DETECTOR 2

|  | WALKING | DESCENDING STAIRS | ASCENDING STAIRS | RUNNING | SKIPPING |
|---|---|---|---|---|---|
| FEATURE VALUE 1, "TIME INTERVAL" | $\mu=2,\ \sigma=1$ | $\mu=1.6,\ \sigma=1$ | $\mu=2.4,\ \sigma=1$ | $\mu=1.6,\ \sigma=1$ | $\mu=2,\ \sigma=1$ |
| FEATURE VALUE 2, "TIME LENGTH" | $\mu=1,\ \sigma=0.5$ | $\mu=0.8,\ \sigma=0.5$ | $\mu=1.2,\ \sigma=0.5$ | $\mu=0.8,\ \sigma=0.5$ | $\mu=1,\ \sigma=0.5$ |
| FEATURE VALUE 3, "START PEAK HEIGHT" | $\mu=-50,\ \sigma=50$ | $\mu=-30,\ \sigma=50$ | $\mu=-100,\ \sigma=50$ | $\mu=-100,\ \sigma=50$ | $\mu=-100,\ \sigma=50$ |
| FEATURE VALUE 4, "END PEAK HEIGHT" | $\mu=-50,\ \sigma=50$ | $\mu=-100,\ \sigma=50$ | $\mu=-30,\ \sigma=50$ | $\mu=-100,\ \sigma=50$ | $\mu=-100,\ \sigma=50$ |
| FEATURE VALUE 5, "MAXIMUM PEAK HEIGHT" | $\mu=200,\ \sigma=100$ | $\mu=150,\ \sigma=100$ | $\mu=100,\ \sigma=100$ | $\mu=250,\ \sigma=100$ | $\mu=250,\ \sigma=100$ |

:

EP 3 459 453 B1

# FIG. 71

<NORMAL DISTRIBUTION>

REFERENCE VALUE PARAMETER =
(MEAN PARAMETER, DISPERSION PARAMETER)

$$p(X_{i,j}|\theta_{i,j}) = N(X_{i,j}|\mu_{i,j})$$

$p(X_{i,j}|\theta_{i,j})$

LIKELIHOOD
ELEMENT

$\sigma_{i,j}$

$\mu_{i,j}$   MOTION FEATURE VALUE $X_{i,j}$

EP 3 459 453 B1

## FIG. 72

| | NATURAL MOTION LIKELIHOOD $P_{action}$ | NATURAL PATTERN LIKELIHOOD $P_{pattern}$ | EVALUATION VALUE | MAXIMUM CANDIDATE | THRESHOLD DETERMINATION |
|---|---|---|---|---|---|
| FAST | 0.15 | 0.67 | 0.101 | ○ | ○ |
| SLOW | 0.09 | 0.56 | 0.050 | -- | -- |
| NORMAL | 0.06 | 0.24 | 0.014 | -- | -- |
| ABNORMAL | 0.04 | 0.11 | 0.004 | -- | -- |

EP 3 459 453 B1

# FIG. 73A

DETECTION PARAMETER 2

POINT C

POINT B

POINT A

DETECTION PARAMETER 1

# FIG. 73B

POINT C

A1

DETECTION PARAMETER 2

d1

POINT B

POINT A

DETECTION PARAMETER 1

EP 3 459 453 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012083705 A1
- US 2016007934 A1

**Non-patent literature cited in the description**

- **BAO.** *Activity Recognition from User-Annotated Acceleration Data,* 2004
- **WEISS.** *The Impact of Personalization on Smartphone-Based Activity Recognition,* 2012
- **GUAN.** *Activity recognition based on semi-supervised learning,* 2007
- **ZHAO.** *Cross-People Mobile-Phone Based Activity Recognition,* 2007
- **MARJAN GHAZVININEJAD et al.** HMM based semi-supervised learning for activity recognition. *Proceedings Of The 2011 International Workshop On Situation Activity & Goal Awareness,* 18 September 2011, ISSN 978-1-4503-0926-4, 95-100
- **GREENE.** *An adaptive gyroscope-based algorithm for temporal gait analysis,* 2012
- **WU.** *A robust step length estimation system for human gait using motion sensors,* 2015
- **DOBKIN.** *Reliability and Validity of Bilateral Ankle Accelerometer Algorithms for Activity Recognition and Walking Speed After Stroke,* 2011